# EUROPEAN PATENT SPECIFICATION

(11) **EP 3 265 564 B1**
(45) Date of publication and mention of the grant of the patent: **26.01.2022**
(21) Application number: 16759471.2
(22) Date of filing: 03.03.2016
(51) Int. Cl.: C12N 15/113, A61K 31/7125

(54) **METHODS FOR MODULATING MECP2 EXPRESSION**
VERFAHREN ZUR MODULIERUNG DER MECP2-EXPRESSION
MÉTHODES DE MODULATION DE L'EXPRESSION DE MECP2

(30) Priority: 03.03.2015 US 201562127693 P
(43) Date of publication of application: 10.01.2018
(73) Proprietor: Ionis Pharmaceuticals, Inc., Carlsbad, CA 92010 (US); Baylor College of Medicine, Houston, TX 77030-3411 (US)
(72) Inventor: ZOGHBI, Huda, Y., Houston, TX 77005 (US); SZTAINBERG, Ezequiel, Houston, TX 77096 (US); FREIER, Susan, M., Carlsbad, CA 92010 (US)
(74) Representative: Carpmaels & Ransford LLP
(86) International application number: PCT/US2016/020610
(87) International publication number: WO 2016/141145

(56) References cited:
- WO-A1-2011/071232
- WO-A1-2013/173608
- WO-A2-2009/027349
- WO-A2-2014/052393
- US-A1- 2009 082 297
- ELISA S NA ET AL: "GABAA Receptor Antagonism Ameliorates Behavioral and Synaptic Impairments Associated with MeCP2 Overexpression", NEUROPSYCHOPHARMACOLOGY., vol. 39, no. 8, 1 July 2014 (2014-07-01), pages 1946-1954, XP055492861, US ISSN: 0893-133X, DOI: 10.1038/npp.2014.43
- JIN J ET AL: "RNAi-induced down-regulation of Mecp2 expression in the rat brain", INTERNATIONAL JOURNAL OF DEVELOPMENTAL NEUROSCIENCE, PERGAMON, OXFORD, GB, vol. 26, no. 5, 1 August 2008 (2008-08-01) , pages 457-465, XP022651250, ISSN: 0736-5748, DOI: 10.1016/J.IJDEVNEU.2008.02.009 [retrieved on 2008-03-04]
- YEHEZKEL SZTAINBERG ET AL: "Reversal of phenotypes in MECP2 duplication mice using genetic rescue or antisense oligonucleotides", NATURE, 25 November 2015 (2015-11-25), XP055455838, GB ISSN: 0028-0836, DOI: 10.1038/nature16159

## Description

### Statement of Government Support

This invention was made with government support under P30HD024064 and 5R01NS057819 awarded by the National Institutes of Health. The government has certain rights in the invention.

### Field

Provided are methods for modulating expression of methyl CpG binding protein 2 (MECP2) mRNA and protein in an animal. Such methods are useful to treat, prevent, or ameliorate neurological disorders, including MECP2 duplication syndrome, by reducing expression and amount of MECP2 mRNA and protein in an animal.

### Background

Methyl CpG binding protein 2 (MECP2) is located on chromosome Xq28 and plays a fundamental role in epigenetics, controlling chromatin states, and expression of thousands of genes (Chahrour et al., Science, 2008, 320:1224-1229; Nan et al., Nature, 1998, 393:386-389; Jones et al., Nat. Genet., 1998, 19:187-191). MECP2 expression must be maintained within a fairly narrow range to assure proper gene expression and neuronal function (Nan et al., Nature, 1988, 393:386-389). MECP2 duplication syndrome caused by overexpression of MECP2 is characterized by autism, intellectual disability, motor dysfunction, anxiety, epilepsy, recurrent respiratory tract infections, and early death, typically in males (Ramocki et al., Am J Med Genet A, 2010, 152A: 1079-1088). Underexpression of MECP2 is associated with Rett Syndrome, which is characterized by normal early growth and development followed by a slowing of development, loss of purposeful use of the hands, distinctive hand movements, slowed brain and head growth, problems with walking, seizures, and intellectual disability, typically in females (Weaving et al., J Med Genet, 2005, 42:1-7).

Currently there is a lack of acceptable options for treating such neurological disorders.

GABAA receptor antagonists may offer a possible therapeutic target for the treatment of MECP2 duplication syndrome (Elisa et al., Neuropsychopharmacology, 2014, 39:1946-1954).

It is therefore an object herein to provide methods for the treatment of such disorders.

### Summary

The invention provides an antisense compound complementarty to a MECP2 nucleic acid for use in treating MECP2 duplication syndrome. The embodiments of the invention are defined in the claims.

Disclosed herein are methods for modulating expression and amount of methyl CpG binding protein 2 (MECP2) mRNA and protein Compounds useful for modulating expression and amount of MECP2 mRNA and protein may be antisense compounds. The antisense compounds may be modified antisense oligonucleotides.

Modulation may occur in a cell or tissue. The cell or tissue may be in an animal. The animal may be a human. MECP2 mRNA levels may be reduced or MECP2 protein levels may be reduced. Such reduction can occur in a time-dependent manner or in a dose-dependent manner.

Also disclosed are methods useful for preventing, treating, and ameliorating disorders and syndromes associated with MECP2 overexpression. A disorder associated with MECP2 overexpression may be a neurological disorder. The neurological disorder may be MECP2 duplication syndrome. MECP2 duplication syndrome may be characterized by having additional copies of MECP2, which leads to overexpression of MECP2.

MECP2 duplication syndrome may be characterized by autism, intellectual disability, motor dysfunction, anxiety, epilepsy, recurrent respiratory tract infections, and early death. MECP2 duplication syndrome may be inherited in an X-linked pattern.

Methods of treatment may include administering a MECP2 antisense compound to an individual in need thereof. Methods of treatment may include administering a MECP2 modified antisense oligonucleotide to an individual in need thereof.

MECP2 levels may be reduced sufficiently to prevent, treat, and ameliorate symptoms of MECP2 duplication syndrome, but not enough to cause symptoms of Rett Syndrome.

### Brief Description of the Drawings

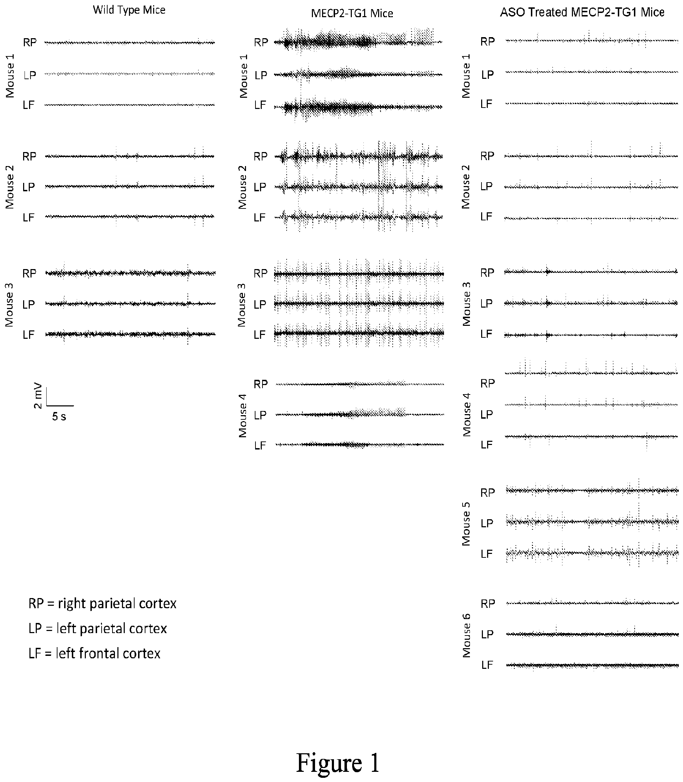

**Figure 1** displays representative EEG traces for WT mice, MECP2-TG1 mice without Isis No. 628785 treatment, and MECP2-TG1 mice that received treatment with Isis No. 628785.

### Detailed Description

It is to be understood that both the foregoing general description and the following detailed description are exemplary and explanatory only and are not restrictive of the invention, as claimed. Herein, the use of the singular includes the plural unless specifically stated otherwise. As used herein, the use of "or" means "and/or" unless stated otherwise. Additionally, as used herein, the use of "and" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described. All

### Definitions

Unless specific definitions are provided, the nomenclature utilized in connection with, and the procedures and techniques of, analytical chemistry, synthetic organic chemistry, and medicinal and pharmaceutical chemistry described herein are those well known and commonly used in the art. Standard techniques may be used for chemical synthesis, and chemical analysis.

Unless otherwise indicated, the following terms have the following meanings:
"2'-O-methoxyethyl" (also 2'-MOE and 2'-OCH₂CH₂-OCH₃ and MOE) refers to an O-methoxyethyl modification of the 2' position of a furanose ring. A 2'-O-methoxyethyl modified sugar is a modified sugar.

"2'-MOE nucleoside" (also 2'-O-methoxyethyl nucleoside) means a nucleoside comprising a 2'-MOE modified sugar moiety.

"2'-substituted nucleoside" means a nucleoside comprising a substituent at the 2'-position of the furanose ring other than H or OH. In certain embodiments, 2' substituted nucleosides include nucleosides with bicyclic sugar modifications.

"5-methylcytosine" means a cytosine modified with a methyl group attached to the 5 position. A 5-methylcytosine is a modified nucleobase.

"Administered concomitantly" refers to the co-administration of two pharmaceutical agents in any manner in which the pharmacological effects of both are manifest in the patient at the same time. Concomitant administration does not require that both pharmaceutical agents be administered in a single pharmaceutical composition, in the same dosage form, or by the same route of administration. The effects of both pharmaceutical agents need not manifest themselves at the same time. The effects need only be overlapping for a period of time and need not be coextensive.

"Administering" means providing a pharmaceutical agent to an animal, and includes, but is not limited to administering by a medical professional and self-administering.

"Amelioration" refers to a lessening, slowing, stopping, or reversing of at least one indicator of the severity of a syndrome or condition. The severity of indicators may be determined by subjective or objective measures, which are known to those skilled in the art.

"Animal" refers to a human or non-human animal, including, but not limited to, mice, rats, rabbits, dogs, cats, pigs, and non-human primates, including, but not limited to, monkeys and chimpanzees.

"Antibody" refers to a molecule characterized by reacting specifically with an antigen in some way, where the antibody and the antigen are each defined in terms of the other. Antibody may refer to a complete antibody molecule or any fragment or region thereof, such as the heavy chain, the light chain, Fab region, and Fc region.

"Antisense activity" means any detectable or measurable activity attributable to the hybridization of an antisense compound to its target nucleic acid. In certain embodiments, antisense activity is a decrease in the amount or expression of a target nucleic acid or protein encoded by such target nucleic acid.

"Antisense compound" means an oligomeric compound that is capable of undergoing hybridization to a target nucleic acid through hydrogen bonding. Examples of antisense compounds include single-stranded and double-stranded compounds, such as, antisense oligonucleotides, siRNAs, shRNAs, ssRNAs, and occupancy-based compounds.

"Antisense inhibition" or "inhibition" means reduction of target nucleic acid levels in the presence of an antisense compound complementary to a target nucleic acid compared to target nucleic acid levels or in the absence of the antisense compound.

"Antisense mechanisms" are all those mechanisms involving hybridization of a compound with a target nucleic acid, wherein the outcome or effect of the hybridization is either target degradation or target occupancy with concomitant stalling of the cellular machinery involving, for example, transcription or splicing.

"Antisense oligonucleotide" means a single-stranded oligonucleotide having a nucleobase sequence that permits hybridization to a corresponding segment of a target nucleic acid.

"Base complementarity" refers to the capacity for the precise base pairing of nucleobases of an antisense oligonucleotide with corresponding nucleobases in a target nucleic acid (i.e., hybridization), and is mediated by Watson-Crick, Hoogsteen or reversed Hoogsteen hydrogen binding between corresponding nucleobases.

"Bicyclic sugar" means a furanose ring modified by the bridging of two atoms. A bicyclic sugar is a modified sugar.

"Bicyclic nucleoside" (also BNA) means a nucleoside having a sugar moiety comprising a bridge connecting two carbon atoms of the sugar ring, thereby forming a bicyclic ring system. In certain embodiments, the bridge connects the 4'-carbon and the 2'-carbon of the sugar ring.

"Cap structure" or "terminal cap moiety" means chemical modifications, which have been incorporated at either terminus of an antisense compound.

"cEt" or "constrained ethyl" means a bicyclic nucleoside having a sugar moiety comprising a bridge connecting the 4'-carbon and the 2'-carbon, wherein the bridge has the formula: 4'-CH(CH₃)-O-2'.

"Constrained ethyl nucleoside" (also cEt nucleoside) means a nucleoside comprising a bicyclic sugar moiety comprising a 4'-CH(CH₃)-O-2' bridge.

"Chemically distinct region" refers to a region of an antisense compound that is in some way chemically different than another region of the same antisense compound. For example, a region having 2'-O-methoxyethyl nucleosides is chemically distinct from a region having nucleosides without 2'-O-methoxyethyl modifications.

"Chimeric antisense compound" means an antisense compound that has at least two chemically distinct regions, each position having a plurality of subunits.

"Co-administration" means administration of two or more pharmaceutical agents to an individual. The two or more pharmaceutical agents may be in a single pharmaceutical composition, or may be in separate pharmaceutical compositions. Each of the two or more pharmaceutical agents may be administered through the same or different routes of administration. Co-administration encompasses parallel or sequential administration.

"Complementarity" means the capacity for pairing between nucleobases of a first nucleic acid and a second nucleic acid.

"Comprise," "comprises," and "comprising" will be understood to imply the inclusion of a stated step or element or group of steps or elements but not the exclusion of any other step or element or group of steps or elements.

"Contiguous nucleobases" means nucleobases immediately adjacent to each other.

"Designing" or "designed to" refer to the process of designing an oligomeric compound that specifically hybridizes with a selected nucleic acid molecule.

"Diluent" means an ingredient in a composition that lacks pharmacological activity, but is pharmaceutically necessary or desirable. For example, in drugs that are injected, the diluent may be a liquid, e.g. saline solution.

"Dose" means a specified quantity of a pharmaceutical agent provided in a single administration, or in a specified time period. In certain embodiments, a dose may be administered in one, two, or more boluses, tablets, or injections. For example, in certain embodiments where subcutaneous administration is desired, the desired dose requires a volume not easily accommodated by a single injection, therefore, two or more injections may be used to achieve the desired dose. In certain embodiments, the pharmaceutical agent is administered by infusion over an extended period of time or continuously. Doses may be stated as the amount of pharmaceutical agent per hour, day, week, or month.

"Effective amount" in the context of modulating an activity or of treating or preventing a condition means the administration of that amount of pharmaceutical agent to an individual in need of such modulation, treatment, or prophylaxis, either in a single dose or as part of a series, that is effective for modulation of that effect, or for treatment or prophylaxis or improvement of that condition. The effective amount may vary among individuals depending on the health and physical condition of the individual to be treated, the taxonomic group of the individuals to be treated, the formulation of the composition, assessment of the individual's medical condition, and other relevant factors.

"Efficacy" means the ability to produce a desired effect.

"Expression" includes all the functions by which a gene's coded information is converted into structures present and operating in a cell. Such structures include, but are not limited to the products of transcription and translation.

"Fully complementary" or "100% complementary" means each nucleobase of a first nucleic acid has a complementary nucleobase in a second nucleic acid. In certain embodiments, a first nucleic acid is an antisense compound and a target nucleic acid is a second nucleic acid.

"Gapmer" means a chimeric antisense compound in which an internal region having a plurality of nucleosides that support RNase H cleavage is positioned between external regions having one or more nucleosides, wherein the nucleosides comprising the internal region are chemically distinct from the nucleoside or nucleosides comprising the external regions. The internal region may be referred to as a "gap" and the external regions may be referred to as the "wings."

"Hybridization" means the annealing of complementary nucleic acid molecules. In certain embodiments, complementary nucleic acid molecules include, but are not limited to, an antisense compound and a target nucleic acid. In certain embodiments, complementary nucleic acid molecules include, but are not limited to, an antisense oligonucleotide and a nucleic acid target.

"Identifying an animal having a MECP2 associated disorder" means identifying an animal having been diagnosed with a MECP2 associated disorder or predisposed to develop a MECP2 associated disorder. Individuals predisposed to develop a MECP2 associated disorder include those having one or more risk factors for developing a MECP2 associated disorder, including, having a personal or family history or genetic predisposition to one or more MECP2 associated disorders. Such identification may be accomplished by any method including evaluating an individual's medical history and standard clinical tests or assessments, such as genetic testing.

"Immediately adjacent" means there are no intervening elements between the immediately adjacent elements.

"Individual" means a human or non-human animal selected for treatment or therapy.

"Inhibiting MECP2" means reducing the level or expression of a MECP2 mRNA and/or protein. In certain embodiments, MECP2 mRNA and/or protein levels are inhibited in the presence of an antisense compound targeting MECP2, including an antisense oligonucleotide targeting MECP2, as compared to expression of MECP2 mRNA and/or protein levels in the absence of a MECP2 antisense compound, such as an antisense oligonucleotide.

"Inhibiting the expression or activity" refers to a reduction or blockade of the expression or activity and does not necessarily indicate a total elimination of expression or activity.

"Internucleoside linkage" refers to the chemical bond between nucleosides.

"Linked nucleosides" means adjacent nucleosides linked together by an internucleoside linkage.

"MECP2 antisense compound" means an antisense compound targeting MECP2.

"MECP2" means the mammalian gene methyl CpG binding protein 2 (MECP2), including the human gene methyl CpG binding protein 2 (MECP2). Human MECP2 has been mapped to human chromosome Xq28.

"MECP2 associated disorder" means any disorder or syndrome associated with any MECP2 nucleic acid or expression product thereof. Such disorders may include a neurological disorder. Such neurological disorders may include MECP2 duplication syndrome.

"MECP2 nucleic acid" means any nucleic acid encoding MECP2. For example, in certain embodiments, a MECP2 nucleic acid includes a DNA sequence encoding MECP2, an RNA sequence transcribed from DNA encoding MECP2 (including genomic DNA comprising introns and exons), and an mRNA sequence encoding MECP2.

"MECP2 mRNA" means any messenger RNA expression product of a DNA sequence encoding MECP2.

"MECP2 protein" means the polypeptide expression product of a MECP2 nucleic acid.

"Mismatch" or "non-complementary nucleobase" refers to the case when a nucleobase of a first nucleic acid is not capable of pairing with the corresponding nucleobase of a second or target nucleic acid.

"Modified internucleoside linkage" refers to a substitution or any change from a naturally occurring internucleoside bond (*i.e.,* a phosphodiester internucleoside bond).

"Modified nucleobase" means any nucleobase other than adenine, cytosine, guanine, thymidine, or uracil. An "unmodified nucleobase" means the purine bases adenine (A) and guanine (G), and the pyrimidine bases thymine (T), cytosine (C), and uracil (U).

"Modified nucleoside" means a nucleoside having, independently, a modified sugar moiety and/or modified nucleobase.

"Modified nucleotide" means a nucleotide having, independently, a modified sugar moiety, modified internucleoside linkage, and/or modified nucleobase.

"Modified antisense oligonucleotide" means an oligonucleotide comprising at least one modified internucleoside linkage, modified sugar, and/or modified nucleobase.

"Modified sugar" means substitution and/or any change from a natural sugar moiety.

"Monomer" means a single unit of an oligomer. Monomers include, but are not limited to, nucleosides and nucleotides, whether naturally occurring or modified.

"Motif' means the pattern of unmodified and modified nucleosides in an antisense compound.

"Natural sugar moiety" means a sugar moiety found in DNA (2'-H) or RNA (2'-OH).

"Naturally occurring internucleoside linkage" means a 3' to 5' phosphodiester linkage.

"Non-complementary nucleobase" refers to a pair of nucleobases that do not form hydrogen bonds with one another or otherwise support hybridization.

"Nucleic acid" refers to molecules composed of monomeric nucleotides. A nucleic acid includes, but is not limited to, ribonucleic acids (RNA), deoxyribonucleic acids (DNA), single-stranded nucleic acids, double-stranded nucleic acids, small interfering ribonucleic acids (siRNA), and microRNAs (miRNA).

"Nucleobase" means a heterocyclic moiety capable of pairing with a base of another nucleic acid.

"Nucleobase complementarity" refers to a nucleobase that is capable of base pairing with another nucleobase. For example, in DNA, adenine (A) is complementary to thymine (T). For example, in RNA, adenine (A) is complementary to uracil (U). In certain embodiments, complementary nucleobase refers to a nucleobase of an antisense compound that is capable of base pairing with a nucleobase of its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be complementary at that nucleobase pair.

"Nucleobase sequence" means the order of contiguous nucleobases independent of any sugar, linkage, and/or nucleobase modification.

"Nucleoside" means a nucleobase linked to a sugar.

"Nucleoside mimetic" includes those structures used to replace the sugar or the sugar and the base and not necessarily the linkage at one or more positions of an oligomeric compound such as for example nucleoside mimetics having morpholino, cyclohexenyl, cyclohexyl, tetrahydropyranyl, bicyclo, or tricyclo sugar mimetics, *e.g.*, non furanose sugar units. Nucleotide mimetic includes those structures used to replace the nucleoside and the linkage at one or more positions of an oligomeric compound such as for example peptide nucleic acids or morpholinos (morpholinos linked by -N(H)-C(=O)-O- or other non-phosphodiester linkage). Sugar surrogate overlaps with the slightly broader term nucleoside mimetic but is intended to indicate replacement of the sugar unit (furanose ring) only. The tetrahydropyranyl rings provided herein are illustrative of an example of a sugar surrogate wherein the furanose sugar group has been replaced with a tetrahydropyranyl ring system. "Mimetic" refers to groups that are substituted for a sugar, a nucleobase, and/or internucleoside linkage. Generally, a mimetic is used in place of the sugar or sugar-internucleoside linkage combination, and the nucleobase is maintained for hybridization to a selected target.

"Nucleotide" means a nucleoside having a phosphate group covalently linked to the sugar portion of the nucleoside.

"Off-target effect" refers to an unwanted or deleterious biological effect associated with modulation of RNA or protein expression of a gene other than the intended target nucleic acid.

"Oligomeric compound" or "oligomer" means a polymer of linked monomeric subunits which is capable of hybridizing to at least a region of a nucleic acid molecule.

"Oligonucleotide" means a polymer of linked nucleosides each of which can be modified or unmodified, independent one from another.

"Parenteral administration" means administration through injection (e.g., bolus injection) or infusion. Parenteral administration includes subcutaneous administration, intravenous administration, intramuscular administration, intraarterial administration, intraperitoneal administration, or intracranial administration, e.g., intrathecal or intracerebroventricular administration.

"Peptide" means a molecule formed by linking at least two amino acids by amide bonds. Without limitation, as used herein, peptide refers to polypeptides and proteins.

"Pharmaceutical agent" means a substance that provides a therapeutic benefit when administered to an individual. For example, in certain embodiments, an antisense oligonucleotide targeted to MECP2 is a pharmaceutical agent.

"Pharmaceutical composition" means a mixture of substances suitable for administering to an individual. For example, a pharmaceutical composition may comprise an antisense oligonucleotide and a sterile aqueous solution.

"Pharmaceutically acceptable derivative" encompasses pharmaceutically acceptable salts, conjugates, prodrugs or isomers of the compounds described herein.

"Pharmaceutically acceptable salts" means physiologically and pharmaceutically acceptable salts of antisense compounds, *i.e.,* salts that retain the desired biological activity of the parent oligonucleotide and do not impart undesired toxicological effects thereto.

"Phosphorothioate linkage" means a linkage between nucleosides where the phosphodiester bond is modified by replacing one of the non-bridging oxygen atoms with a sulfur atom. A phosphorothioate linkage is a modified internucleoside linkage.

"Portion" means a defined number of contiguous (*i.e.,* linked) nucleobases of a nucleic acid. In certain embodiments, a portion is a defined number of contiguous nucleobases of a target nucleic acid. In certain embodiments, a portion is a defined number of contiguous nucleobases of an antisense compound.

"Prevent" or "preventing" refers to delaying or forestalling the onset or development of a disorder or syndrome for a period of time from minutes to days, weeks to months, or indefinitely.

"Prodrug" means a therapeutic agent that is prepared in an inactive form that is converted to an active form (i.e., drug) within the body or cells thereof by the action of endogenous enzymes or other chemicals and/or conditions.

"Prophylactically effective amount" refers to an amount of a pharmaceutical agent that provides a prophylactic or preventative benefit to an animal.

"Region" is defined as a portion of the target nucleic acid having at least one identifiable structure, function, or characteristic.

"Ribonucleotide" means a nucleotide having a hydroxy at the 2' position of the sugar portion of the nucleotide. Ribonucleotides may be modified with any of a variety of substituents.

"Salt" means a physiologically and pharmaceutically acceptable salt(s) of antisense compounds, i.e., salts that retain the desired biological activity of the parent oligonucleotide and do not impart undesired toxicological effects thereto.

"Segments" are defined as smaller or sub-portions of regions within a target nucleic acid.

"Shortened" or "truncated" versions of antisense oligonucleotides taught herein have one, two or more nucleosides deleted.

"Side effects" means physiological responses attributable to a treatment other than desired effects. In certain embodiments, side effects include, without limitation, injection site reactions, liver function test abnormalities, renal function abnormalities, liver toxicity, renal toxicity, central nervous system abnormalities, and myopathies.

"Single-stranded oligonucleotide" means an oligonucleotide which is not hybridized to a complementary strand.

"Sites" as used herein, are defined as unique nucleobase positions within a target nucleic acid.

"Slows progression" means decrease in the development of the disorder or syndrome.

"Specifically hybridizable" refers to an antisense compound having a sufficient degree of complementarity between an antisense oligonucleotide and a target nucleic acid to induce a desired effect, while exhibiting minimal or no effects on non-target nucleic acids under conditions in which specific binding is desired, *i.e.,* under physiological conditions in the case *of in vivo* assays and therapeutic treatments.

"Stringent hybridization conditions" or "stringent conditions" refer to conditions under which an oligomeric compound will hybridize to its target sequence, but to a minimal number of other sequences.

"Targeting" or "targeted" means the process of design and selection of an antisense compound that will specifically hybridize to a target nucleic acid and induce a desired effect.

"Target nucleic acid," "target RNA," and "target RNA transcript" and "nucleic acid target" all mean a nucleic acid capable of being targeted by antisense compounds. In certain embodiments, the target nucleic acid is a MECP2 nucleic acid.

"Target region" means a portion of a target nucleic acid to which one or more antisense compounds is targeted.

"Target segment" means the sequence of nucleotides of a target nucleic acid to which an antisense compound is targeted. "5' target site" refers to the 5'-most nucleotide of a target segment. "3' target site" refers to the 3'-most nucleotide of a target segment.

"Therapeutically effective amount" means an amount of a pharmaceutical agent that provides a therapeutic benefit to an individual.

"Treat" or "treating" or "treatment" refers administering a composition to effect an alteration or improvement of the disorder or syndrome.

"Unmodified nucleobases" mean the purine bases adenine (A) and guanine (G), and the pyrimidine bases thymine (T), cytosine (C) and uracil (U).

"Unmodified nucleotide" means a nucleotide composed of naturally occuring nucleobases, sugar moieties, and internucleoside linkages. In certain embodiments, an unmodified nucleotide is an RNA nucleotide (*i.e.* β-D-ribonucleosides) or a DNA nucleotide (*i.e.* β-D-deoxyribonucleoside).

"Wing segment" means a plurality of nucleosides modified to impart to an oligonucleotide properties such as enhanced inhibitory activity, increased binding affinity for a target nucleic acid, or resistance to degradation by in vivo nucleases.

### Certain Disclosures

Disclosed herein are methods for inhibiting MECP2 mRNA and protein expression. Disclosed herein are methods, compounds, and compositions for decreasing MECP2 mRNA and protein levels.

Disclosed herein are antisense compounds targeted to a MECP2 nucleic acid. The MECP2 nucleic acid may be 2. the sequence set forth in GENBANK Accession No. NM_004992.3 (incorporated herein as SEQ ID NO: 1) and the complement of GENBANK Accession No. NT_167198.1 truncated from nucleotides 4203000 to 4283000 (incorporated herein as SEQ ID NO: 2).

Disclosed herein are methods for the treatment, prevention, or amelioration of disorders and syndromes associated with MECP2 in an individual in need thereof. Also contemplated are methods for the preparation of a medicament for the treatment, prevention, or amelioration of a disorder or syndrome associated with MECP2. MECP2 associated disorders and syndromes include neurological disorders. MECP2 associated disorders include MECP2 duplication syndrome.

### Antisense Compounds

Oligomeric compounds include, but are not limited to, oligonucleotides, oligonucleosides, oligonucleotide analogs, oligonucleotide mimetics, antisense compounds, antisense oligonucleotides, and siRNAs. An oligomeric compound may be "antisense" to a target nucleic acid, meaning that is is capable of undergoing hybridization to a target nucleic acid through hydrogen bonding.

In certain embodiments, an antisense compound has a nucleobase sequence that, when written in the 5' to 3' direction, comprises the reverse complement of the target segment of a target nucleic acid to which it is targeted. In certain such embodiments, an antisense oligonucleotide has a nucleobase sequence that, when written in the 5' to 3' direction, comprises the reverse complement of the target segment of a target nucleic acid to which it is targeted.

In certain embodiments, an antisense compound targeted to a target nucleic acid is 12 to 30 subunits in length. In certain embodiments, an antisense compound targeted to a target nucleic acid is 12 to 25 subunits in length. In certain embodiments, an antisense compound targeted to a target nucleic acid is 12 to 22 subunits in length. In certain embodiments, an antisense compound targeted to a target nucleic acid is 14 to 20 subunits in length. In certain embodiments, an antisense compound targeted to a target nucleic acid is 15 to 25 subunits in length. In certain embodiments, an antisense compound targeted to a target nucleic acid is 18 to 22 subunits in length. In certain embodiments, an antisense compound targeted to a target nucleic acid is 19 to 21 subunits in length. In certain embodiments, the antisense compound is 8 to 80, 12 to 50, 13 to 30, 13 to 50, 14 to 30, 14 to 50, 15 to 30, 15 to 50, 16 to 30, 16 to 50, 17 to 30, 17 to 50, 18 to 30, 18 to 50, 19 to 30, 19 to 50, or 20 to 30 linked subunits in length.

In certain embodiments, an antisense compound targeted to a target nucleic acid is 12 subunits in length. In certain embodiments, an antisense compound targeted to a target nucleic acid is 13 subunits in length. In certain embodiments, an antisense compound targeted to a target nucleic acid is 14 subunits in length. In certain embodiments, an antisense compound targeted to a target nucleic acid is 15 subunits in length. In certain embodiments, an antisense compound targeted to a target nucleic acid is 16 subunits in length. In certain embodiments, an antisense compound targeted to a target nucleic acid is 17 subunits in length. In certain embodiments, an antisense compound targeted to a target nucleic acid is 18 subunits in length. In certain embodiments, an antisense compound targeted to a target nucleic acid is 19 subunits in length. In certain embodiments, an antisense compound targeted to a target nucleic acid is 20 subunits in length. In certain embodiments, an antisense compound targeted to a target nucleic acid is 21 subunits in length. In certain embodiments, an antisense compound targeted to a target nucleic acid is 22 subunits in length. In certain embodiments, an antisense compound targeted to a target nucleic acid is 23 subunits in length. In certain embodiments, an antisense compound targeted to a target nucleic acid is 24 subunits in length. In certain embodiments, an antisense compound targeted to a target nucleic acid is 25 subunits in length. In certain embodiments, an antisense compound targeted to a target nucleic acid is 26 subunits in length. In certain embodiments, an antisense compound targeted to a target nucleic acid is 27 subunits in length. In certain embodiments, an antisense compound targeted to a target nucleic acid is 28 subunits in length. In certain embodiments, an antisense compound targeted to a target nucleic acid is 29 subunits in length. In certain embodiments, an antisense compound targeted to a target nucleic acid is 30 subunits in length. In certain embodiments, the antisense compound targeted to a target nucleic acid is 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 linked subunits in length, or a range defined by any two of the above values. In certain embodiments the antisense compound is an antisense oligonucleotide, and the linked subunits are nucleosides.

In certain embodiments antisense oligonucleotides targeted to a target nucleic acid may be shortened or truncated. For example, a single subunit may be deleted from the 5' end (5' truncation), or alternatively from the 3' end (3' truncation). A shortened or truncated antisense compound targeted to a target nucleic acid may have two subunits deleted from the 5' end, or alternatively may have two subunits deleted from the 3' end, of the antisense compound. Alternatively, the deleted nucleosides may be dispersed throughout the antisense compound, for example, in an antisense compound having one nucleoside deleted from the 5' end and one nucleoside deleted from the 3' end.

When a single additional subunit is present in a lengthened antisense compound, the additional subunit may be located at the 5' or 3' end of the antisense compound. When two or more additional subunits are present, the added subunits may be adjacent to each other, for example, in an antisense compound having two subunits added to the 5' end (5' addition), or alternatively to the 3' end (3' addition), of the antisense compound. Alternatively, the added subunits may be dispersed throughout the antisense compound, for example, in an antisense compound having one subunit added to the 5' end and one subunit added to the 3' end.

It is possible to increase or decrease the length of an antisense compound, such as an antisense oligonucleotide, and/or introduce mismatch bases without eliminating activity. For example, in Woolf et al. (Proc. Natl. Acad. Sci. USA 89:7305-7309, 1992), a series of antisense oligonucleotides 13-25 nucleobases in length were tested for their ability to induce cleavage of a target RNA in an oocyte injection model. Antisense oligonucleotides 25 nucleobases in length with 8 or 11 mismatch bases near the ends of the antisense oligonucleotides were able to direct specific cleavage of the target mRNA, albeit to a lesser extent than the antisense oligonucleotides that contained no mismatches. Similarly, target specific cleavage was achieved using 13 nucleobase antisense oligonucleotides, including those with 1 or 3 mismatches.

Gautschi et al (J. Natl. Cancer Inst. 93:463-471, March 2001) demonstrated the ability of an oligonucleotide having 100% complementarity to the bcl-2 mRNA and having 3 mismatches to the bcl-xL mRNA to reduce the expression of both bcl-2 and bcl-xL *in vitro* and *in vivo.* Furthermore, this oligonucleotide demonstrated potent anti-tumor activity *in vivo.*

Maher and Dolnick (Nuc. Acid. Res. 16:3341-3358,1988) tested a series of tandem 14 nucleobase antisense oligonucleotides, and a 28 and 42 nucleobase antisense oligonucleotides comprised of the sequence of two or three of the tandem antisense oligonucleotides, respectively, for their ability to arrest translation of human DHFR in a rabbit reticulocyte assay. Each of the three 14 nucleobase antisense oligonucleotides alone was able to inhibit translation, albeit at a more modest level than the 28 or 42 nucleobase antisense oligonucleotides.

### Antisense Compound Motifs

In certain embodiments, antisense compounds targeted to a target nucleic acid have chemically modified subunits arranged in patterns, or motifs, to confer to the antisense compounds properties such as enhanced inhibitory activity, increased binding affinity for a target nucleic acid, or resistance to degradation by *in vivo* nucleases.

Chimeric antisense compounds typically contain at least one region modified so as to confer increased resistance to nuclease degradation, increased cellular uptake, increased binding affinity for the target nucleic acid, and/or increased inhibitory activity. A second region of a chimeric antisense compound may optionally serve as a substrate for the cellular endonuclease RNase H, which cleaves the RNA strand of an RNA:DNA duplex.

Antisense compounds having a gapmer motif are considered chimeric antisense compounds. In a gapmer an internal region having a plurality of nucleotides that supports RNaseH cleavage is positioned between external regions having a plurality of nucleotides that are chemically distinct from the nucleosides of the internal region. In the case of an antisense oligonucleotide having a gapmer motif, the gap segment generally serves as the substrate for endonuclease cleavage, while the wing segments comprise modified nucleosides. In certain embodiments, the regions of a gapmer are differentiated by the types of sugar moieties comprising each distinct region. The types of sugar moieties that are used to differentiate the regions of a gapmer may in some embodiments include β-D-ribonucleosides, β-D-deoxyribonucleosides, 2'-modified nucleosides (such 2'-modified nucleosides may include 2'-MOE, and 2'-O-CH₃, among others), and bicyclic sugar modified nucleosides (such bicyclic sugar modified nucleosides may include those having a 4'-(CH₂)n-O-2' bridge, where n=1 or n=2 and 4'-CH₂-O-CH₂-2'). In certain embodiments, wings may include several modified sugar moieties, including, for example 2'-MOE. In certain embodiments, wings may include several modified and unmodified sugar moieties. In certain embodiments, wings may include various combinations of 2'-MOE nucleosides and 2'-deoxynucleosides.

Each distinct region may comprise uniform sugar moieties, variant, or alternating sugar moieties. The wing-gap-wing motif is frequently described as "X-Y-Z", where "X" represents the length of the 5' wing, "Y" represents the length of the gap, and "Z" represents the length of the 3' wing. "X" and "Z" may comprise uniform, variant, or alternating sugar moieties. In certain embodiments, "X" and "Y" may include one or more 2'-deoxynucleosides. "Y" may comprise 2'-deoxynucleosides. As used herein, a gapmer described as "X-Y-Z" has a configuration such that the gap is positioned immediately adjacent to each of the 5' wing and the 3' wing. Thus, no intervening nucleotides exist between the 5' wing and gap, or the gap and the 3' wing. Any of the antisense compounds described herein can have a gapmer motif. In certain embodiments, "X" and "Z" are the same; in other embodiments they are different.

In certain embodiments, gapmers provided herein include, for example 20-mers having a motif of 5-10-5. In certain embodiments, gapmers provided herein include, for example 19-mers having a motif of 5-9-5. In certain embodiments, gapmers provided herein include, for example 18-mers having a motif of 5-8-5. In certain embodiments, gapmers provided herein include, for example 18-mers having a motif of 4-8-6. In certain embodiments, gapmers provided herein include, for example 18-mers having a motif of 6-8-4. In certain embodiments, gapmers provided herein include, for example 18-mers having a motif of 5-7-6.

### Target Nucleic Acids, Target Regions and Nucleotide Sequences

Nucleotide sequences that encode MECP2 include, without limitation, the following: GENBANK Accession No. NM_004992.3 (incorporated herein as SEQ ID NO: 1) and the complement of GENBANK Accession No. NT_167198.1 truncated from nucleotides 4203000 to 4283000 (incorporated herein as SEQ ID NO: 2).

It is understood that the sequence set forth in each SEQ ID NO in the Examples contained herein is independent of any modification to a sugar moiety, an internucleoside linkage, or a nucleobase. As such, antisense compounds defined by a SEQ ID NO may comprise, independently, one or more modifications to a sugar moiety, an internucleoside linkage, or a nucleobase. Antisense compounds described by Isis Number (Isis No) indicate a combination of nucleobase sequence and motif.

In certain embodiments, a target region is a structurally defined region of the target nucleic acid. For example, a target region may encompass a 3' UTR, a 5' UTR, an exon, an intron, an exon/intron junction, a coding region, a translation initiation region, translation termination region, or other defined nucleic acid region. The structurally defined regions for MECP2 can be obtained by accession number from sequence databases such as NCBI .

In certain embodiments, a target region may encompass the sequence from a 5' target site of one target segment within the target region to a 3' target site of another target segment within the same target region.

Targeting includes determination of at least one target segment to which an antisense compound hybridizes, such that a desired effect occurs. In certain embodiments, the desired effect is a reduction in mRNA target nucleic acid levels. In certain embodiments, the desired effect is reduction of levels of protein encoded by the target nucleic acid or a phenotypic change associated with the target nucleic acid.

A target region may contain one or more target segments. Multiple target segments within a target region may be overlapping. Alternatively, they may be non-overlapping. In certain embodiments, target segments within a target region are separated by no more than about 300 nucleotides. In certain emodiments, target segments within a target region are separated by a number of nucleotides that is, is about, is no more than, is no more than about, 250, 200, 150, 100, 90, 80, 70, 60, 50, 40, 30, 20, or 10 nucleotides on the target nucleic acid, or is a range defined by any two of the preceeding values. In certain embodiments, target segments within a target region are separated by no more than, or no more than about, 5 nucleotides on the target nucleic acid. In certain embodiments, target segments are contiguous. Contemplated are target regions defined by a range having a starting nucleic acid that is any of the 5' target sites or 3' target sites listed herein.

Suitable target segments may be found within a 5' UTR, a coding region, a 3' UTR, an intron, an exon, or an exon/intron junction. Target segments containing a start codon or a stop codon are also suitable target segments. A suitable target segment may specifcally exclude a certain structurally defined region such as the start codon or stop codon.

The determination of suitable target segments may include a comparison of the sequence of a target nucleic acid to other sequences throughout the genome. For example, the BLAST algorithm may be used to identify regions of similarity amongst different nucleic acids. This comparison can prevent the selection of antisense compound sequences that may hybridize in a non-specific manner to sequences other than a selected target nucleic acid (i.e., non-target or off-target sequences).

There may be variation in activity (e.g., as defined by percent reduction of target nucleic acid levels) of the antisense compounds within an active target region. In certain embodiments, reductions in MECP2 mRNA levels are indicative of inhibition of MECP2 expression. Reductions in levels of an MECP2 protein are also indicative of inhibition of target mRNA expression. Phenotypic changes are indicative of inhibition of MECP2 expression. Improvement in neurological function is indicative of inhibition of MECP2 expression. Improved motor function, activity, social behavior, and memory are indicative of inhibition of MECP2 expression. Reduction of anxiety-like behaviors is indicative of inhibition of MECP2 expression.

### Hybridization

In some embodiments, hybridization occurs between an antisense compound disclosed herein and an MECP2 nucleic acid. The most common mechanism of hybridization involves hydrogen bonding (e.g., Watson-Crick, Hoogsteen or reversed Hoogsteen hydrogen bonding) between complementary nucleobases of the nucleic acid molecules.

Hybridization can occur under varying conditions. Stringent conditions are sequence-dependent and are determined by the nature and composition of the nucleic acid molecules to be hybridized.

Methods of determining whether a sequence is specifically hybridizable to a target nucleic acid are well known in the art. In certain embodiments, the antisense compounds provided herein are specifically hybridizable with a MECP2 nucleic acid.

### Complementarity

An antisense compound and a target nucleic acid are complementary to each other when a sufficient number of nucleobases of the antisense compound can hydrogen bond with the corresponding nucleobases of the target nucleic acid, such that a desired effect will occur (e.g., antisense inhibition of a target nucleic acid, such as a MECP2 nucleic acid).

Non-complementary nucleobases between an antisense compound and a target nucleic acid may be tolerated provided that the antisense compound remains able to specifically hybridize to a target nucleic acid. Moreover, an antisense compound may hybridize over one or more segments of a target nucleic acid such that intervening or adjacent segments are not involved in the hybridization event (e.g., a loop structure, mismatch or hairpin structure).

In certain embodiments, the antisense compounds provided herein, or a specified portion thereof, are, or are at least, 70%, 80%, 85%, 86%, 87%, 88%, 89%, 90%, 91%, 92%, 93%, 94%, 95%, 96%, 97%, 98%, 99%, or 100% complementary to a MECP2 nucleic acid, a target region, target segment, or specified portion thereof. Percent complementarity of an antisense compound with a target nucleic acid can be determined using routine methods.

For example, an antisense compound in which 18 of 20 nucleobases of the antisense compound are complementary to a target region, and would therefore specifically hybridize, would represent 90 percent complementarity. In this example, the remaining noncomplementary nucleobases may be clustered or interspersed with complementary nucleobases and need not be contiguous to each other or to complementary nucleobases. As such, an antisense compound which is 18 nucleobases in length having 4 (four) noncomplementary nucleobases which are flanked by two regions of complete complementarity with the target nucleic acid would have 77.8% overall complementarity with the target nucleic acid and would thus fall within the scope of the present invention. Percent complementarity of an antisense compound with a region of a target nucleic acid can be determined routinely using BLAST programs (basic local alignment search tools) and PowerBLAST programs known in the art (Altschul et al., J. Mol. Biol., 1990, 215, 403 410; Zhang and Madden, Genome Res., 1997, 7, 649 656). Percent homology, sequence identity or complementarity, can be determined by, for example, the Gap program (Wisconsin Sequence Analysis Package, Version 8 for Unix, Genetics Computer Group, University Research Park, Madison Wis.), using default settings, which uses the algorithm of Smith and Waterman (Adv. Appl. Math., 1981, 2, 482 489).

In certain embodiments, the antisense compounds provided herein, or specified portions thereof, are fully complementary (*i.e.,* 100% complementary) to a target nucleic acid, or specified portion thereof. For example, an antisense compound may be fully complementary to a MECP2 nucleic acid, or a target region, or a target segment or target sequence thereof. As used herein, "fully complementary" means each nucleobase of an antisense compound is capable of precise base pairing with the corresponding nucleobases of a target nucleic acid. For example, a 20 nucleobase antisense compound is fully complementary to a target sequence that is 400 nucleobases long, so long as there is a corresponding 20 nucleobase portion of the target nucleic acid that is fully complementary to the antisense compound. Fully complementary can also be used in reference to a specified portion of the first and /or the second nucleic acid. For example, a 20 nucleobase portion of a 30 nucleobase antisense compound can be "fully complementary" to a target sequence that is 400 nucleobases long. The 20 nucleobase portion of the 30 nucleobase oligonucleotide is fully complementary to the target sequence if the target sequence has a corresponding 20 nucleobase portion wherein each nucleobase is complementary to the 20 nucleobase portion of the antisense compound. At the same time, the entire 30 nucleobase antisense compound may or may not be fully complementary to the target sequence, depending on whether the remaining 10 nucleobases of the antisense compound are also complementary to the target sequence.

The location of a non-complementary nucleobase may be at the 5' end or 3' end of the antisense compound. Alternatively, the non-complementary nucleobase or nucleobases may be at an internal position of the antisense compound. When two or more non-complementary nucleobases are present, they may be contiguous (i.e., linked) or non-contiguous. In one embodiment, a non-complementary nucleobase is located in the wing segment of a gapmer antisense oligonucleotide.

In certain embodiments, antisense compounds that are, or are up to 11, 12, 13, 14, 15, 16, 17, 18, 19, or 20 nucleobases in length comprise no more than 4, no more than 3, no more than 2, or no more than 1 non-complementary nucleobase(s) relative to a target nucleic acid, or specified portion thereof.

In certain embodiments, antisense compounds that are, or are up to 11, 12, 13, 14, 15, 16, 17, 18, 19, 20, 21, 22, 23, 24, 25, 26, 27, 28, 29, or 30 nucleobases in length comprise no more than 6, no more than 5, no more than 4, no more than 3, no more than 2, or no more than 1 non-complementary nucleobase(s) relative to a target nucleic acid, or specified portion thereof.

The antisense compounds provided herein also include those which are complementary to a portion of a target nucleic acid. As used herein, "portion" refers to a defined number of contiguous (i.e. linked) nucleobases within a region or segment of a target nucleic acid. A "portion" can also refer to a defined number of contiguous nucleobases of an antisense compound. In certain embodiments, the antisense compounds, are complementary to at least an 8 nucleobase portion of a target segment. In certain embodiments, the antisense compounds are complementary to at least a 9 nucleobase portion of a target segment. In certain embodiments, the antisense compounds are complementary to at least a 10 nucleobase portion of a target segment. In certain embodiments, the antisense compounds, are complementary to at least an 11 nucleobase portion of a target segment. In certain embodiments, the antisense compounds, are complementary to at least a 12 nucleobase portion of a target segment. In certain embodiments, the antisense compounds, are complementary to at least a 13 nucleobase portion of a target segment. In certain embodiments, the antisense compounds, are complementary to at least a 14 nucleobase portion of a target segment. In certain embodiments, the antisense compounds, are complementary to at least a 15 nucleobase portion of a target segment. Also contemplated are antisense compounds that are complementary to at least a 9, 10, 11, 12, 13, 14, 15, 16, 17, 18, 19, 20, or more nucleobase portion of a target segment, or a range defined by any two of these values.

### Identity

The antisense compounds provided herein may also have a defined percent identity to a particular nucleotide sequence, SEQ ID NO, or compound represented by a specific Isis number, or portion thereof. As used herein, an antisense compound is identical to the sequence disclosed herein if it has the same nucleobase pairing ability. For example, a RNA which contains uracil in place of thymidine in a disclosed DNA sequence would be considered identical to the DNA sequence since both uracil and thymidine pair with adenine. Shortened and lengthened versions of the antisense compounds described herein as well as compounds having non-identical bases relative to the antisense compounds provided herein also are contemplated. The non-identical bases may be adjacent to each other or dispersed throughout the antisense compound. Percent identity of an antisense compound is calculated according to the number of bases that have identical base pairing relative to the sequence to which it is being compared.

In certain embodiments, the antisense compounds, or portions thereof, are at least 70%, 75%, 80%, 85%, 90%, 95%, 96%, 97%, 98%, 99% or 100% identical to one or more of the antisense compounds or SEQ ID NOs, or a portion thereof, disclosed herein.

In certain embodiments, a portion of the antisense compound is compared to an equal length portion of the target nucleic acid. In certain embodiments, an 8, 9, 10, 11, 12, 13, 14, 15, 16, 17, 18, 19, 20, 21, 22, 23, 24, or 25 nucleobase portion is compared to an equal length portion of the target nucleic acid.

In certain embodiments, a portion of the antisense oligonucleotide is compared to an equal length portion of the target nucleic acid. In certain embodiments, an 8, 9, 10, 11, 12, 13, 14, 15, 16, 17, 18, 19, 20, 21, 22, 23, 24, or 25 nucleobase portion is compared to an equal length portion of the target nucleic acid.

### Modifications

A nucleoside is a base-sugar combination. The nucleobase (also known as base) portion of the nucleoside is normally a heterocyclic base moiety. Nucleotides are nucleosides that further include a phosphate group covalently linked to the sugar portion of the nucleoside. For those nucleosides that include a pentofuranosyl sugar, the phosphate group can be linked to the 2', 3' or 5' hydroxyl moiety of the sugar. Oligonucleotides are formed through the covalent linkage of adjacent nucleosides to one another, to form a linear polymeric oligonucleotide. Within the oligonucleotide structure, the phosphate groups are commonly referred to as forming the internucleoside linkages of the oligonucleotide.

Modifications to antisense compounds encompass substitutions or changes to internucleoside linkages, sugar moieties, or nucleobases. Modified antisense compounds are often preferred over native forms because of desirable properties such as, for example, enhanced cellular uptake, enhanced affinity for nucleic acid target, increased stability in the presence of nucleases, or increased inhibitory activity.

Chemically modified nucleosides may also be employed to increase the binding affinity of a shortened or truncated antisense oligonucleotide for its target nucleic acid. Consequently, comparable results can often be obtained with shorter antisense compounds that have such chemically modified nucleosides.

### Modified Internucleoside Linkages

The naturally occuring internucleoside linkage of RNA and DNA is a 3' to 5' phosphodiester linkage. Antisense compounds having one or more modified, i.e. non-naturally occurring, internucleoside linkages are often selected over antisense compounds having naturally occurring internucleoside linkages because of desirable properties such as, for example, enhanced cellular uptake, enhanced affinity for target nucleic acids, and increased stability in the presence of nucleases.

Oligonucleotides having modified internucleoside linkages include internucleoside linkages that retain a phosphorus atom as well as internucleoside linkages that do not have a phosphorus atom. Representative phosphorus containing internucleoside linkages include, but are not limited to, phosphodiesters, phosphotriesters, methylphosphonates, phosphoramidate, and phosphorothioates. Methods of preparation of phosphorous-containing and non-phosphorous-containing linkages are well known.

In certain embodiments, antisense compounds targeted to a MECP2 nucleic acid comprise one or more modified internucleoside linkages. In certain embodiments, the modified internucleoside linkages are interspersed throughout the antisense compound. In certain embodiments, the modified internucleoside linkages are phosphorothioate linkages. In certain embodiments, each internucleoside linkage of an antisense compound is a phosphorothioate internucleoside linkage.

### Modified Sugar Moieties

Antisense compounds can optionally contain one or more nucleosides wherein the sugar group has been modified. Such sugar modified nucleosides may impart enhanced nuclease stability, increased binding affinity, or some other beneficial biological property to the antisense compounds. In certain embodiments, nucleosides comprise chemically modified ribofuranose ring moieties. Examples of chemically modified ribofuranose rings include without limitation, addition of substitutent groups (including 5' and 2' substituent groups, bridging of non-geminal ring atoms to form bicyclic nucleic acids (BNA), replacement of the ribosyl ring oxygen atom with S, N(R), or C(R₁)(R₂) (R, R₁ and R₂ are each independently H, C₁-C₁₂ alkyl or a protecting group) and combinations thereof. Examples of chemically modified sugars include 2'-F-5'-methyl substituted nucleoside (see PCT International Application WO 2008/101157 Published on 8/21/08 for other disclosed 5',2'-bis substituted nucleosides) or replacement of the ribosyl ring oxygen atom with S with further substitution at the 2'-position (see published U.S. Patent Application US2005-0130923, published on June 16, 2005) or alternatively 5'-substitution of a BNA (see PCT International Application WO 2007/134181 Published on 11/22/07 wherein LNA is substituted with for example a 5'-methyl or a 5'-vinyl group).

Examples of nucleosides having modified sugar moieties include without limitation nucleosides comprising 5'-vinyl, 5'-methyl (R or S), 4'-S, 2'-F, 2'-OCH₃, 2'-OCH₂CH₃, 2'-OCH₂CH₂F and 2'-O(CH₂)₂OCH₃ substituent groups. The substituent at the 2' position can also be selected from allyl, amino, azido, thio, O-allyl, O-C₁-C₁₀ alkyl, OCF₃, OCH₂F, O(CH₂)₂SCH₃, O(CH₂)₂-O-N(Rₘ)(Rₙ), O-CH₂-C(=O)-N(Rₘ)(Rₙ), and O-CH₂-C(=O)-N(R₁)-(CH₂)₂-N(Rₘ)(Rₙ), where each R₁, Rₘ and Rₙ is, independently, H or substituted or unsubstituted C₁-C₁₀ alkyl.

As used herein, "bicyclic nucleosides" refer to modified nucleosides comprising a bicyclic sugar moiety. Examples of bicyclic nucleosides include without limitation nucleosides comprising a bridge between the 4' and the 2' ribosyl ring atoms. In certain embodiments, antisense compounds provided herein include one or more bicyclic nucleosides comprising a 4' to 2' bridge. Examples of such 4' to 2' bridged bicyclic nucleosides, include but are not limited to one of the formulae: 4'-(CH₂)-O-2' (LNA); 4'-(CH₂)-S-2'; 4'-(CH₂)₂-O-2' (ENA); 4'-CH(CH₃)-O-2' and 4'-CH(CH₂OCH₃)-O-2' (and analogs thereof see U.S. Patent 7,399,845, issued on July 15, 2008); 4'-C(CH₃)(CH₃)-O-2' (and analogs thereof see published International Application WO/2009/006478, published January 8, 2009); 4'-CH₂-N(OCH₃)-2' (and analogs thereof see published International Application WO/2008/150729, published December 11, 2008); 4'-CH₂-O-N(CH₃)-2' (see published U.S. Patent Application US2004-0171570, published September 2, 2004 ); 4'-CH₂-N(R)-O-2', wherein R is H, C₁-C₁₂ alkyl, or a protecting group (see U.S. Patent 7,427,672, issued on September 23, 2008); 4'-CH₂-C(H)(CH₃)-2' (see Chattopadhyaya et al., J. Org. Chem., 2009, 74, 118-134); and 4'-CH₂-C-(=CH₂)-2' (and analogs thereof see published International Application WO 2008/154401, published on December 8, 2008).

Further reports related to bicyclic nucleosides can also be found in published literature (see for example: Singh et al., Chem. Commun., 1998, 4, 455-456; Koshkin et al., Tetrahedron, 1998, 54, 3607-3630; Wahlestedt et al., Proc. Natl. Acad. Sci. U. S. A., 2000, 97, 5633-5638; Kumar et al., Bioorg. Med. Chem. Lett., 1998, 8, 2219-2222; Singh et al., J. Org. Chem., 1998, 63, 10035-10039; Srivastava et al., J. Am. Chem. Soc., 2007, 129(26) 8362-8379; Elayadi et al., Curr. Opinion Invest. Drugs, 2001, 2, 558-561; Braasch et al., Chem. Biol., 2001, 8, 1-7; and Orum et al., Curr. Opinion Mol. Ther., 2001, 3, 239-243; U.S. Patent Nos. 6,268,490; 6,525,191; 6,670,461; 6,770,748; 6,794,499; 7,034,133; 7,053,207; 7,399,845; 7,547,684; and 7,696,345; U.S. Patent Publication No. US2008-0039618; US2009-0012281; U.S. Patent Serial Nos. 60/989,574; 61/026,995; 61/026,998; 61/056,564; 61/086,231; 61/097,787; and 61/099,844; Published PCT International applications WO 1994/014226; WO 2004/106356; WO 2005/021570; WO 2007/134181; WO 2008/150729; WO 2008/154401; and WO 2009/006478. Each of the foregoing bicyclic nucleosides can be prepared having one or more stereochemical sugar configurations including for example a-L-ribofuranose and β-D-ribofuranose (see PCT international application PCT/DK98/00393, published on March 25, 1999 as WO 99/14226).

In certain embodiments, bicyclic sugar moieties of BNA nucleosides include, but are not limited to, compounds having at least one bridge between the 4' and the 2' position of the pentofuranosyl sugar moiety wherein such bridges independently comprises 1 or from 2 to 4 linked groups independently selected from - [C(Rₐ)(R_{b})]ₙ-, -C(Rₐ)=C(R_{b})-, -C(Rₐ)=N-, -C(=O)-, -C(=NRₐ)-, -C(=S)-, -O-, -Si(Rₐ)₂-, -S(=O)ₓ-, and -N(Rₐ)-; wherein:
x is 0, 1, or 2;
n is 1, 2, 3, or 4;
each Rₐ and R_{b} is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.

In certain embodiments, the bridge of a bicyclic sugar moiety is -[C(Rₐ)(R_{b})]ₙ-, -[C(Rₐ)(R_{b})]ₙ-O-, -C(RₐR_{b})-N(R)-O- or -C(RₐR_{b})-O-N(R)-. In certain embodiments, the bridge is 4'-CH₂-2', 4'-(CH₂)₂-2', 4'-(CH₂)₃-2', 4'-CH₂-O-2', 4'-(CH₂)₂-O-2', 4'-CH₂-O-N(R)-2' and 4'-CH₂-N(R)-O-2'- wherein each Ris, independently, H, a protecting group or C₁-C₁₂ alkyl.

In certain embodiments, bicyclic nucleosides are further defined by isomeric configuration. For example, a nucleoside comprising a 4'-2' methylene-oxy bridge, may be in the a-L configuration or in the β-D configuration. Previously, a-L-methyleneoxy (4'-CH₂-O-2') BNA's have been incorporated into antisense oligonucleotides that showed antisense activity (Frieden et al., Nucleic Acids Research, 2003, 21, 6365-6372).

In certain embodiments, bicyclic nucleosides include, but are not limited to, (A) a-L-methyleneoxy (4'-CH₂-O-2') BNA , (B) β-D-methyleneoxy (4'-CH₂-O-2') BNA , (C) ethyleneoxy (4'-(CH₂)₂-O-2') BNA , (D) aminooxy (4'-CH₂-O-N(R)-2') BNA, (E) oxyamino (4'-CH₂-N(R)-O-2') BNA, and (F) methyl(methyleneoxy) (4'-CH(CH₃)-O-2') BNA, (G) methylene-thio (4'-CH₂-S-2') BNA, (H) methylene-amino (4'-CH₂-N(R)-2') BNA, (I) methyl carbocyclic (4'-CH₂-CH(CH₃)-2') BNA, and (J) propylene carbocyclic (4'-(CH₂)₃-2') BNA as depicted below. 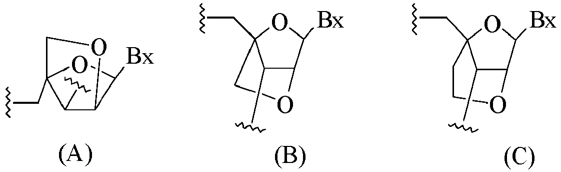 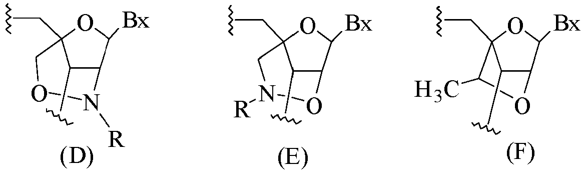 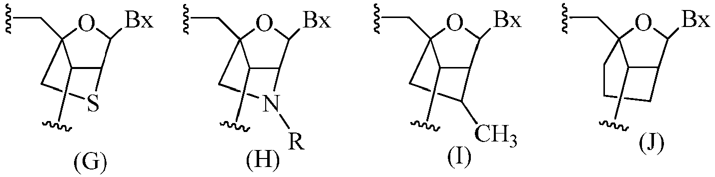 wherein Bx is the base moiety and R is independently H, a protecting group or C₁-C₁₂ alkyl.

In certain embodiments, bicyclic nucleosides are provided having Formula I: 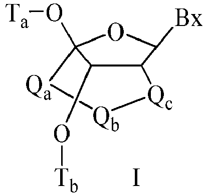 wherein:
Bx is a heterocyclic base moiety;
-Qₐ-Q_{b}-Q_{c}- is -CH₂-N(R_{c})-CH₂-, -C(=O)-N(R_{c})-CH₂-, -CH₂-O-N(R_{c})-, -CH₂-N(R_{c})-O- or -N(R_{c})-O-CH₂;
R_{c} is C₁-C₁₂ alkyl or an amino protecting group; and
Tₐ and T_{b} are each, independently H, a hydroxyl protecting group, a conjugate group, a reactive phosphorus group, a phosphorus moiety or a covalent attachment to a support medium.

In certain embodiments, bicyclic nucleosides are provided having Formula II: 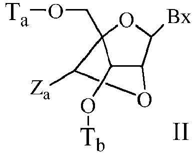 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Zₐ is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl, acyl, substituted acyl, substituted amide, thiol or substituted thio.

In one embodiment, each of the substituted groups is, independently, mono or poly substituted with substituent groups independently selected from halogen, oxo, hydroxyl, OJ_{c}, NJ_{c}J_{d} SJ_{c}, N₃, OC(=X)J_{c}, and NJₑC(=X)NJ_{c}J_{d}, wherein each J_{c}, J_{d} and Jₑ is, independently, H, C₁-C₆ alkyl, or substituted C₁-C₆ alkyl and X is O or NJ_{c}.

In certain embodiments, bicyclic nucleosides are provided having Formula III: 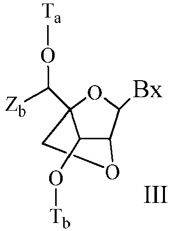 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Z_{b} is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl or substituted acyl (C(=O)-).

In certain embodiments, bicyclic nucleosides are provided having Formula IV: 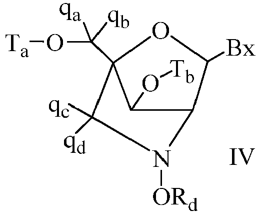 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
R_{d} is C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl;
each qₐ, q_{b}, q_{c} and q_{d} is, independently, H, halogen, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl, C₁-C₆ alkoxyl, substituted C₁-C₆ alkoxyl, acyl, substituted acyl, C₁-C₆ aminoalkyl or substituted C₁-C₆ aminoalkyl;

In certain embodiments, bicyclic nucleosides are provided having Formula V: 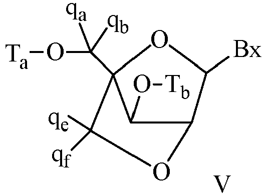 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
qₐ, q_{b}, qₑ and q_{f} are each, independently, hydrogen, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxy, substituted C₁-C₁₂ alkoxy, OJⱼ, SJⱼ, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖ or N(H)C(=S)NJⱼJₖ;
or qₑ and q_{f} together are =C(q_{g})(qₕ);
q_{g} and qₕ are each, independently, H, halogen, C₁-C₁₂ alkyl or substituted C₁-C₁₂ alkyl.

The synthesis and preparation of the methyleneoxy (4'-CH₂-O-2') BNA monomers adenine, cytosine, guanine, 5-methyl-cytosine, thymine and uracil, along with their oligomerization, and nucleic acid recognition properties have been described (Koshkin et al., Tetrahedron, 1998, 54, 3607-3630). BNAs and preparation thereof are also described in WO 98/39352 and WO 99/14226.

Analogs of methyleneoxy (4'-CH₂-O-2') BNA and 2'-thio-BNAs, have also been prepared (Kumar et al., Bioorg. Med. Chem. Lett., 1998, 8, 2219-2222). Preparation of locked nucleoside analogs comprising oligodeoxyribonucleotide duplexes as substrates for nucleic acid polymerases has also been described (Wengel et al., WO 99/14226 ). Furthermore, synthesis of 2'-amino-BNA, a novel comformationally restricted high-affinity oligonucleotide analog has been described in the art (Singh et al., J. Org. Chem., 1998, 63, 10035-10039). In addition, 2'-amino- and 2'-methylamino-BNA's have been prepared and the thermal stability of their duplexes with complementary RNA and DNA strands has been previously reported.

In certain embodiments, bicyclic nucleosides are provided having Formula VI: 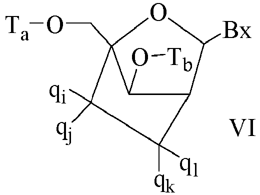 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
each qᵢ, qⱼ, qₖ and q₁ is, independently, H, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxyl, substituted C₁-C₁₂ alkoxyl, OJⱼ, SJⱼ, SOJⱼ, SO₂Jⱼ, NJⱼJₖ, N₃, CN, C(=O)OJⱼ C(=O)NJⱼJₖ, C(=O)Jⱼ, O-C(=O)NJⱼJₖ, N(H)C(=NH)NJⱼJₖ, N(H)C(=O)NJⱼJₖ or N(H)C(=S)NJⱼJₖ; and
qᵢ and qⱼ or q₁ and qₖ together are =C(q_{g})(qₕ), wherein q_{g} and qₕ are each, independently, H, halogen, C₁-C₁₂ alkyl or substituted C₁-C₁₂ alkyl.

One carbocyclic bicyclic nucleoside having a 4'-(CH₂)₃-2' bridge and the alkenyl analog bridge 4'-CH=CH-CH₂-2' have been described (Freier et al., Nucleic Acids Research, 1997, 25(22), 4429-4443 and Albaek et al., J. Org. Chem., 2006, 71, 7731-7740). The synthesis and preparation of carbocyclic bicyclic nucleosides along with their oligomerization and biochemical studies have also been described (Srivastava et al., J. Am. Chem. Soc., 2007,129(26), 8362-8379).

As used herein, "4'-2' bicyclic nucleoside" or "4' to 2' bicyclic nucleoside" refers to a bicyclic nucleoside comprising a furanose ring comprising a bridge connecting two carbon atoms of the furanose ring connects the 2' carbon atom and the 4' carbon atom of the sugar ring.

As used herein, "monocylic nucleosides" refer to nucleosides comprising modified sugar moieties that are not bicyclic sugar moieties. In certain embodiments, the sugar moiety, or sugar moiety analogue, of a nucleoside may be modified or substituted at any position.

As used herein, "2'-modified sugar" means a furanosyl sugar modified at the 2' position. In certain embodiments, such modifications include substituents selected from: a halide, including, but not limited to substituted and unsubstituted alkoxy, substituted and unsubstituted thioalkyl, substituted and unsubstituted amino alkyl, substituted and unsubstituted alkyl, substituted and unsubstituted allyl, and substituted and unsubstituted alkynyl. In certain embodiments, 2' modifications are selected from substituents including, but not limited to: O[(CH₂)ₙO]ₘCH₃, O(CH₂)ₙNH₂, O(CH₂)ₙCH₃, O(CH₂)ₙF, O(CH₂)ₙONH₂, OCH₂C(=O)N(H)CH₃, and O(CH₂)ₙON[(CH₂)ₙCH₃]₂, where n and m are from 1 to about 10. Other 2'-substituent groups can also be selected from: C₁-C₁₂ alkyl, substituted alkyl, alkenyl, alkynyl, alkaryl, aralkyl, O-alkaryl or O-aralkyl, SH, SCH₃, OCN, Cl, Br, CN, F, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving pharmacokinetic properties, or a group for improving the pharmacodynamic properties of an antisense compound, and other substituents having similar properties. In certain embodiments, modifed nucleosides comprise a 2'-MOE side chain (Baker et al., J. Biol. Chem., 1997, 272, 11944-12000). Such 2'-MOE substitution have been described as having improved binding affinity compared to unmodified nucleosides and to other modified nucleosides, such as 2'- *O-*methyl, O-propyl, and O-aminopropyl. Oligonucleotides having the 2'-MOE substituent also have been shown to be antisense inhibitors of gene expression with promising features for *in vivo* use (Martin, Helv. Chim. Acta, 1995, 78, 486-504; Altmann et al., Chimia, 1996, 50, 168-176; Altmann et al., Biochem. Soc. Trans., 1996, 24, 630-637; and Altmann et al., Nucleosides Nucleotides, 1997, 16, 917-926).

As used herein, a "modified tetrahydropyran nucleoside" or "modified THP nucleoside" means a nucleoside having a six-membered tetrahydropyran "sugar" substituted in for the pentofuranosyl residue in normal nucleosides (a sugar surrogate). Modified THP nucleosides include, but are not limited to, what is referred to in the art as hexitol nucleic acid (HNA), anitol nucleic acid (ANA), manitol nucleic acid (MNA) (see Leumann, Bioorg. Med. Chem., 2002, 10, 841-854), fluoro HNA (F-HNA) or those compounds having Formula VII: 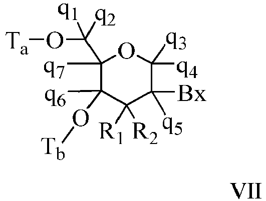 wherein independently for each of said at least one tetrahydropyran nucleoside analog of Formula VII:
Bx is a heterocyclic base moiety;
Tₐ and T_{b} are each, independently, an internucleoside linking group linking the tetrahydropyran nucleoside analog to the antisense compound or one of Tₐ and T_{b} is an internucleoside linking group linking the tetrahydropyran nucleoside analog to the antisense compound and the other of Tₐ and T_{b} is H, a hydroxyl protecting group, a linked conjugate group or a 5' or 3'-terminal group;
q₁, q₂, q₃, q₄, q₅, q₆ and q₇ are each independently, H, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl; and each of R₁ and R₂ is selected from hydrogen, hydroxyl, halogen, substituted or unsubstituted alkoxy, NJ₁J₂, SJ₁, N₃, OC(=X)J₁, OC(=X)NJ₁J₂, NJ₃C(=X)NJ₁J₂ and CN, wherein X is O, S or NJ₁ and each J₁, J₂ and J₃ is, independently, H or C₁-C₆ alkyl.

In certain embodiments, the modified THP nucleosides of Formula VII are provided wherein q₁, q₂, q₃, q₄, q₅, q₆ and q₇ are each H. In certain embodiments, at least one of q₁ q₂, q₃, q₄, q₅, q₆ and q₇ is other than H. In certain embodiments, at least one of q₁, q₂, q₃, q₄, q₅, q₆ and q₇ is methyl. In certain embodiments, THP nucleosides of Formula VII are provided wherein one of R₂ and R₂ is fluoro. In certain embodiments, R₁ is fluoro and R₂ is H; R₁ is methoxy and R₂ is H, and R₁ is H and R₂ is methoxyethoxy.

As used herein, "2'-modified" or "2'-substituted" refers to a nucleoside comprising a sugar comprising a substituent at the 2' position other than H or OH. 2'-modified nucleosides, include, but are not limited to, bicyclic nucleosides wherein the bridge connecting two carbon atoms of the sugar ring connects the 2' carbon and another carbon of the sugar ring; and nucleosides with non-bridging 2'substituents, such as allyl, amino, azido, thio, O-allyl, O-C₁-C₁₀ alkyl, -OCF₃, O-(CH₂)₂-O-CH₃, 2'-O(CH₂)₂SCH₃, O-(CH₂)₂-O-N(Rₘ)(Rₙ), or O-CH₂-C(=O)-N(Rₘ)(Rₙ), where each Rₘ and Rₙ is, independently, H or substituted or unsubstituted C₁-C₁₀ alkyl. 2'-modifed nucleosides may further comprise other modifications, for example at other positions of the sugar and/or at the nucleobase.

As used herein, "2'-F" refers to a nucleoside comprising a sugar comprising a fluoro group at the 2' position.

As used herein, "2'-OMe" or "2'-OCH₃" or "2'-O-methyl" each refers to a nucleoside comprising a sugar comprising an -OCH₃ group at the 2' position of the sugar ring.

As used herein, "MOE" or "2'-MOE" or "2'-OCH₂CH₂OCH₃" or "2'-O-methoxyethyl" each refers to a nucleoside comprising a sugar comprising a -OCH₂CH₂OCH₃ group at the 2' position of the sugar ring.

As used herein, "oligonucleotide" refers to a compound comprising a plurality of linked nucleosides. In certain embodiments, one or more of the plurality of nucleosides is modified. In certain embodiments, an oligonucleotide comprises one or more ribonucleosides (RNA) and/or deoxyribonucleosides (DNA).

Many other bicyclo and tricyclo sugar surrogate ring systems are also known in the art that can be used to modify nucleosides for incorporation into antisense compounds (see for example review article: Leumann, Bioorg. Med. Chem., 2002, 10, 841-854).
Such ring systems can undergo various additional substitutions to enhance activity.

Methods for the preparations of modified sugars are well known to those skilled in the art.

In nucleotides having modified sugar moieties, the nucleobase moieties (natural, modified or a combination thereof) are maintained for hybridization with an appropriate nucleic acid target.

In certain embodiments, antisense compounds comprise one or more nucleosides having modified sugar moieties. In certain embodiments, the modified sugar moiety is 2'-MOE. In certain embodiments, the 2'-MOE modified nucleosides are arranged in a gapmer motif. In certain embodiments, the modified sugar moiety is a bicyclic nucleoside having a (4'-CH(CH₃)-O-2') bridging group. In certain embodiments, the (4'-CH(CH₃)-O-2') modified nucleosides are arranged throughout the wings of a gapmer motif.

### Compositions and Methods for Formulating Pharmaceutical Compositions

Antisense oligonucleotides may be admixed with pharmaceutically acceptable active or inert substances for the preparation of pharmaceutical compositions or formulations. Compositions and methods for the formulation of pharmaceutical compositions are dependent upon a number of criteria, including, but not limited to, route of administration, extent of disorder, or dose to be administered.

An antisense compound targeted to a MECP2 nucleic acid can be utilized in pharmaceutical compositions by combining the antisense compound with a suitable pharmaceutically acceptable diluent or carrier. A pharmaceutically acceptable diluent includes phosphate-buffered saline (PBS). PBS is a diluent suitable for use in compositions to be delivered parenterally. Accordingly, in one embodiment, employed in the methods described herein is a pharmaceutical composition comprising an antisense compound targeted to a MECP2 nucleic acid and a pharmaceutically acceptable diluent. In certain embodiments, the pharmaceutically acceptable diluent is PBS. In certain embodiments, the antisense compound is an antisense oligonucleotide.

Pharmaceutical compositions comprising antisense compounds encompass any pharmaceutically acceptable salts, esters, or salts of such esters, or any other oligonucleotide which, upon administration to an animal, including a human, is capable of providing (directly or indirectly) the biologically active metabolite or residue thereof. Accordingly, for example, the disclosure is also drawn to pharmaceutically acceptable salts of antisense compounds, prodrugs, pharmaceutically acceptable salts of such prodrugs, and other bioequivalents. Suitable pharmaceutically acceptable salts include, but are not limited to, sodium and potassium salts.

A prodrug can include the incorporation of additional nucleosides at one or both ends of an antisense compound which are cleaved by endogenous nucleases within the body, to form the active antisense compound.

### Conjugated Antisense Compounds

Antisense compounds may be covalently linked to one or more moieties or conjugates which enhance the activity, cellular distribution or cellular uptake of the resulting antisense oligonucleotides. Typical conjugate groups include cholesterol moieties and lipid moieties. Additional conjugate groups include carbohydrates, phospholipids, biotin, phenazine, folate, phenanthridine, anthraquinone, acridine, fluoresceins, rhodamines, coumarins, and dyes.

Antisense compounds can also be modified to have one or more stabilizing groups that are generally attached to one or both termini of antisense compounds to enhance properties such as, for example, nuclease stability. Included in stabilizing groups are cap structures. These terminal modifications protect the antisense compound having terminal nucleic acid from exonuclease degradation, and can help in delivery and/or localization within a cell. The cap can be present at the 5'-terminus (5'-cap), or at the 3'-terminus (3'-cap), or can be present on both termini. Cap structures are well known in the art and include, for example, inverted deoxy abasic caps. Further 3' and 5'-stabilizing groups that can be used to cap one or both ends of an antisense compound to impart nuclease stability include those disclosed in WO 03/004602 published on January 16, 2003.

### Cell culture and antisense compounds treatment

The effects of antisense compounds on the level, activity or expression of MECP2 nucleic acids can be tested *in vitro* in a variety of cell types. Cell types used for such analyses are available from commerical vendors (e.g. American Type Culture Collection, Manassus, VA; Zen-Bio, Inc., Research Triangle Park, NC; Clonetics Corporation, Walkersville, MD) and are cultured according to the vendor's instructions using commercially available reagents (e.g. Invitrogen Life Technologies, Carlsbad, CA). Illustrative cell types include, but are not limited to, HepG2 cells, Hep3B cells, and primary hepatocytes. In certain embodiments, cells are patient cells, such as B-lymphoblast cells.

### In vitro testing of antisense oligonucleotides

Described herein are methods for treatment of cells with antisense oligonucleotides, which can be modified appropriately for treatment with other antisense compounds.

Cells may be treated with antisense oligonucleotides when the cells reach approximately 60-80% confluency in culture.

One reagent commonly used to introduce antisense oligonucleotides into cultured cells includes the cationic lipid transfection reagent LIPOFECTIN (Invitrogen, Carlsbad, CA). Antisense oligonucleotides may be mixed with LIPOFECTIN in OPTI-MEM 1 (Invitrogen, Carlsbad, CA) to achieve the desired final concentration of antisense oligonucleotide and a LIPOFECTIN concentration that may range from 2 to 12 ug/mL per 100 nM antisense oligonucleotide.

Another reagent used to introduce antisense oligonucleotides into cultured cells includes LIPOFECTAMINE (Invitrogen, Carlsbad, CA). Antisense oligonucleotide is mixed with LIPOFECTAMINE in OPTI-MEM 1 reduced serum medium (Invitrogen, Carlsbad, CA) to achieve the desired concentration of antisense oligonucleotide and a LIPOFECTAMINE concentration that may range from 2 to 12 ug/mL per 100 nM antisense oligonucleotide.

Another reagent used to introduce antisense oligonucleotides into cultured cells includes TURBOFECT (Thermo Scientific, Carlsbad, CA).

Another technique used to introduce antisense oligonucleotides into cultured cells includes electroporation.

Cells are treated with antisense oligonucleotides by routine methods. Cells may be harvested 16-24 hours after antisense oligonucleotide treatment, at which time RNA or protein levels of target nucleic acids are measured by methods known in the art and described herein. In general, when treatments are performed in multiple replicates, the data are presented as the average of the replicate treatments.

The concentration of antisense oligonucleotide used varies from cell line to cell line. Methods to determine the optimal antisense oligonucleotide concentration for a particular cell line are well known in the art. Antisense oligonucleotides are typically used at concentrations ranging from 1 nM to 300 nM when transfected with LIPOFECTAMINE. Antisense oligonucleotides are used at higher concentrations ranging from 625 to 20,000 nM when transfected using electroporation.

### RNA Isolation

RNA analysis can be performed on total cellular RNA or poly(A)+ mRNA. Methods of RNA isolation are well known in the art. RNA is prepared using methods well known in the art, for example, using the TRIZOL Reagent (Invitrogen, Carlsbad, CA) according to the manufacturer's recommended protocols.

### Analysis of inhibition of target levels or expression

Inhibition of levels or expression of a MECP2 nucleic acid can be assayed in a variety of ways known in the art. For example, target nucleic acid levels can be quantitated by, e.g., Northern blot analysis, competitive polymerase chain reaction (PCR), or quantitaive real-time PCR. RNA analysis can be performed on total cellular RNA or poly(A)+ mRNA. Methods of RNA isolation are well known in the art. Northern blot analysis is also routine in the art. Quantitative real-time PCR can be conveniently accomplished using the commercially available ABI PRISM 7600, 7700, or 7900 Sequence Detection System, available from PE-Applied Biosystems, Foster City, CA and used according to manufacturer's instructions.

### Quantitative Real-Time PCR Analysis of Target RNA Levels

Quantitation of target RNA levels may be accomplished by quantitative real-time PCR using the ABI PRISM 7600, 7700, or 7900 Sequence Detection System (PE-Applied Biosystems, Foster City, CA) according to manufacturer's instructions. Methods of quantitative real-time PCR are well known in the art.

Prior to real-time PCR, the isolated RNA is subjected to a reverse transcriptase (RT) reaction, which produces complementary DNA (cDNA) that is then used as the substrate for the real-time PCR amplification. The RT and real-time PCR reactions are performed sequentially in the same sample well. RT and real-time PCR reagents may be obtained from Invitrogen (Carlsbad, CA). RT real-time-PCR reactions are carried out by methods well known to those skilled in the art.

Gene (or RNA) target quantities obtained by real time PCR are normalized using either the expression level of a gene whose expression is constant, such as cyclophilin A, or by quantifying total RNA using RIBOGREEN (Invitrogen, Inc. Carlsbad, CA). Cyclophilin A expression is quantified by real time PCR, by being run simultaneously with the target, multiplexing, or separately. Total RNA is quantified using RIBOGREEN RNA quantification reagent (Invetrogen, Inc. Eugene, OR). Methods of RNA quantification by RIBOGREEN are taught in Jones, L.J., et al, (Analytical Biochemistry, 1998, 265, 368-374). A CYTOFLUOR 4000 instrument (PE Applied Biosystems) is used to measure RIBOGREEN fluorescence.

Probes and primers are designed to hybridize to a MECP2 nucleic acid. Methods for designing real-time PCR probes and primers are well known in the art, and may include the use of software such as PRIMER EXPRESS Software (Applied Biosystems, Foster City, CA).

### Analysis of Protein Levels

Antisense inhibition of MECP2 nucleic acids can be assessed by measuring MECP2 protein levels. Protein levels of MECP2 can be evaluated or quantitated in a variety of ways well known in the art, such as immunoprecipitation, Western blot analysis (immunoblotting), enzyme-linked immunosorbent assay (ELISA), quantitative protein assays, protein activity assays (for example, caspase activity assays), immunohistochemistry, immunocytochemistry or fluorescence-activated cell sorting (FACS). Antibodies directed to a target can be identified and obtained from a variety of sources, such as the MSRS catalog of antibodies (Aerie Corporation, Birmingham, MI), or can be prepared via conventional monoclonal or polyclonal antibody generation methods well known in the art.

### In vivo testing of antisense compounds

Antisense compounds, for example, antisense oligonucleotides, are tested in animals to assess their ability to inhibit expression of MECP2 and produce phenotypic changes, such as, improved behavior, motor function, and cognition. In certain embodiments, motor function is measured by walking initiation analysis, rotarod, grip strength, pole climb, open field performance, balance beam, hindpaw footprint testing in the animal. In certain embodiments, behavior is measured by elevated plus maze and three-chamber social interaction. Testing may be performed in normal animals, or in experimental models. For administration to animals, antisense oligonucleotides are formulated in a pharmaceutically acceptable diluent, such as phosphate-buffered saline. Administration includes parenteral routes of administration, such as intraperitoneal, intravenous, and subcutaneous. Calculation of antisense oligonucleotide dosage and dosing frequency is within the abilities of those skilled in the art, and depends upon factors such as route of administration and animal body weight. Following a period of treatment with antisense oligonucleotides, RNA is isolated from CNS tissue or CSF and changes in MECP2 nucleic acid expression are measured.

### Certain Indications

Disclosed herein are methods of treating an individual comprising administering one or more pharmaceutical compositions described herein. The individual may have a neurological disorder. The individual may be at risk for developing a neurological disorder, including, but not limited to, MECP2 duplication syndrome. The invention provides an antisense compound complementary to a MECP2 nucleic acid for use in treating MECP2 duplication syndrome In certain embodiments, the individual has been identified as having a MECP2 associated disorder. Disclosed herein are methods for prophylactically reducing MECP2 expression in an individual. Certain embodiments include administering to an individual a therapeutically effective amount of an antisense compound targeted to a MECP2 nucleic acid.

In one embodiment, administration of a therapeutically effective amount of an antisense compound targeted to a MECP2 nucleic acid is accompanied by monitoring of MECP2 levels in an individual, to determine an individual's response to administration of the antisense compound. An individual's response to administration of the antisense compound may be used by a physician to determine the amount and duration of therapeutic intervention.

In certain embodiments, administration of an antisense compound targeted to a MECP2 nucleic acid results in reduction of MECP2 mRNA and or protein expression by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in improved motor function in an animal. In certain embodiments, administration of a MECP2 antisense compound improves motor function by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in improved anxiety in an animal. In certain embodiments, administration of a MECP2 antisense compound improves anxiety by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in improved social interaction in an animal. In certain embodiments, administration of a MECP2 antisense compound improves social interaction by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in improved activity in an animal. In certain embodiments, administration of a MECP2 antisense compound improves activity by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in reduction of seizures. In certain embodiments, administration of a MECP2 antisense compound reduces seizures by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in normalized EEG discharges.

Pharmaceutical compositions comprising an antisense compound targeted to MECP2 may be used for the preparation of a medicament for treating a patient suffering or susceptible to a neurological disorder including MECP2 duplication syndrome.

### EXAMPLES

### Non-limiting disclosure

While certain methods described herein have been described with specificity in accordance with certain embodiments, the following examples serve only to illustrate the compounds described herein and are not intended to limit the same.

### Example 1: Effect of antisense oligonucleotides targeting MECP2 in vivo

Antisense oligonucleotides (ASOs) that target human Methyl CpG Binding Protein 2 (MECP2), the complement of GENBANK accession number NT_167198.1 truncated from 4203000 to 4283000, SEQ ID NO: 2, were synthesized using standard solid phase oligonucleotide synthetic methods. They are chimeric oligonucleotides ("gapmers"), composed of a central "gap" region consisting of 2'-deoxynucleotides, which is flanked on both sides (5' and 3') by "wings" that are composed of modified nucleotides. The internucleoside (backbone) linkages are phosphorothioate or phosphodiester throughout the oligonucleotides. The sequences and structures of the antisense oligonucleotides and their start and stop sites along SEQ ID NO: 2 are shown in the table below.

**Table 1: Antisense oligonucleotides targeted to human MECP2**

| Isis No. | Sequence (5' to 3') | Start site | Stop site | SEQ ID NO . |
|---|---|---|---|---|
| 628724 | | 69220 | 69239 | 12 |
| 628749 | | 7615 | 7634 | 13 |
| 628772 | | 22839 | 22858 | 14 |
| 628775 | | 24936 | 24955 | 15 |
| 628785 | | 30744 | 30763 | 16 |

Superscript "m" indicates 5-methylcytosine. Subscripts: "o" indicates a phosphodiester internucleoside linkage, "s" indicates a phosphorothioate internucleoside linkage, "e" indicates a 2'-methoxyethyl modified nucleoside, and "d" indicates a 2'-deoxynucleoside.

The antisense oligonucleotides were analyzed for their effects on MECP2 mRNA and protein levels in transgenic MECP2 duplication mice that overexpress wild type human MECP2 (F1 hybrid MECP2-TG1 mice(FVB/N x 129)(Samaco et al., Nat Genet, 2012). At 8 weeks of age, FVB/N x 129 mice display hypoactivity in the open field test, increased anxiety in the open field and elevated plus maze tests, abnormal social behavior in the 3-chamber test, and increased motor coordination in the rotarod test. Seven week old *MECP2-TG* mice were given stereotactic intracerebral injection of 500 µg of an antisense oligonucleotide listed in Table 1 or saline into the right ventricle of the brain. Wild type mice were given stereotactic intracerebral injection of saline into the right ventricle of the brain as a control. Each group consisted of two or three mice. Two weeks following the injection, the mice were sacrificed, and cortical brain samples were collected for analysis of MECP2 mRNA and protein levels. MECP2 and GAPDH protein levels were analyzed by western blot performed on the cortical sample lysates. Rabbit antiserum raised against the N-terminus of MECP2 and mouse anti-GAPDH 6C5 (Advanced Immunochemicals, Long Beach, CA) were used as the primary antibodies. Western blot images were quanitifed using Image J software, and the MECP2 protein levels normalized to GAPDH levels are shown in Table 2 below.

Total MECP2 mRNA, human MECP2 mRNA (both the e1 and e2 isoforms), and mouse MECP2 mRNA (both the e1 and e2 isoforms) were separeately analyzed by RT-qPCR. The primers common to human and mouse used for total MECP2 mRNA were: 5'-TATTTGATCAATCCCCAGGG-3', SEQ ID NO: 3 , and 5'-CTCCCTCTCCCAGTTACCGT-3', SEQ ID NO: 4. The human specific primers used for MECP2-e1 were 5'-AGGAGAGACTGGAAGAAAAGTC-3', SEQ ID NO: 5, and 5'-CTTGAGGGGTTTGTCCTTGA-3', SEQ ID NO: 6. The human specific primers used for MECP2-e2 were 5'-CTCACCAGTTCCTGCTTTGATGT-3', SEQ ID NO: 7, and 5'-CTTGAGGGGTTTGTCCTTGA-3', SEQ ID NO: 6. The mouse specific primers used for MECP2-e1 were 5'-AGGAGAGACTGGAGGAAAAGTC-3', SEQ ID NO: 8, and 5'-CTTAAACTTCAGTGGCTTGTCTCTG-3', SEQ ID NO: 9. The mouse specific primers used for MECP2-e2 were 5'-CTCACCAGTTCCTGCTTTGATGT-3', SEQ ID NO: 7, and 5'-CTTAAACTTCAGTGGCTTGTCTCTG-3', SEQ ID NO: 9. MECP2 mRNA levels were normalized to Hprt mRNA levels, which were analyzed using primer 5'-CGGGGGACATAAAAGTTATTG-3', SEQ ID NO: 10, and 5'-TGCATTGTTTTACCAGTGTCAA-3', SEQ ID NO: 11. Results are presented in Table 2 below as average normlized MECP2 mRNA levels relative to saline treated wild type (WT) mice. The results show that all of the antisense oligonucleotides tested inhibited MECP2 mRNA and protein levels in the transgenic mice, and human MECP2 mRNA levels were specifically inhibited, whereas mouse MECP2 mRNA levels were not inhibited. Isis Number 628785 was the most potent in the first experiments and was carried forward. Entries listed as "n/a" indicate that the corresponding experiment was not performed.

**Table 2: MECP2 mRNA and protein levels in transgenic mice following ASO administration**

| Mouse/Isis No. | MECP2 protein level | Total MECP2 mRNA | Human mRNA | | Mouse mRNA | | SEQ ID NO. |
|---|---|---|---|---|---|---|---|
| | | | MECP2-e1 isoform | MECP2-e2 isoform | MECP2-e 1 isoform | MECP2-e2 isoform | |
| WT/PBS | 1.0 | 1.0 | 0.0 | 0.0 | 1.0 | 1.3 | |
| TG/PBS | 2.0 | 3.3 | 0.9 | 8.3 | 1.2 | 1.4 | |
| TG/628724 | 1.5 | 2.0 | n/a | n/a | n/a | n/a | 12 |
| TG/628749 | 1.6 | 2.5 | n/a | n/a | n/a | n/a | 13 |
| TG/628772 | 1.7 | 2.7 | n/a | n/a | n/a | n/a | 14 |
| TG/628775 | 1.4 | 2.1 | n/a | n/a | n/a | n/a | 15 |
| TG/628785 | 1.3 | 1.6 | 0.3 | 2.3 | 1.0 | 1.3 | 16 |

### Example 2: Effect of gradual infusion of antisense oligonucleotide targeting MECP2 in vivo

In order to gradually infuse antisense oligonucleotide into the right ventricle of the brain, micro-osmotic pumps (Alzet model 1004, Durect, Cupertino, CA) were filled with 500µg of Isis No. 628785 or a control oligonucleotide that is not targeted to MECP2, dissolved in 100µl saline. The pump was then connected through a plastic catheter to a cannula (Alzet Brain Infusion Kit 3, Durect, Cupertino, CA). The pump was designed to deliver the drug at a rate of 0.11µl per hour for 28 days. The cannula and pump assembly was primed in sterile saline for two days at 37°C. Mice were anesthetized with isoflurane and placed on a computer-guided stereotaxic instrument (Angle Two Stereotaxic Instrument, Leica Microsystems, Bannockburn, IL). Anesthesia (isoflurane 3%) was continuously delivered via a small face mask. Ketoprofen 5mg/kg was administered subcutaneously at the initiation of the surgery. After sterilizing the surgical site with betadine and 70% alcohol, a midline incision was made over the skull and a subcutaneous pocket was generated on the back of the animal. Next, the pump was inserted into the pocket and the cannula was stereotactically implanted to deliver the drug in the right ventricle using the following coordinates: AP = -0.2 mm, ML = 1 mm, DV = -3 mm. The incision was sutured shut. Carprofen-containing food pellets were provided for 5 days after the surgery. 28 days after the initiation of the treatment the pump was disconnected from the cannula and removed. Two additional weeks were given to the animals to recover.

Isis No. 628785 was gradually infused into the right ventricles of the brains of 7-week old WT or TG mice using the micro-osmotic pumps. Each treatment group consisted of 4 or 5 animals. At the end of the four-week treatment period, western blot was performed as described in Example 1 to analyze MECP2 protein levels at 4, 8, and 12 weeks following the initiation of antisense oligonucleotide treatment. The results are shown in Table 3 below.

**Table 3: MECP2 protein levels following antisense oligonucleotide infusion**

| Mouse/Isis No. | MECP2 protein level (relative to WT/Control) | | |
|---|---|---|---|
| | 4 weeks | 8 weeks | 12 weeks |
| WT/Control | 1.0 | 1.0 | 1.0 |
| TG/Control | 2.9 | 2.7 | 2.3 |
| TG/628785 | 1.6 | 1.8 | 2.2 |

### Example 3: Behavioral effects of antisense oligonucleotide targeting human MECP2 in vivo

Following infusion of antisense oligonucleotide as described in Example 2, a battery of behavioral assays were performed to assess phenotypic effects of oligonucleotide treatment in TG mice treated with Isis No. 628785 or a control oligonucleotide and WT mice treated with a control oligonucleotide. Each treatment group contained at least 15 animals.

An open field test was performed two weeks and six weeks after the completion of the 4 week infusion by placing mice into the center of an open arena after habituation in the test room (40 x 40 x 30 cm). Their behavior was tracked by laser photobeam breaks for 30 min. Horizontal locomotor activity, rearing activity, time spent in the center of the arena, and entries to the center were analyzed using AccuScan Fusion software (Omnitech, Columbus, OH). The results are reported in table 4 below. The results show that the TG mice displayed hypoactivity in the open field test relative to WT mice at both time points, and treatment of TG mice with Isis No. 628785 restored activity close to WT levels.

**Table 4: Open field test**

| Mouse/Isis No. | Horizontal activity (activity counts) | | Rearing episodes | | Time in center (s) | | Entries to center | |
|---|---|---|---|---|---|---|---|---|
| | 2 weeks | 6 weeks | 2 weeks | 6 weeks | 2 weeks | 6 weeks | 2 weeks | 6 weeks |
| WT/Control | 7134 | 5632 | 277 | 236 | 179 | n/a | 147 | 103 |
| TG/Control | 4116 | 3493 | 156 | 106 | 105 | n/a | 65 | 45 |
| TG/628785 | 5550 | 6114 | 170 | 205 | 93 | n/a | 75 | 99 |

Mice were tested in an elevated plus maze two weeks and six weeks after the completion of the 4 week infusion. After habituation in the test room, mice were placed in the center part of the maze facing one of the two open arms. Mouse behavior was video-tracked for 10 minutes, and the time the mice spent in the open arms and the entries to the open arms were recorded and analyzed using ANY-maze system (Stoelting, Wood Dale, IL). The results are shown in Table 5 below. The results show that the TG mice displayed increased anxiety in the elevated plus maze test relative to WT mice at both time points, and treatment of TG mice with Isis No. 628785 restored anxiety levels close to WT levels.

**Table 5: Elevated plus maze**

| Mouse/Isis No. | Time in open arms (s) | | Entries into open arms | |
|---|---|---|---|---|
| | 2 weeks | 6 weeks | 2 weeks | 6 weeks |
| WT/Control | 139 | 81 | 21 | 12 |
| TG/Control | 76 | 13 | 12 | 2 |
| TG/628785 | 91 | 55 | 11 | 7 |

Mice were assessed in a three-chamber social interaction test three weeks and seven weeks after the completion of the 4 week infusion. The apparatus comprised a clear Plexiglas box with removable partitions that separated the box into three chambers: left, central, and right. In the left and right chambers a cylindrical wire cup was placed with the open side down. Age and gender-matched mice were used as novel partners. Two days before the test, the novel partner mice were habituated to the wire cups (3 inches diameter by 4 inches in height) for 1 hour per day. After habituation in the test room, each mouse was placed in the central chamber and allowed to explore the three chambers for 10 minutes (habituation phase). The time spent in each chamber during the habituation phase was recorded automatically and analyzed using ANY-maze system (Stoelting, Wood Dale, IL). Next, a novel partner mouse was placed under a wire cup in either the left or the right chamber. An inanimate object was placed as a control under the wire cup of the opposite chamber. The location of the novel mouse was randomized between the left and right chambers for each test mouse to control for side preference. The mouse tested was allowed to explore again for an additional 10 minutes. The time spent investigating the novel partner (defined by rearing, sniffing or pawing at the wire cup) and the time spent investigating the inanimate object were measured manually. The results are shown in Table 6 below. The results show that the TG mice displayed hypoactivity and decreased social interaction in the three-chamber social interaction test relative to WT mice at both time points, and treatment of TG mice with Isis No. 628785 restored social interaction with a novel partner to WT levels at the 6 week time point.

**Table 6: Three-chamber social interaction test**

| Mouse/Isis No. | Time spent investigating chambers during habituation phase (s) | | | | Time spent investigating novel partner or inanimate object (s) | | | |
|---|---|---|---|---|---|---|---|---|
| | Left | | Right | | Novel partner | | Inanimate object | |
| | 2 weeks | 6 weeks | 2 weeks | 6 weeks | 2 weeks | 6 weeks | 2 weeks | 6 weeks |
| WT/Control | 52.4 | n/a | 44.1 | n/a | 141 | 107 | 38 | 37 |
| TG/Control | 35.5 | n/a | 29.9 | n/a | 106 | 59 | 33 | 28 |
| TG/628785 | 37.7 | n/a | 23.0 | n/a | 94 | 106 | 27 | 28 |

Mice were assessed in an accelerating rotarod test three weeks after the completion of the 4 week infusion. After habituation in the test room, motor coordination was measured using an accelerating rotarod apparatus (Ugo Basile, Varese, Italy). Mice were tested 2 consecutive days, 4 trials each, with an interval of 60 minutes between trials to rest. Each trial lasted for a maximum of 10 minutes; mice that never fell were given a measurement of 600 seconds. The rod accelerated from 4 to 40 r.p.m. in the first 5 minutes. The time that it took for each mouse to fall from the rod (latency to fall) was recorded. Results are shown in Table 7 below. The results show that the TG mice displayed increased performance in the rotarod test relative to WT mice, and treatment of TG mice with Isis No. 628785 restored performance to WT levels.

**Table 7: Accelerating rotarod test**

| Mouse/Isis No. | Latency to fall (s) | |
|---|---|---|
| | Day 1 | Day 2 |
| WT/Control | 174 | 300 |
| TG/Control | 275 | 400 |
| TG/628785 | 183 | 282 |

The results in tables 4-7 above show that treatment with Isis No. 628785 targeting MECP2 reversed behavioral phenotypes of the TG mice. The TG mice treated with Isis No. 628785 performed similarly to WT mice in the rotarod test 3-4 weeks after completion of the infusion. By 6-7 weeks after completion of the infusion, the hypoactivity, anxiety-like behaviors and social behavior of the TG mice were reversed, as evidenced by the open field, elevated plus maze and three-chamber tests, respectively.

### Example 4: Dose response of antisense oligonucleotide targeting human MECP2 in patient cells

In order to test for a dose dependent effect of Isis No. 628785 on human cells, B-lymphoblast cells from two individuals affected with MECP2-duplication symdrome and age-matched control cells were cultured in suspension in RPMI 1640 medium with L-glutamine, penicillin-streptomycin, and 10% (v/v) fetal bovice serum. A day before transfection, cells were seeded in triplicate for each treatment in 6-well plates at 10⁶ cells per well in a total volume of 2 mL medium. Cells were transfected with Isis No. 628785 or control oligonucleotide at a concentration listed in Table 8 below with TurboFect transfection reagent (Thermo Scientific, Carlsbad, CA). Cells were harvested and RNA was extracted 48 hours after transfection, and MECP2 mRNA levels were analyzed as described in Example 1. Results are presented in Table 8 below as average normlized MECP2 mRNA levels for both patients' cells relative to untreated control cells. The results show that Isis No. 628785 inhibited MECP2 expression in human MECP2 duplication patient cells.

**Table 8: Antisense oligonucleotide treatment of patient lymphoblasts**

| Cell type/Isis No. | Concentration (nM) | Total relative MECP2 mRNA |
|---|---|---|
| Control/Control | 600 | 1.0 |
| Patient/Control | 600 | 3.1 |
| Patient/628785 | 150 | 2.2 |
| Patient/628785 | 300 | 1.6 |
| Patient/628785 | 600 | 1.3 |

### Example 5: Reduction of seizure activity with an antisense oligonucleotide targeting human MECP2 in vivo

Without treatment, seizures and accompanying abnormal electrographic discharges occur in MECP2-TG1 mice as they age. In order to test the effect of antisense oligonucleotide treatment on seizure activity in MECP2-TG1 mice, electrocephalography recordings were performed and behavioral seizure activity was observed.

25-35 week old MECP2-TG1 mice that had been treated as described in Example 2 were anaesthetized with isoflurane and mounted in a stereotaxic frame for the surgical implantation of three recording electrodes (Teflon-coated silver wire, 125 µm in diameter) in the subdural space of the left frontal cortex, the left parietal cortex, and the right parietal cortex, with a reference electrode placed in the occipital region of the skull. After 3-5 days of surgical recovery, cortical EEG activity and behavior were recorded for 2h per day over 3-5 days. Strong electrographic seizure events were typically accompanied by behavioral seizures. Figure 1 displays EEG traces for WT mice, MECP2-TG1 mice without Isis No. 628785 treatment, and MECP2-TG1 mice that received treatment with Isis No. 628785. Treatment of MECP2-TG1 mice with Isis No. 628785 eliminated both behavioral seizures and abnormal EEG discharges.

## Claims

1. An antisense compound complementary to a MECP2 nucleic acid for use in treating MECP2 duplication syndrome.

2. The antisense compound for use according to claim 1, wherein the antisense compound is administered by parenteral administration, wherein optionally the parenteral administration is any of intracerebroventricular administration or intrathecal administration.

3. The antisense compound for use according to any preceding claim, wherein:
(a) administration of the antisense compound reduces MECP2 mRNA and or protein levels, wherein optionally the administering reduces MECP2 mRNA and or protein levels by at least 15, 20, 25, 30, 35, 40, 45, 50, 55, 60, or 65 percent; and/or
(b) administration of the antisense compound improves motor function, wherein optionally motor function is improved by at least 10, 15, 20, 25, 30, or 35 percent; and/or
(c) administration of the antisense compound improves anxiety, wherein optionally the administering improves anxiety by at least 5, 10, 15, 20, 25, 30, 35, 40, 45, or 50 percent; and/or
(d) administration of the antisense compound improves social interaction, wherein optionally the administering improves social interaction by at least 5, 10, 15, 20, 25, 30, 35, 40, 45, or 50 percent; and/or
(e) administration of the antisense compound improves activity, wherein optionally the administering improves activity by at least 5, 10, 15, 20, 25, 30, 35, 40, 45, 50, 55, 60, 65, 70, 75, or 80 percent; and/or
(f) administration of the antisense compound reduces seizures; and/or
(g) administration of the antisense compound normalizes EEG discharges.

4. The antisense compound for use according to any preceding claim, wherein the MECP2 nucleic acid has the nucleobase sequence of SEQ ID NO: 1 or SEQ ID NO: 2.

5. The antisense compound for use according to any preceding claim, wherein the antisense compound is a single-stranded oligonucleotide.

6. The antisense compound for use according to any preceding claim, wherein the antisense compound is a gapmer.

7. The antisense compound for use according to any preceding claim, wherein at least one internucleoside linkage of the antisense compound, or each internucleoside linkage of the antisense compound, is a modified internucleoside linkage, wherein optionally the modified internucleoside linkage is a phosphorothioate internucleoside linkage.

8. The antisense compound for use according to any of claims 1-6, wherein at least one internucleoside linkage of the antisense compound is a phosphorothioate internucleoside linkage and at least one internucleoside linkage is a phosphodiester internucleoside linkage.

9. The antisense compound for use according to any preceding claim, wherein at least one nucleobase of the antisense compound is a modified nucleobase, wherein optionally the modified nucleobase is a 5-methylcytosine.

10. The antisense compound for use according to any preceding claim, wherein at least one nucleoside of the antisense compound comprises a modified sugar, wherein optionally the modified sugar is a bicyclic sugar or comprises a 2'-O-methoxyethyl group or comprises a 2'-O-methyl group.

11. The antisense compound for use according to claim 10, wherein the bicyclic sugar comprises a chemical bridge between the 4'and 2' positions of the sugar, wherein the chemical bridge is selected from: 4'-(CH₂)ₙ₋O-2', wherein n is 1 or 2; 4'-CH₂-O-CH₂-2'; 4'-CH(CH₃)-O-2'; and 4'-CH(CH₂OCH₃)-O-2' .

12. The antisense compound for use according to any preceding claim, wherein at least one nucleoside of the antisense compound comprises a sugar surrogate.

13. The antisense compound for use according to any preceding claim, wherein the antisense compound comprises a conjugate group.

14. The antisense compound for use according to any of claims 1-4 or 6-13, wherein the antisense compound is a double-stranded compound.

15. The antisense compound for use according to any preceding claim, wherein the antisense compound is 100% complementary to the MECP2 nucleic acid.

## Patentansprüche

1. Zu einer MECP2-Nukleinsäure komplementäre Antisense-Verbindung zur Verwendung bei der Behandlung des MECP2-Duplikationssyndroms.

2. Antisense-Verbindung zur Verwendung nach Anspruch 1, wobei die Antisense-Verbindung durch parenterale Verabreichung verabreicht wird, wobei die parenterale Verabreichung intracerebroventrikuläre Verabreichung oder intrathekale Verabreichung ist.

3. Antisense-Verbindung zur Verwendung nach einem der vorhergehenden Ansprüche, wobei:
(a) die Verabreichung der Antisense-Verbindung den MECP2-mRNA- und/oder -Proteinpegel reduziert, wobei optional die Verabreichung den MECP2-mRNA- und/oder -Proteinpegel um mindestens 15, 20, 25, 30, 35, 40, 45, 50, 55, 60 oder 65 Prozent reduziert; und/oder
(c) die Verabreichung der Antisense-Verbindung die motorische Funktion verbessert, wobei optional die motorische Funktion um mindestens 10, 15, 20, 25, 30 oder 35 Prozent verbessert wird; und/oder
(c) die Verabreichung der Antisense-Verbindung Angst abmildert, wobei optional die Verabreichung Angst um mindestens 5, 10, 15, 20, 25, 30, 35, 40, 45 oder 50 Prozent abmildert; und/oder
(d) die Verabreichung der Antisense-Verbindung soziale Interaktion verbessert, wobei optional die Verabreichung soziale Interaktion um mindestens 5, 10, 15, 20, 25, 30, 35, 40, 45 oder 50 Prozent verbessert; und/oder
(e) die Verabreichung der Antisense-Verbindung Aktivität verbessert, wobei optional die Verabreichung Aktivität um mindestens 5, 10, 15, 20, 25, 30, 35, 40, 45, 50, 55, 60, 65, 70, 75 oder 80 Prozent verbessert; und/oder
(f) die Verabreichung der Antisense-Verbindung Anfälle reduziert; und/oder
(g) die Verabreichung der Antisense-Verbindung EEG-Entladungen normalisiert.

4. Antisense-Verbindung zur Verwendung nach einem der vorhergehenden Ansprüche, wobei die MECP2-Nukleinsäure die Nukleobasensequenz von SEQ ID NO: 1 oder SEQ ID NO: 2 aufweist.

5. Antisense-Verbindung zur Verwendung nach einem der vorhergehenden Ansprüche, wobei die Antisense-Verbindung ein einzelsträngiges Oligonukleotid ist.

6. Antisense-Verbindung zur Verwendung nach einem der vorhergehenden Ansprüche, wobei die Antisense-Verbindung ein Gapmer ist.

7. Antisense-Verbindung zur Verwendung nach einem der vorhergehenden Ansprüche, wobei mindestens eine Internukleosid-Verknüpfung der Antisense-Verbindung oder jede Internukleosid-Verknüpfung der Antisense-Verbindung eine modifizierte Internukleosid-Verknüpfung ist, optional wobei die modifizierte Internukleosid-Verknüpfung eine Phosphorthioat-Internukleosid-Verknüpfung ist.

8. Antisense-Verbindung zur Verwendung nach einem der Ansprüche 1-6, wobei mindestens eine Internukleosid-Verknüpfung der Antisense-Verbindung eine Phosphorthioat-Internukleosid-Verknüpfung ist und mindestens eine Internukleosid-Verknüpfung eine Phosphodiester-Internukleosid-Verknüpfung ist.

9. Antisense-Verbindung zur Verwendung nach einem der vorhergehenden Ansprüche, wobei mindestens eine Nukleobase der Antisense-Verbindung eine modifizierte Nukleobase ist, optional wobei die modifizierte Nukleobase ein 5-Methylcytosin ist.

10. Antisense-Verbindung zur Verwendung nach einem der vorhergehenden Ansprüche, wobei mindestens ein Nukleosid der Antisense-Verbindung einen modifizierten Zucker umfasst, optional wobei der modifizierte Zucker ein bicyclischer Zucker ist oder eine 2'-0-Methoxyethylgruppe umfasst oder eine 2'-O-Methylgruppe umfasst.

11. Antisense-Verbindung zur Verwendung nach Anspruch 10, wobei der bicyclische Zucker eine chemische Brücke zwischen der 4'- und der 2'-Position des Zuckers umfasst, wobei die chemische Brücke ausgewählt ist aus: 4'-(CH₂)ₙ-O-2', wobei n 1 oder 2 ist; 4-CH₂-O-CH₂-2; 4'-CH(CH₃)-O-2 ' ; und 4'-CH(CH₂OCH₃)-O-2'.

12. Antisense-Verbindung zur Verwendung nach einem der vorhergehenden Ansprüche, wobei mindestens ein Nukleosid der Antisense-Verbindung ein Zuckersurrogat umfasst.

13. Antisense-Verbindung zur Verwendung nach einem der vorhergehenden Ansprüche, wobei die Antisense-Verbindung eine Konjugatgruppe umfasst.

14. Antisense-Verbindung zur Verwendung nach einem der Ansprüche 1-4 oder 6-13, wobei die Antisense-Verbindung eine doppelsträngige Verbindung ist.

15. Antisense-Verbindung zur Verwendung nach einem der vorhergehenden Ansprüche, wobei die Antisense-Verbindung zu 100 % komplementär zu der MECP2-Nukleinsäure ist.

## Revendications

1. Composé antisens complémentaire à un acide nucléique de MECP2 pour une utilisation dans le traitement du syndrome de duplication de MECP2.

2. Composé antisens pour une utilisation selon la revendication 1, le composé antisens étant administré par administration parentérale, éventuellement l'administration parentérale étant l'une quelconque parmi une administration intracérébroventriculaire et une administration intrathécale.

3. Composé antisens pour une utilisation selon une quelconque revendication précédente,
(a) l'administration du composé antisens réduisant des taux d'ARNm et/ou de protéines de MECP2, éventuellement l'administration réduisant de 15, 20, 25, 30, 35, 40, 45, 50, 55, 60 ou 65 pour cent les taux d'ARNm et/ou de protéines de MECP2 ; et/ou
(b) l'administration du composé antisens améliorant la fonction motrice, éventuellement la fonction motrice étant améliorée d'au moins 10, 15, 20, 25, 30 ou 35 pour cent ; et/ou
(c) l'administration du composé antisens améliorant l'anxiété, éventuellement l'administration améliorant l'anxiété d'au moins 5, 10, 15, 20, 25, 30, 35, 40, 45 ou 50 pour cent ; et/ou
(d) l'administration du composé antisens améliorant l'interaction sociale, éventuellement l'administration améliorant d'au moins 5, 10, 15, 20, 25, 30, 35, 40, 45 ou 50 pour cent l'interaction sociale ; et/ou
(e) l'administration du composé antisens améliorant l'activité, éventuellement l'administration améliorant l'activité d'au moins 5, 10, 15, 20, 25, 30, 35, 40, 45, 50, 55, 60, 65, 70, 75 ou 80 pour cent ; et/ou
(f) l'administration du composé antisens réduisant les crises ; et/ou
(g) l'administration du composé antisens normalisant les décharges d'EEG.

4. Composé antisens pour une utilisation selon une quelconque revendication précédente, l'acide nucléique de MECP2 possédant la séquence de nucléobases de SEQ ID NO: 1 ou SEQ ID NO: 2.

5. Composé antisens pour une utilisation selon une quelconque revendication précédente, le composé antisens étant un oligonucléotide à simple brin.

6. Composé antisens pour une utilisation selon une quelconque revendication précédente, le composé antisens étant un gapmère.

7. Composé antisens pour une utilisation selon une quelconque revendication précédente, au moins une liaison internucléosidique du composé antisens, ou chaque liaison internucléosidique du composé antisens, étant une liaison internucléosidique modifiée, éventuellement la liaison internucléosidique modifiée étant une liaison internucléosidique phosphorothioate.

8. Composé antisens pour une utilisation selon l'une quelconque des revendication 1 à 6, au moins une liaison internucléosidique du composé antisens étant une liaison internucléosidique phosphorothioate et au moins une liaison internucléosidique étant une liaison internucléosidique phosphodiester.

9. Composé antisens pour une utilisation selon une quelconque revendication précédente, au moins une nucléobase du composé antisens étant une nucléobase modifiée, éventuellement la nucléobase modifiée étant une 5-méthylcytosine.

10. Composé antisens pour une utilisation selon une quelconque revendication précédente, au moins un nucléoside du composé antisens comprenant un sucre modifié, éventuellement le sucre modifié étant un sucre bicyclique ou comprenant un groupe 2'-O-méthoxyéthyle ou comprenant un groupe 2'-O-méthyle.

11. Composé antisens pour une utilisation selon la revendication 10, le sucre bicyclique comprenant un pont chimique entre les positions 4' et 2' du sucre, le pont chimique étant choisi parmi : 4'-(CH₂)ₙ-O-2', n étant 1 ou 2 ; 4'-CH₂-O-CH₂-2' ; 4'-CH(CH₃)-O-2' ; et 4'-CH(CH₂OCH₃)-O-2'.

12. Composé antisens pour une utilisation selon une quelconque revendication précédente, au moins un nucléoside du composé antisens comprenant un substitut de sucre.

13. Composé antisens pour une utilisation selon une quelconque revendication précédente, le composé antisens comprenant un groupe conjugué.

14. Composé antisens pour une utilisation selon l'une quelconque des revendications 1 à 4 ou 6 à 13, le composé antisens étant un composé à double brin.

15. Composé antisens pour une utilisation selon une quelconque revendication précédente, le composé antisens étant 100 % complémentaire à l'acide nucléique de MECP2.


### SEQUENCE LISTING

<110> Ionis Pharmaceuticals, Inc. Baylor College of Medicine
<120> METHODS FOR MODULATING MECP2 EXPRESSION
<130> BIOL0263WO
<150> 62/127,693
   <151> 2015-03-03
<160> 16
<170> PatentIn version 3.5
<210> 1
   <211> 10241
   <212> DNA
   <213> Homo sapiens
<400> 1
<210> 2
   <211> 80001
   <212> DNA
   <213> Homo sapiens
<400> 2
   gctaccatca actctttctc ctagactgtt ccagggcctt gcaactagcc ttgtgctgta        60
   gttttgtttc atcacgtcca gttctccact ctacacctgc aacatagatc agacagctcc       120
   tggctcaaaa tcctctgagg gcttctcatc ttagaataaa ctctcggttc tggccgggtg       180
   cggtggctta cgcctgtagt cccagcactt tgggagtccg aggcgggcgg atcacttgaa       240
   ctcaggagtt tgagaccagc ctgggcaaca tggtgaactc ccatctctat caaaaataca 300
   aaaacttagc caggcgtggt ggttcgcatc tgtggtccca gctacttagg acgctgagga 360
   gggaggatcg cttgagctca gggtggacgt tgcagtgagc caagattgcg ccactgcact 420
   gcagcctggg tgacagaatg agaccccatc cccacccccc ccaaaaaaga atgaactccc 480
   agttctcata gtggccccag ctgcctttcc aatcacattc cctaccactc tccagcaaca 540
   ctgacttcct cgttagtccc caacatgcca ggcatagtct ctcctcatgt cctttgaact 600
   tgcctggaat gttctttccc cagatattca tatgagggag taaaatgagg gtgaaaacca 660
   gcagatatct aaatagcacc cccttcactt agtttatctt tctcaaagcc cttatcacta 720
   tgtgaaatga tatattatac ttatttgtat gctagtatga atcttcccgg caagaatgtt 780
   agtttgctgt ctgttcagta ccgtgcatcc agagcctgga agagtgcctg gcacatagca 840
   ggtagtcaat aaatgaatgg gggcaagcag ccaaatcaga atcaggtttt cttgctaagc 900
   atagaactaa cagaaggatc attgaatgga ttggataatg actggcatca gggtaaggtc 960
   cccttaacaa acactcctgt cctgaacacc tggttagcta acagttttct catactctta 1020
   ttttcccaaa acacaattgc tggatctcag ctccaaatca actcttctag gaaagtgaaa 1080
   aattgctgga tctcagctcc caaatcatat cttccaggca gagctaacat tgccccttat 1140
   tcacacctcc accaaaccat ctgatccaac agtgacaggt gtcacgaggc cttggcatgc 1200
   actctcttcc cccgccagag ttctgcgaaa gccagggttg cgatttgttg tcagtttatt 1260
   ccccgcctct atgagagtgt gagcactggg caggctcgga tgaaataatg cattgagtag 1320
   gcctctgaaa ccaaggcccc tcagctgggg caacgtcagg ctccagggtg ggcaactttg 1380
   ctgcttctgc cgaagatagt gatattgaga aaatgtgggt gcaatgaaac gcttattgca 1440
   gcgcactcgg tgcatctgtg gacagagggt caatcgcccc tcagagcagc gcaaacaggc 1500
   gtcccaagcc taggccttca cttgccccag catccgcaag ggtccattaa tccttaacat 1560
   tcaaattccg cccactaaac cagtccctcc gcgcccaagc cgcctctttt ccccaaacga 1620
   cggccgaaag cagccaatca acagctggag gggtccgccc ccttttccct ggccgaaatg 1680
   gacaggaaat ctcgccaatt gacggcatcg ccgctgagac ctcccccctc ccccgtcctc 1740
   cccgtcccag cccggccatc acagccaatg acgggcgggc tcgcagcggc gccgagggcg 1800
   gggcgcgggc gcgcaggtgc agcagcgcgc gggccggcca agagggcggg gcgcgacgtc 1860
   ggccgtgcgg ggtcccggcg tcggcggcgc gcgcgctccc tcctctcgga gagagggctg 1920
   tggtaaaagc cgtccggaaa atggccgccg ccgccgccgc cgcgccgagc ggaggaggag 1980
   gaggaggcga ggaggagaga ctgtgagtgg gaccgccgtg gccgcgggcg gggacccttg 2040
   ccggggggcg ggggtcaggg gcgggacgtg gcgcgggagg ggcccgcggg gtcggacgac 2100
   acggctggcg gatggcgtcc ctcctctcta ccctccccct cccgccgccg ccggtggcga 2160
   ctctcccctc ggcccgtcac ccgtgctcgc gggtgaccgt cctcggcgcg gcctccctgg 2220
   agccgccttc gcctgacgcc cctcttcctc ccgccctcga cgcgcatccc ggcccccggc 2280
   cccgcgggcg cccctgtcgc cggggttcgc ctgtcggggc tgcgcgcgct cctgcccttc 2340
   tcggggcttt gggccgcggc gccgtcgcgc gcccgcggcc ccggcctctc cctggatcgc 2400
   gctctccccc tccctccctc gcgcgccccc tctcccgtta ctcggccccc ccaccggcgc 2460
   gcgtgcgcac ttcgcctccc gtcgggagag tgcgccacaa gggctcctga gctctcaccc 2520
   ccatctctgg gctttgcctc cccctccttc tcgcccattc catgaatttc tgccccccgc 2580
   tacccccccg cgagcgagta ggtccaccgg ctcctttccc catctagcag gaacaagtag 2640
   gtggggatta ttatccacaa aagggactag acattgtgtt ctgggtccca caactcatca 2700
   taaagaggtg gttatagttc ccatcaggag ccgtgggtag gggactgtgc gtccagcagc 2760
   acccgaggct cttcggcgcc agaggctcta aggtgcgagc gtgtccccag ggtgctcaga 2820
   ggttctttgg agtgctgtgg cctcggaatg tgagcaccct cccatcctac cctccccttc 2880
   gccggcgatc ttccagttac ataagtggag tgggacatag taggtaacgg gctctcatcg 2940
   ccctggagcg ctcgggatca ccggtaccag atggggcaag ttcatcgcga cgctgtggct 3000
   cctgttaatt gtgcctgaaa ggttatcctc tgttcagttt ggtcatgacc gaatcaccca 3060
   aactgaaact cagatgactt ttatatggca gtggcaatgt ttctgtggtt ttgcactaaa 3120
   gacttaaggt cattatgaag agtgtaaaag gattgctgtc gcaggggaga ccgtttatga 3180
   cattgctaag tatggacccc gagggggaaa aagctcattc tggaattgct ctgtggcggt 3240
   tgcttctgtt gcgagtgtgt tcgggtgtgg gttggtcaca gcagaaatgg gctccaccac 3300
   aaaattgata ttcctgactc atggtcaggc agatgtgtgc ttctggttat ttttccagga 3360
   acaggaatgg gctctgccga gcctttcaca cgttgtgtct ctgctgtgcg ggatcagtac 3420
   cccagactgt gagataacag gatcgaattc aggggttggt tggtgatcca ggaaccatac 3480
   ctacttggat gtgatactga gtggcctaga cgttgtagga ctctcataag ttttagacat 3540
   tttgttatac tgtaaaaatg aagcatatac ggcctcagta gtaacattgc actaataggt 3600
   aattattact ctagagaatt ttgggttcca tggctggaat gttcatcagt atctcagcaa 3660
   tttcagttaa taaccagatt aagctgacct cttgagtctt ttgaaatctg ctgggtccct 3720
   ataataagac agggtctgga ttttttaatc ttttgggaat ttccaccaag gatttaggga 3780
   aataggcttt tatgggcttt tcaggatttc tatttcaaat cccaatgagg aagtctagga 3840
   aacacttgct tttattttct tgcctgaata tgtcagaacc ttaaatcacc caatcaatct 3900
   agaagtacta cagttgatta aagtaagtca ccacaggtca cccattcgtg gcaggtcagc 3960
   taggtggctg atcactggaa tacagtcata tccccaggca tacacacgag cctcacttga 4020
   cgatcccaaa gtttgtacat ctgagcttga gacgtgacaa gttgaggtct ctgctttgtt 4080
   cttcatagtt ggcagtttgt ctcctcgggt gcccctccgg ttagctgttt atcatacgtc 4140
   tagccctatc aacagtgcag gcagcttcca gagtatagtt ttctctgcaa agtgcctggt 4200
   ggaggggtac ctccttttcc attgtcctaa tggctccact gggcttccat accagccctt 4260
   tccagtgtct taacagctca aagaaaccca tctgtggctt tcttgttagc actggcctat 4320
   cacaccttcc tattgcctgt taactaaaat tttgcctgga atgctttaag cttagagtac 4380
   tgggtctttg tttcacccga ttgataggag agtgtcatgc agtcagccga actccgactt 4440
   ctgtgctgtt taaatcctta ggtaggtctg gggcaaggca caaaccactg tgtattcaaa 4500
   gggttatggt gcttgcattc tgtgtgtata tgtttgtaat ttccaaccag agtcgtttcc 4560
   cgtgcctgcc ctgcctctgt gcaatctagt ctagtagggt agggaggcaa gctgtggagt 4620
   ttgaaaaaaa aaaaaaaagc tagatgagtt taataggctg ttaccccctc cagaacattc 4680
   ctcccatgtg gagatgaaac gagaagttca tttctttaac ctaaaataaa gaacaacaga 4740
   gataaaattg ttagttcctt attggagcct ggtttccatt gctaatgtac ctgcttggga 4800
   ctctgcatga taaatgacgt gtcctggaac ctgaaatctt ttataaaggt taatatcagc 4860
   agaaatcttt taattagcta agagcagctt gttctcaagc tgcccgagtt atcttggagg 4920
   agagaatcac aggaaatgtt tgatatttct agtaaccagt gttttcagtg cgtggggtaa 4980
   agcacactcc tagactggcc agtcccaaag agtgtggcag gacacctgct tgttttccct 5040
   gccctctgct atggtgacat ccttgcagac cagtcttgcg tggctggcag acgcaatctt 5100
   tttttcttgg gtagagcatt atcaccgctg cgatggaagg accttgtcct cggtgcctcc 5160
   ataccagccc tttccagtga aactactgct gtcccggcaa gcccctgtgc gtgtgcatca 5220
   ttccgggtag gacaagatgt gaacaggtcc ctcttctttg ggcttaagta gaagttgtgc 5280
   tcttctgctt ttacctgtgt gctcttctgc ttttacctgt tctcttctgc ctttctcttg 5340
   caagaaacct gtcgaaagct tgctttgcat gccattgctt aaatactttg atggtatgta 5400
   tggtatgtgg gataactgag tgggagccgg aattgggggg tgggcagggc atccccaccc 5460
   ccacccccac atcaaagggg gaatgaccat ttcgttagag aataaagcct gagtcttata 5520
   acttcttcaa gcacatgtat gctgggtctc ctggcgtgtg aatgtgttcc cgctgtgctg 5580
   tgtggctgtt ttgcagttac tgtagacact gtagtctggg ctctcattat tgcctctgaa 5640
   gttgacagga ccaagcctta gtaaggatgc acttgtcttc ctagcccaag agctaggggt 5700
   gttttatata tatattaact ttctttttta gatttcataa actgctctca ttttctcttt 5760
   tctttctttc tttttttttt tttttttttg agacagtctc actctgtcac ccatgctgga 5820
   gtgcagtggc acgatctcag ctcactgcaa cctctgcctc ccgggctcaa gcagttctct 5880
   gcctcagcct cccaagtagc tgggattaca ggcacctgcc accacgcctg gctaattttt 5940
   gtattttttt tttttttttt tttttttttt tagtcgagac ggggtttcac catcttggcc 6000
   acgctggtct tgaactcctg acctcgtgat ccacccgctt cagcctccca aagtgcaggg 6060
   attacaggcg tgagccaccg cacccagcct ctggctcttt tctttttgac atgaaatctt 6120
   aagttaatta tcttaaactt taagaactag aaggattctt agtccagcac catcagttta 6180
   caaacaaaaa aaacttgtgt ccctgagaag ttgtgacttt tccagggcct cctagccagg 6240
   ataccagttt ggtttttatt atatagctag ccgagtaata ctattaactt gacttctggg 6300
   atttcaaagt attctatgac ttgactgttt aagggaatta tgaggcctca ctgaacctca 6360
   aaagataatt ttaggtacca tcctggtagc tgtagagcag caacagacca gtaaaagact 6420
   tggttggtgt tgcccttctt ctgggtttga ttacatgagt aattgtgtga atagtctcta 6480
   agttgtatgc tctgagcttt cgtttttagc ttataaaagt gctactcttg ggccagcata 6540
   gtatcagaaa ttagctaatt catatggggc agtttaggct ttaactagga aatgatggtt 6600
   atcttaatga caaaaaaggt actgacaaaa gtcctttttt gaacatgtgt tcaaaagaaa 6660
   aagagaaact atcaaactaa taactgagtt tgttgaacat cgagattgac tgatctgaga 6720
   ggcttaaaac tgattgccct gaaatagact tgcatgtttt ggatgatggc tttgggtctc 6780
   ccagagcact tggtttccct tggtgctgat tttttctctc aggattaaag cccctttatg 6840
   tgatgttgtt acagcagctt attgcaactt cattcagctg cttgaaaaag aagggagact 6900
   gcctctaggt tccatgtgtt ctttcaggga tggtattttg atgttgcgta ttctcttgaa 6960
   catgtattct tccctgagaa tttctgtgtg ccgtggtaga agaaatactt gccagaaatc 7020
   gccactcatg gtatgctttt gtagtgtcga agtgtcccct agaggtgaca aggcttgtga 7080
   tagtgttgat tctaacaagc atgaatcttt cctttatttt agcactgtgt gttacgtgcc 7140
   agtaatttgc agcttatcct ttgtttctag ctaggtaagc tgggaaatag cctagtactt 7200
   tgtctatgtg tttatcttca aaatgtccca aatagccctg ggaaaaaggt cgtgcagctc 7260
   aatgggggct ttcaacttac aattttcttt gttttaggct ccataaaaat acagactcac 7320
   cagttcctgc tttgatgtga catgtgactc cccagaatac accttgcttc tgtagaccag 7380
   ctccaacagg attccatggt agctgggatg ttagggctca ggtaagtaac cttccttttt 7440
   ttttttttag tatatgtcct ggtttggcca tctgtttttt ttttttttaa aaaaaaaaaa 7500
   aaaaggaaaa gaggaaaaaa atatactact cttggacagt ataaaagtac cccaaagact 7560
   aaagacataa ctgtgccaaa ctgtgccata taataaaaaa aagtcacttc cctgagccct 7620
   gaaaggtcag tgtgtgtagg gttacttggt cgccacagcg tgatctgggg gcgggcgtca 7680
   gattagagcc ggaactggtg atctgcaact tcagttcacc ttgaagcagg tcagctgagc 7740
   tgagagcgct tgcactgagc ccttttgcgc tgctgctgtt gccttagcgg tcttcagcat 7800
   gtgtttgctt tggtttgggg taaatggctt agtggtcaac attagtgagc agtggtaatg 7860
   cattttcaga tatgggaact ggtatgtggt tggttcccta gaaggacacc ctcctgaaag 7920
   ctgtctcaga acaccggggg ccatggctaa tgtcatgtgc ttgctacact cctcccatgg 7980
   taactaaaga gagtacccag atatacatct ggttcttggg actctagagg aggatgagta 8040
   ctttgtaaaa acctgggggc cccagtcatt ctaggtctga cactcaggtg ctgtatcagc 8100
   tgcagtttga actacttggc accattgtgt ggactttagt tttgtaaaaa tgaatggttc 8160
   cttaatttct caaccttcag gctcaacatc tcaagctgtt tgttgttgtt gttgttgttg 8220
   ttgttttgtt ttgtcttttt gattagtggt cactccgcat ttgatgtttt gacatgtgga 8280
   tttcagaact tctgtgtggt aaagcagaat gttccaattg aattttccca ttttttttcc 8340
   cctaagcaaa aatgtgagtt ttcacttatg cggccattgt gattccgact gagaccctaa 8400
   gtcgttcttt gttgtctctc tagtggtttc tgacacctca gtgtgaagct gttgtagaca 8460
   tccataagaa atagcctgct tagggattat gtgagggcaa ggtttggctg aaggagaata 8520
   gaaggtgtgg aaggaagcgg aagaccagaa gagcatcaca ctgcctcaag tcccaaattt 8580
   gattctgctc ctgatcctgt gactcaaatt gtcattctct tttactgctg tggggtgtgc 8640
   tctgggccca gctgacagga cattctttgt actccacgta tttatgctgg tgggagttgt 8700
   atggctagtg ctttgtgctt tgtctcagaa attgtgtgga ctaaatgtaa tatgggcagt 8760
   gaatgggttt cttctggaag atgttaggcc tggagggtgg tttgggtttt tacgagtctt 8820
   tttctcagat tacagattac gaatggatgt tataaaacag gcagtgttga caccatacag 8880
   tttctcctaa gaaatgtaca gagagttcaa cctgaggaag ctaattgaaa ataaactttt 8940
   aaaaagaaat gtagaaagag gaaatttaaa aagtatctta tgaatgggaa agatgtagcc 9000
   atttgagctt caaagaactt ttgagggaat tctcaaagga cagtacccgt ctttagtaga 9060
   taaatgcttg atatctattt ttcttctttt actttttaaa gaattactaa aataatacat 9120
   ggatttatag aaaaatcaaa ccacttaaaa attgatcagg tggctgggca aggtggctca 9180
   tgcctgtaat cacagcgctt tgggaggcct aggcgggtgg atcacctgaa gtcaggagtt 9240
   cgagaccagc ctggctaaca cggcgaaacc ccatctctat taaaaataca aaaattagct 9300
   ggccatggtg gcttgagcgt gtagtcccag ctactcggaa ggctgaggca ggagaatcac 9360
   ttgaacctgg gaggcggagg ttgcagtgag ccaagatcac accactgcac tcctgcctgg 9420
   gtgacagagt gagactcggt ctcaaaaaaa aaaaaaaaaa aaagtatcag gtaaaaaaat 9480
   gaaagctccc cgcttaatct ctgctcccac tacccagaat aactgctgtt aatagcttaa 9540
   tgtagatcct tctaagtctt ctaagactaa actgtgtact ttttgtgttg taattaacag 9600
   gagggttttt gttttgtttt gttttttctt tttttgagat ggagtcttgc tctgtcatcc 9660
   aggctggagt acagtggcac aatcttggct cactgcaacc tccacctcct gggttcaagt 9720
   gattctcctg cctcagcctc ctgagtagct gggattacag gcgcctgcca ccacacccag 9780
   ctaatttttg tatttttagt agagatgggt ttcaccatgt tggccaggct ggtgtcgaac 9840
   tcctgacctc aggcgatcca cctgccttgg cctcccaaag tgctgggatt acaggcgtga 9900
   gccaccgtgc ctggcctcag gatggtgtta tatatacata ttctgcattt ttccatctca 9960
   tatattcatc tctgcctcat ttcttttaaa tagcagtgta gtccagggct gacttaccgt 10020
   catgcatttc gttgtctctc tatgttatta aaaatgctac aggattgaaa ttcctcatgt 10080
   gccagtcatt ttgaagaatg gctgcctaga cttggaattt ctggtggata ccaccacatc 10140
   actgtccaaa atggctatcc cagttcccac aacagtctga gaaagtgcca ttttcccata 10200
   tcgtcccaga attggatata catcttttaa attggtgccg cttggattat tactgaggct 10260
   aaacctcaat aatcattgtc atagctctta atgtattaaa gatgttaact ctttgtcatg 10320
   cagatatttt cctgttttgt cttacgtctt gattcttgtc tttctgatat ataaaattta 10380
   tgttatgatt tattcttttg ctttcatact taggaaatcc ttttttactc tttttttttt 10440
   tttttttttt tttttttgag acggagtctc gctctgtcgc ccaggttgga gtgcaatggc 10500
   gtgatcttgg ctcactgcaa gctccatctc ccgggttcat tccattctcc tgcctcagcc 10560
   tcccgagtag ctgggactac aggcgcccgc caccacgcct ggctaatttt tttgtatttt 10620
   tagtagagac agggtttcac catgttagcc aggctggtct cgatctcctg accttgtgat 10680
   ccgcccgtct ctgcctccca aagtgctggg attacaggcg tgagccaccg cgccccgcca 10740
   ggaaatcctt tcctactctt aacaacagat caatagtcat ctatattttc ttcctaaaaa 10800
   acctgatgat gtattatatt catacatacg tatttaatac atacaaaagg gccagatgcg 10860
   gtggctcatg cctgtaatcc cagcactttg ggaggctgag gcgggtggat cacctgaggt 10920
   caagagttca agaccagcct ggccaagatg gtgaaatccc gtctctacta aaaatacaaa 10980
   aattagccag gcgtggtggc gggcacctgt aatctcaact actggggtga ctgaggcagg 11040
   agaatcgctt gaacccagga ggcagaagtt tcggtgagcc aagatcacgc cactgcactc 11100
   ctgcctgggc gatagagtga ggctctgtct caaaaataaa aaataaaaat aataatttta 11160
   tagttaagtt gccctgtgtt tttcctgtga ttaaaaaaaa aaatacctgt tttgtattta 11220
   actttggggt gggataaggt atagcccact tagtcagttg tgtctaagcc acttgatgaa 11280
   ccagtcaatc cagtcttcac ctttaacaaa tgctgatttc ttcaagtgag caaaatatct 11340
   cttaaacttt ttcctatttt ctctataaag ttgctttttt ctgaattttt tctttatgag 11400
   gcttgatggg cagccctatc agcagacaat cattcattca cccaacaaat atttgagtat 11460
   ctgcttcaag ccaggggcag tgctaagccc tggaagtggt atcttctcct tatccgggag 11520
   tggggctaca gtgttcattc cagaagactc aagaaaatag cagatcatca caaaagtagg 11580
   ttttacataa atgaggagga atccacaggg ttgtgtatat gtgtgtgtat tttaaattct 11640
   gacataagtt ggagctttag ggtagactgt gacataagag tagtttgttt tccaactcat 11700
   gtaattgccg gctgattttt gtcttgaatt atgataatgg taacttctgt tgaaacaatt 11760
   ctgtattcat cttagcttgg ttgggatgca ataaaagttt tgtactggta aaaatgataa 11820
   ttttcttcca tgatgatttt gaagcttttc tgtaacacct gataaaaata gaggggccag 11880
   gcatggtggc tcatgcctgt agtcccagct actcgggagg atgaggtgag aggatcccat 11940
   gagcccagga gttttaggct acagtgagcc atcatcacac cactgtactc ctgtactcta 12000
   gcctgggcag cctcgttctt tagagtaaga cactgtctct ttaaaaaaaa aaaaaaagaa 12060
   aaaagaaaaa aagaaaaggc ttttagtgaa tatattttta gggtagcaag tgtaattttt 12120
   ataaattgca ttatttgaat ctaataaata ccctcaaagc tgaagtcgaa ttctgatttt 12180
   gattgaaaat attttttact taaaggaaga gggatagaat aaatgacacg attaaggaaa 12240
   agacttcttg tataaggccg gggtttttta aatgattttt tgaatagtta gatggacaat 12300
   acggaattaa aaaggtggtg taaaaagtta tacggggctg ggcacagtga ctcacgcctg 12360
   caatcccagt actttgggag gccgaggtgg gtggatcacc tgaggtcagg agttcgagac 12420
   cagcctagcc aacatgacga aacatcattt ctactaaaaa tacaaaaatt agctgggctt 12480
   ggtggtatgc acctgtaatc cagctactcg ggaggctgag gcagaagaat cgtttgaacc 12540
   caggaggcag aggttgcagt gagccaagat cgtgccattg cactccagcc tgggcgacag 12600
   agtgagactc catctcagaa aaataataat agaaaaaagt tatatggggc tgggcatggt 12660
   ggctcatgcc tgtaatccca gcagtttggg aggctgaggc agcttgatca cttgaggtca 12720
   ggaattcgag actagcctgg ccaacatggt gaaactccgt ctccactaaa aatacaaaaa 12780
   ttagccaagc gtggtggtgc gtgcctgtag tcccagctac ttgggaagct gaggcaggag 12840
   aatcgcctga acctgggagg cagaggttgc agtgagccaa gatcatgccg ttgcactcca 12900
   gcctggacga caagagcgga actccgtctc aaaaaaaaaa aaaaaagagt tatattgtaa 12960
   agtatctctg ctaccctgcc ccaaacattc atttctttcc ctggaggcag ccactattac 13020
   ctgtttcttt aaaaaaaaaa aaaaattgtg gccgggcacg gtggctcacg cctgtaatcc 13080
   tagcactttg ggaggtggag gtgggtggat catgaggtca agagatcgag accatcttgg 13140
   ccaacatggt aaaaccccat ctctactaaa aatacaaaaa attagccggg catggtggcg 13200
   ggcacctgta gtcccagcta ctcgggaggc tgaggcagga gaatcgcttg aacctgggag 13260
   gcagaggttg cagtgaactg agatcacgcc gctgcactcc agcctggcaa cagaatgaga 13320
   ctccatctca aaaaaaaaag cgtgtgtgta cccgtgtgtt aaatttttat tataaaatat 13380
   atataacagg ctgggcgtgg tggctcacgc ctgtaatccc agcactttgg gaggccgagg 13440
   caggctgatc acgaggtcag gagatcgaga ccatcctggc gagcacggtg aaacctcgtc 13500
   tttactaaaa atacaaaaaa ttagccgggc atggtggcgg gcgcctgtag tcccagctac 13560
   tctgagagcc tgaggcagga gaatggcgtg aacccaggag gcggagcttg cagtgagccg 13620
   agatcgcgtc actgcactcc agcctgggcg acagaccgag actccgtctc aaaaaatata 13680
   tatcgatata tataacaatt tacagtttta atcatcttaa ctgtacagtt cagtggcact 13740
   gagtacattc acattgttgt acagccatta ctaccatcca tctctacaac ctttcgtctt 13800
   gcaaaactga aatttcatac ctattaaaca ctaactccca cttctcccct ttcccccacc 13860
   ccaccctctg gcaactacta ttctactctt tgtctctatt ggacatattt ttgtgtgttt 13920
   aggtttgtgt atatatttta catgtgtgta taaatacaca agggtatgtg ggcgttgtgt 13980
   ttttacacat actataaaat attgtgcaca tttgtttatt ttaatgtatc ctgtagatta 14040
   tttagaatcg gttcatacag agccgtatca ttccttttaa gggttgggtt taaatagtct 14100
   gttatggatg taccacaatt tatttaacca gtttttaaat tttttactta ttttttttaa 14160
   tgttttttta gagacaggat cccactccat cacccagact ggagtgcaac agcatgatca 14220
   tagcttactg cagccttgaa ctccggggct gatgtgatcc tcccacctca ggctctcttg 14280
   tagctgggac tacagggatg tgccgccatg cctggctaaa ttttttattt ttatttttat 14340
   ttttattttt ggtagacacg gggtttcact gtgttgccca ggctggtctt gaactccttg 14400
   ggcttgagta atcctcctgc cttggcctcc caaagtgccg agattacaac catgagccat 14460
   ggagcccgac aagagtagcc ttttatttgc agaccattag agaaaaagtt gcgtgtaagt 14520
   agataagcat acgaatttat cactgcaata ctgtttgtag agatcacaga gctgttaggc 14580
   taggaagaag tttttagcag ttagcactag aaagcaaaac tttggaaact tccatttcat 14640
   caaaagtgac ctcatctgag tagggcatgt gagggaagga cctttttact cgacaagggg 14700
   aacttgccag ttcttgtttg tgaaccttga agaaagtgga gcatttttat tttgggacca 14760
   ttctaggttg tggtagttcc ttggtactcg gggttggaga atctccctct gctgctggac 14820
   cttctgcctc ctcatgctgt aatatgcata agaggtgtcc tagaagtatt caggaacagg 14880
   ctgtggttgc tttgttgttc tgtgactgtt gaactcttta tatagcagta cttttttaag 14940
   ctgtactaat tgagcattta ttatgtgtca gttactgtgc tagctgctgg aaactatcct 15000
   accctcagca gtcttctggt ctaatgggag gtacagacag gcaaatgggc tatgacttta 15060
   caagaggcta acttctgtga tggagagaag ttccagccac taagggagca cataaaaatc 15120
   agaggagatc ctaccaagaa ttgggaggta ggtcagggag gagacttctt tgaaaaatgg 15180
   catctaagac ctgaacagca aggaaacatt agctaggtca aggacaaagc atcagtgggg 15240
   agagatgcat gagtcttttg gaaggaccag catgtgtaga ggctctgaca ggagagaatc 15300
   tctcaggccc attcagagag tccactgtgt gaagacgagg catgaacaac cagccaggac 15360
   atgtggagag gagcattttt ctttttttct tttttctttc tttttttttt tttttttttt 15420
   ttgagacgtc tttctctgtc acccaggctg gagtgcaatg gcatgatctc ggctcactgc 15480
   aacccccact tccagggttc aagcaattct tctgccttag cctcccaagc agctaggatt 15540
   acaggcaccc gccaccatgc ccggttaatt tttgtatttt tagtagagac ggagtttcac 15600
   catgttggcc tcaaactcct gaccttaggt gatccacccg tctcggcctc ccagagtgct 15660
   gggattacag gtgagagccc ccacgcccgg ccaggctcgg ctttctttcc aggccccgtc 15720
   acactcttag atgttccctg ggccccagaa ggcagcgtga ggtgctctgc ccatctatgc 15780
   tgtccttttc caccccaccc tatccactgg gcccctgggg ctcccactcc actccaggtc 15840
   tcttcatgga cccttctttc ccttcacttc tgtcccagga gacctgggag actcatggga 15900
   acaggcatca tcttctccag cacagttcat tgtctaatgc acgaaagtgc tgggctgtga 15960
   gaaggtcagg agcttacatg gaatgggagt cagctgtggt acaaagtacg ctgcttagtt 16020
   ctactgattt tttatttttt atttttacat aagactttga tggtaaagga agcagtgtgt 16080
   gagcaagctg actcctgtct tgtcaagtac caggtccact ctgatgagcc cctgtggaca 16140
   gcactgctgt aactccccaa gcctgggctc aggaggtggg ccctaagcgc tgttcccagc 16200
   tcttcctcca tgctgtcagc tcttccacca ttcttcctgg cgtcattcta aggtcctgtc 16260
   ttcccccgcc ctcttccttc ctggcttcta cgctcagctc aagtagtagc tgtttgggta 16320
   cttttcaccc agacttagga aatgttatca gttggtcgta ttcctgtcag ccctcttact 16380
   atggaactca aataggacag attcagctga attcccagcc ctgtggttgc actctttgtc 16440
   tgccctgctt ccccaaggta gcctgctccc taaaggtcac cgagatactc cccatgcaag 16500
   gtttttgttg tattacacat gacaacatgg aataaagtgt tttgcaaatt tactggatga 16560
   tatcctacta tgtggaacat cctacaactt actcttttca ctcaacattt gacttttgaa 16620
   cctggtctgt attgctccgt gcagaccaat gttcattcat ttgatatgtc tccttgtgcc 16680
   atagaaatag ttttacaata gaagcctgtc cagagccatg agggagaagg ggattgtaga 16740
   gaaacaagga ttggcatctg gtttatgttt acatccttta gtcctgttaa ccgagaagaa 16800
   gatattttta tttccatgtt tgcgaaagct ttccacaaaa aaaagcatcc attttctaga 16860
   cttattaata gggcagtcca aaggttttat ttttagattt gaaagaaagc aaagtaggct 16920
   tctgagatat gcacatttcc ctgtgtccct gtctcttttg caacattcca cattgctgct 16980
   aaaacttgaa catgagcact ttcaagcatg tgcttacttt ctatgtatga ctcatttttt 17040
   aaaacattaa agcattttcg gccgggtgtg gtggctcacg cctatattcc tagcacttta 17100
   gaaggccgag gcgggtagat cacttgaggt caggagttca agaccagcct agccaatata 17160
   gtgaaacccc atctccacta aaaatacaaa aattagccgg gcatggtggc gggcgcctgt 17220
   aatcccagct actcgggagg ctgaggcagg agaattgctt gaacccagga ggtggaggtt 17280
   gcagtgagca gagatcgagc cactgcactc tagcctaggt gacagagcga gactctgtct 17340
   caaaaaaata aaaataaagc attaaagcac tttgtctggt ggtatgtaag ttttggcatt 17400
   acttagataa gttgtatttt aagtctttct acacacaata acttattttt aaaatacaag 17460
   tttctgtgta cttccgggtt ttgtgttttt gtaaacagca gaaggtatta gtccatatta 17520
   agtactaatt tgttctaata ttttcagctg tttttggaat tacacttggc aaaaaaatag 17580
   acaaatgtct gcagttgtga cagtttgttt ttttgttctt tttaactcca gaataaataa 17640
   ttctagtgag tttttttctt ggcttgtaag aacatgggaa tgattagagt catcataaca 17700
   cttaagtagt tatagaaggt tgaacatctg tagtcagttg tataaaattt cctagtcatc 17760
   cttccaggtt gtttaagaca aacaaataca tagtatttat taagctctta taaattttaa 17820
   ttgtaaaaca tcattgtaca aaactttgaa aatgcgtata ggtacaaaga taaaaaatta 17880
   ggccaggcat gccatggctc acgcctgtaa tcccagcact ttgggaggcc aaggtgggag 17940
   gatcttttga gcccaggagt tcaagaccag cctgagcaac atattgagac cctatctcta 18000
   caaaaatatc ataaaacaaa atagaatcac tctggcgatt ccactatcca aaaaaaaaaa 18060
   aaactgctgt taatatttgg tatgtatctt tctagaccaa ggcctccaac tttggttgag 18120
   aattcagccg ggtctgtgac cttggatggg aaaaacattc actctttatt ttcactaatg 18180
   tctctgaaaa cccagatttt tagctgggca tggtggctcg tgcctgtaat cccagtgctt 18240
   tggaagcccg tggagggcag atcacttgag cccaggagtt tgagaccagc ctgggcaaca 18300
   tagcaagacc ctgtctttta aaaaaaaaaa aaatccagcc gggtgtggtg gctcatgcct 18360
   gtaatcccag cactttgaga ggctgaggca ggcagatcac ctgaggtcag gagtttgaga 18420
   ccagcctgac caacatggtg aaatcctgtc tctactaaaa atacaaaaat cagccgggca 18480
   tggtggtggg cgcctgtaat cccagctact ccggaggctg aggcaggaga atcacttgaa 18540
   cccggaaggt agaggttgca gtgagccaag atcacaccat tgcactccag cctgggcgac 18600
   agagcgagac tctgtctcaa aaaaaaaaaa aaaaatccac attttcttca atgacaattg 18660
   taagcaacag atcatggtag tattagaata gcagtgactg tcaccagtag agatcaaaga 18720
   tactttcata tcatattata gttgttacag atattttgaa atatatatgt ccatcactgc 18780
   ttcaaactta aggtaattat tagacccatc tctagatctg gttatttaat gatttactga 18840
   agaagcacat attatttgag aacgaactct tgaaatatgt tgttaatttc tgtgcatttt 18900
   acacatttca aaacattctt ctgaggaggg gtccagaagg ccaaaagggt ccatggcaga 18960
   aaaaggttaa gaacacctgt tctgtacttt gtaccatgtg gctacaaata taaaaacaga 19020
   cgttaagccg ggtatggtga tgcacacctg tagttccacc tacttgggag gctgaggtgg 19080
   gaaaatcact tgagcccagg agtttgaggc cagcctgggc aacagagcaa gaccccatct 19140
   cttaaaaacc agtcagcact tgactagtca gttagtgggg gaaaacacag gtacagtaaa 19200
   aggagtcaaa attacactta aacttaggtc atcttattat atcaaacatt tattaacatg 19260
   tttagtgttt tgtgtatatt atgcaaagtg taatgcaact aaaactgttt aacacttctc 19320
   gatatcacat aaatttgttg gtgcataata gcctaaaagt tacttttgct gttggcattt 19380
   tattttatta cttacttatt tatttattta tttatttatt ttgagacaga gtcacactgt 19440
   cgccaggctg gagtgcagtg gtgcgatctc agctcactgc aacctctgcc tccctggttc 19500
   aagcgattct cccgcctcag cctcctgact agctgggact acaggtgcgc gccaccatgc 19560
   ccagctaatt tttgtatttt tagtagagat agggtttcgc catgttggcc aggatggtct 19620
   tcatctcttg accttgtgat ctgcccacct cggcctccca aagtgctagg attacaggca 19680
   tgagccacca cgctgggcct gctgttggca ttttaaacat ccttctagag tctttcttat 19740
   ttgcctgtaa gcacgtacac atgttgttgt tgttttgaga caaggtctgt cgcccaagct 19800
   ggagcacagt ggtgcaatca cagctcacta cagcttcgac ctcttgggtg caagtgatcc 19860
   tcccaatcag cctcccaagt aggtgggact acaggtgtcc gctaccatgc ccagctaatt 19920
   tttgtatttt ttttgtagag acggagtttc accatgttgc ccagactggt ctcaaactcc 19980
   tttgctcaaa cgatccttcc gcctcggcct cccaaagtgc cgggattaca agtgtgcacc 20040
   tctgtgcctg gcccacatat tgttttagat gaatgagatc atatatgtac ttttgtaact 20100
   tgctaccttt cctcaacaaa atgttgtaaa tatccatgtc cataaaaata gacgtatatc 20160
   ttcacttttc cctaaaatga aaataactta acttgcattt tcttttttgt ttttgttttt 20220
   gttttgagac aggctcttgc tctgttgctc aggctggagt gcagtgatgt cacagctcac 20280
   tacagcctcc acctccagtt tcaagtattc cacctacctc agcctccaag atagctaaga 20340
   ctacaggcac atgtcacgac gcctggctaa tttttaaaat attttttgca gaaatgggat 20400
   ttcactatgt tgcccaggct ggcctcaaac tgctggcctc aagtgatctt cctgccttgg 20460
   cctcccaaag tgctgggatt acagtcgtga gccactgtgc ccagcctgca ttttcttatt 20520
   ataaaagtaa tacatgttca atggacaaaa aattttcaga aaatatgcaa agatgaaaag 20580
   taaaaattgt ccataaatct gtcatctaca gataaagata acttctggat aatgtttttc 20640
   taccatcatt tttagtaatc acataacatt tcgttgtatg tctatgcctt catttaatta 20700
   agaggcattt ccattatttc tgcatgttca tgactctgaa tgatgatatg tctgcctgct 20760
   gatggctaca accctgtttc tgcatttcaa acctctctct tgagctccag atttgatggc 20820
   ctcgtcagca tttacttgag ttgctcataa gtgtctcaaa tttaacaagt cccagtcttg 20880
   atttttttcc cccttcaaac ctattcctca tgttgtctct atttcagtaa acaatatcaa 20940
   catccaccca gttactcagt ccaaaattct aggagtcatc cttgattctg ctctttcttt 21000
   tttttttttt ttttgagacg gagtctcgct ctgtcgccca ggccggactg cggactgcag 21060
   tggcgcgatc tcggctcact gcaagctccg cttcccgggt tcacgccatt ctcctgcctc 21120
   agcctcccga gtagctggga ctacaggcgc ccgccaccgc gcccggctaa ttttttgtat 21180
   ttttagtaga gacggggttt caccttgtta gccaggatgg tctcgatctc ctgacctcat 21240
   gatccacccg cctcggcctc ccaaagtgct gggattacag gcgtgagcca ccgcgcccgg 21300
   cctctttttt ttttttgata cggagtctca ttctcgtcat ccaggctgga gtgcagtggt 21360
   gcaatcttgg ctcactgcaa cctccgcctc ccgggttcaa gcaattcttc tgccccaacc 21420
   tcctgagtag ctgggcctgg ctaatttttt gtatttttag tagagatggg gtttcaccat 21480
   gttggccagg ctggtcctga actcctgacc ttgtgatccg cccacctcag cctcccaaag 21540
   tgctgggatt acaggcgtga gccactgcac ccgccctgat tctgctcttt ctctcatcat 21600
   ccctgatctg tcagcaagtc ctgttaggcc aaccacttct cagcatctgc actgccagta 21660
   tgctagtcca agccacaaaa cacctctcag aggtacgcaa caggactttg ttgttgttgt 21720
   gtgtgtgtgt gttttttttt tttttacggt agaattattt tataacttga agtgtaggga 21780
   tgaccttcct tttaaaattt tatttatttt cttaattttt tattcccata gattttaggg 21840
   gaacaggtgg tatttggtga catgagtaag ttctttaatg gtgatttctg agattttggt 21900
   gcacccatca cccgagcagt gtacactgaa cccaatttgt agtcttttat ccctcacccc 21960
   ctcccaccct ttccctcaag tccccaaagt ccattgtatc attctgatgc ctttgcatcc 22020
   tcatagctta gctcccactt atgagtgaga acatacggtg tttggttttc cattcctgag 22080
   ttacttcact tagaataata gtctctagtt ccatccaggt tgctgtgaat gccattaagt 22140
   cattcctttt tatggctgag tagtattcct ttgtatatat atgccacaat ttctctattc 22200
   actttctgat tgatgggcat ttgggctggt tctatatttt tgtaattgtg aattgtgctg 22260
   ctataagcat gcgtgtgcaa gtatcattat gcaacgggac ttcggatgac atattgaaca 22320
   acttcctcac tgatatcctt gtgaaacgct acatatttca tgggtctgtt tgttatacca 22380
   acactcaatt ctgaatgatg gaattatttc acatttagtc tctttttcat ctagccatta 22440
   tagtttagtt taaacaccaa gttccattcc tgctgcatgc tgaaggattt gcagtacatc 22500
   aggctatagt ccatcaattc attccacaat tgagtaagta aggtatctat agtacctcag 22560
   gccctgtgtt aggcagggat ggggtaacct agacagattt ggtacctgtc ctcatggagc 22620
   ttgactttag cggcagagac agatcttaga taattgtacc agtacttaag tgttcagaag 22680
   gacaagtatg gaggtgggag ccactccagt cctctacagt cacgtgctgc acaacaacgt 22740
   ttcaaccgac aacagagcac acatacagtg gtgttcccat aagattagaa tgccatattt 22800
   ttactgtacc ttttctatgt ttagatatac aaatacttac cattgtgttc cagtgatcta 22860
   cagtattcag tatagtacca tgctgtacag ttttatagcc caggagcaat aggctatata 22920
   ccgtatagcc taggtgtgta gtatgctaca ccatctagat ttgtgtaaga acactctctg 22980
   atgttagcac aaataaatcg cccaatgaca catttctcag aacatatccc tgttgttgag 23040
   cgactcatga ctgtacaatg ggggaacagc tcagcctgcc ttccccaagg aggcaacgcc 23100
   gatgcaggga accgagggtt gaacagaagt gggcaaggtg cagagaggaa aaaggctttc 23160
   cagatggaga agagcatgtg tgaaggccct cagtctggca agagcttagt cctgtgtggc 23220
   aggagctgaa cgaggggtta ggggagagtg gcaaagggtc cgggccatca gagaggtcaa 23280
   caggggctgg atcatgcagg attttgaatg ttaacatcag agcgatagaa aacatgaagg 23340
   gtaaggtggt ttgggttttg aatttttatc aaaatattgc aactactcgg caacaagtta 23400
   gatataacaa aagaggtcat cgtaaaaaca gcagtctacc ccttcccctt cccttccctg 23460
   actcagacat gacagctttt aactgtttct gattttagtt cctttgatgg ttacctccaa 23520
   aattaaatca tatgcttata catctttctt gatttgccaa ctttagggaa ctgtgttaac 23580
   tccctgaaat gaaagagaat gacttagctt acgttacctc cccatgttga aattcatatt 23640
   cacatttctc tgtaattttt tttttgagat ggagtctcac tctgtcgccc aggctggagt 23700
   gcagtgatgt gatctcggct cactgaaacc tctgcctccc gggttcaagc aattctcgtg 23760
   cctcagcctc ctgagtagct gggactacag gcgcacacca gtgcactggg ctaatttttg 23820
   tatttttagt agaggcgggg tttcaccatg ttggccaggc tggtcttaaa ctcctgacct 23880
   caggtgatct gcctgccttg gcctcccaaa gtgctgggat tacaggtgtg agctaccatg 23940
   ccaagcccac atttctctgt aaattttaaa cagtatttta gtccatatgt cagttcccct 24000
   ctttttaaga taaggatatt aataacccta cttttccttc cactttccct ccttaacttc 24060
   tgcgtcttaa aagctgtact tgaccatacc cattcttgca tttgttaaca tgtgtgttct 24120
   gccctgcaac cacagccaag ttgtagctta attctaaagt cgaaaataac attatttaca 24180
   ttattacaca tgtgagtatt gttcttggcg agaccaaata atgaatgtgc ccaggattac 24240
   atttccttct ttacagatct ggaccactca aaggagaatg accaatcaag gtcaaatgga 24300
   ttcccttttc ttagactctg tcacctgctt aaaatccttg ctaggattta atctacttca 24360
   tattcaaacc ttggcttgct tatatagctc tttgttttgc ccaccatttc tagttgcctt 24420
   tttattttgt ttactattcc tccttgatta tgttgtaaaa tgctattagc agtgctgttt 24480
   atttcgtacg attcggcttt ttccccactg aagacctttc tcttggaagg cctctgccct 24540
   tctgcaatct gggcaagtat ctgctctttg atttaaggtt gcatatacag ctgtacctgg 24600
   accttctcct cactgggctc ttgggttgga gcccgtgtct taaaacccat atcttccttt 24660
   cacatggttt actccctcgt tttctggact gcatccttaa ataatgtgct cagatagagt 24720
   gcatggggag taaactgtag tggataccgt tgtcacctac cccatatcca tttcttcttt 24780
   gcccattcct aacagaatcc tgatattatt cacctttcta ccttattccc aacttcagaa 24840
   aggatcctta agtcattcca cacattgcat tcctttggca actgttactg gtcaaggatg 24900
   gacatacgat ctaagttggt tcactcagac tgaagggaag gacttccatg gcacgttcag 24960
   ggaagacttt ctctttttct ctcactggag taagtgagaa aggtgttgct gggggctctt 25020
   ctgtaacctc agactgaggg acaaagcctg aggataaagc ctgctcacca aggaaggcgg 25080
   agcagagaaa cgggaagaac ctgggtcttg atgccgttgc ttagctggtg attgcactgt 25140
   ggctggggcc tgtcctacct gagctaggga ctcagatagg atggactttc ttactgaatg 25200
   agccagcttc agttttcttt tctgttactt gtaaccaaaa gcattgtaac agataggtct 25260
   tctgagtcac tgcatgcctg aaatcccttt cttctactct tacacttgat tggctgagta 25320
   tagggttcca agttgaaaat cattttttct cctaactttg gaggcatctc ttctagtatg 25380
   cagagtaaca aatgcaacaa atgcctgatg ccaatcttat tatggttctt ttgtaggtgc 25440
   ccttcttttc tcttccttcc ctggaagttt ttaggatttc gtctccttat ttttagtgtc 25500
   ctgaaatctc atgagaatat gtctagagtt gtgtcttgtt cactcgttgt actgtggtca 25560
   ttcaatctga atacttttgt caactctttt ttttaaaaaa aaattactaa tttacatgct 25620
   ttcattttct ctatggggac tactgtttaa ctttggacct cttatattgg tcttctgtct 25680
   tctctcatat ttttcattta ttgggcggtg gggggtgctc tgaattttgg aagatctcct 25740
   gcatttattt tctttttcct tttttctttt tttttgaggc agagtcttgc tctgtcaccc 25800
   aggctggagt gcagtagcct gatctcggct cactgcaacc tctgccttcc aggttcaagc 25860
   aattctgcct ctgcctccca cgtagctggg attacagacc tgcaccacca cgcccggcta 25920
   atttttgtat ttttaataga gatggggttt caccatgttg ccaggctggt ctcgaactcc 25980
   tgacctcaag tgatccgccc gcctcagcct cccaaagtgg tgtgagccac cgcacccagc 26040
   ctcctgactt tattttctat cccttctgtt aaattcttta tttcagccaa accttttttt 26100
   tttttttttt aacttactgt gaagcctgtg tgcctactgc ctagattaga caattgttaa 26160
   cattttggcg tattttcctg gtctgtatag atagggtgca tttttatacc tttttgctca 26220
   ccatttaaaa ggaactcaca gatgtcacga catttcaccc ctaagtactt cagtatacat 26280
   ctcctaaaaa taaggacatt ttccaatata agcacaatac cccgatcaca tctttaaaag 26340
   ttaacaataa ttctctaatg ttatcaggca cccactttaa attcagattt cctccaaatg 26400
   tctcttatcg ctgttgaggg gctccaaagc aattaagatt tatacattgc atttgaatat 26460
   gcttcctttg tctcttattt atttatttat tttattttga gacagagtct cgctgtctcc 26520
   caggctggag tgcagtggtg tgatctcagc tcactgcaac ctccgccttc caggttcaag 26580
   cgattctcct gcctcagcct cccaaatagc tgggattaca ggtacgcacc accacgccca 26640
   gctaactttt ttgtattttt agtagagaca gggtttctac taaaccatgt tggccaggct 26700
   ggttttgaac tcctgacctc aagtgatctg cccgcctcgg cctcccaaag ttctaggatt 26760
   ataggcgtga gccaccatgc ccagcccctt tgtctctttt aacaagaaca gcctacccac 26820
   ctctccttca cttgtgatac tgactttttg aagcagtcag cccaattgtc atctagaagg 26880
   gcccacattt taagatacat atttttagag atggcgtgtc actatgttgc ctaggctggc 26940
   cttgaactcc tggcctcaag taatcctcct gcctcagcct cccaggtagc tgggactaca 27000
   ggcttgtgcc accacgacta gctggcccac attttagatg tctctctttt cttgtgatat 27060
   aatttaactt gtttgtccat atatattact tgtagacaag aagttaggtc tacaggcatg 27120
   ttcaggttaa acattttggg caagaatatt tcataggcag tactgtgtac ttcatatagc 27180
   atcagctcac caggcactta atggttggtt accctgtggt ttgtgatgct acgttttaac 27240
   acttgattaa agaaggaggt gactgccaaa tctccccatt gtagacatac agttttcctg 27300
   tggtaattga tatataatct gtgatgtgat gctttggcac catacaaata tcctattgcc 27360
   caacagccat ttgcataatt attttagcat tcattgatcc ttgcctgaaa ccattattac 27420
   attgggggtt gcaaaatgat agttttttta attgtcacct cttctacatt tagtactgtc 27480
   attcctctgt aatgaatttt ccctcatcaa ataaggaggc attatatttc ctcctaaaaa 27540
   gacagtatag atgcttaatc tttgccttta ttacatttca gagtaaggag ttggtagaat 27600
   aatcacctca aatggtggca aaatatttgc tgttgtgttt ccttttctct ttcatgtcaa 27660
   tatggactca tagggattta tttattaaat gttttacaat tcaaattggg tcaacaagag 27720
   tcccttcatg ttggttccta tattctcctg gcataccctc attaattagt ctttgagcaa 27780
   tttcttgttt tctggcacaa gatattccat cccaatatgt gcttttcgtg tcctagaact 27840
   gattccaagg agctctggtt ccttatagtg gcaacggtat ttagaatcca acatgtgggt 27900
   actaaatgtg ctgattctta ctggggtaac attatatcta ggcactttca gaggacatcg 27960
   ctagaaaata tattcagttt ttatttttta tttttaattt ttttgtagag acaaggtctc 28020
   actatgttgc ccaagctggt ttcgaactga gctcaagcga tcctcccacc tcagcctccc 28080
   aaagtgctgg gattacatct gtcagggcaa actgctggta aatattttca caaaagaaaa 28140
   ataaaaaaac caaagtgctg ggattacaga tgtgagccac tgtgccctcc attttttttt 28200
   taagtcataa atacataatg atgtcttcta tggcaattta acattacagg gttcttcctt 28260
   aacttttttt tttttttttc ttttttttga ggcagggtct tgctctattg cccaggctgg 28320
   agtggtgcag tggtacaatc ctaattcact gcagcttcaa atgcctaggc ttcaaacaat 28380
   cctcctccct cagcctcccg agtagctaag actacaggtg catatcacca tgcccagcta 28440
   atttttaaaa attttttgta gagatggggg gtctcgttgc gttgcccagg ctggtctcga 28500
   actcctggcc tcaagtgatc ctcccatctc gatttcccaa agtgctggga ttacaggtgt 28560
   gagccaccat gcctggccaa cttttatttg atctgtatat atcttttcct tacactgaaa 28620
   atgttgggtc ttactaatgt taacatagtt acttatttgc tttatcttgt aatagacata 28680
   aaattacaaa attataatac caatattacg ataaatagct gggcatggcg gcgctcgcct 28740
   gtagtcccag ctactcagga agctgaggtg gagggactgc ttgaacctaa gagtttgaag 28800
   ctgcagtgag ctgtggttgt gccactgtac tcctacccgg acaacagagt gagtgggggg 28860
   aaaaaaaaga aataatggaa gatccagaga aagccccaaa tatttaaaaa ttaaacaata 28920
   cactttgcag cttcctcacc tctctcagcc ttcatagaat tgaagagagt tagggccttg 28980
   cactagatta ggctttgcct taaggaaatg ttgtgtttga tttgatcttt tatccagacc 29040
   attaaaactt ccttaacgtt tattagtaat aaggctattt cactttcata tcatttgtgt 29100
   gttcactgga gtagcacttt tagtttcctt caagaacttt gattcacaac ttggctaact 29160
   ttggtacaag aggcctagct tttggcttct cggctttcaa catgcctctc actaagcctg 29220
   atcatttggt ttttggttta aagagaaaga catatgactc ttccttttac ttgaatgctt 29280
   agaggccatt gtagggttat taattggcct aatttcagta ctgtcttgtc caagggaata 29340
   gggaggccca aggagagaga gagagatggg ggaacagctg attggtggag tagtcagaat 29400
   atacacattt atcacttaag ttttgccatc ttttcttttt ctttttcttt tttttttttg 29460
   agacagtctt gctctgtcac ccaggctggc atgcagtggc atgatctcag ttcactgcaa 29520
   cctctgcctc ctgggctcaa gcaattctcc tgcctcaacc tcccgagtag ctggtattac 29580
   aagcgtgcgc caccataccc agctaatttt tatatttttt agtagagaca gggtttgcca 29640
   tgttggccag gctggtctcg aattcctgac ctcaagtgat ccacccgcct tggcctccca 29700
   aagtgcgggg attacaggcg taagccactg ccgcctggcc aagtttgcca ccttttttat 29760
   tttttttatt ttttgagatg gagtctcact ccatcaccca ggctggagtg cagtgatgca 29820
   atctcggctc actgcagtct ctgcctcctg gattcaagcg attctcctgc ctcagcctcc 29880
   cgagtagctg ggactacagg catgtgccag cacgcccagc ttgttttttg tatttttagt 29940
   agagacgggg tttcaccatg ttggccaggc tggtctccaa ctcctgacct caggtgatct 30000
   gcccggctca gcctcccaaa gtgctgggat tacaggcgtg agccaccccg cccggccaag 30060
   tttgccatct tttatgggtg cagtttgtgg tgccgcaaaa tgatgatagt agtaacatca 30120
   gagagcattg atcacagatc accataacaa atataataat gagaaatttg aaacattgtg 30180
   aaaattacca aaatgtgaca gagacatgaa gtaagcacat gctattggaa aaatggcact 30240
   ggtaaacttg cttgatgcag ggttgccaca caccttcaat ttgttaaaaa ctcagtatct 30300
   gcgaagtaca gtaaagcaaa gcacaatcaa atgaggtgcg cctgcatatt acaaccaaga 30360
   ggcttttcgc agaaacacga gtgtgcttca acattcaaaa atcagttggt gtaattcacc 30420
   acattaagat aataaaggag gccgggtgcg gtggctcaca cctgtaatcc cagcactttc 30480
   agaagccgag gtggacagat cacttgaggc caggagtttg agagcagcct ggccaacata 30540
   gtgaaatcct gtctctacta aaaatagaaa aattagccgg gcagggtggc acgcaccttt 30600
   agtcccagct attcgggagg ctgaggcagg tgaatccttg aaaccaagag gcggaggttg 30660
   cagtaagctg agatcacgcc aatgcattct agcctgggca acagtgagac ccagtatcca 30720
   aaaaaaaaaa aaaaaaaaaa aaagataata aaggagaaaa accatatagt ctattcaata 30780
   aatacagaaa acgcatctga caaaattcag tgccctttca tgataaaaat tcagcagatt 30840
   agaaatagag gaagtgcatg tatgaaagag ctacagatag attgtactta attgtgaaat 30900
   attgaacatg ttaccccgaa aatcaggaaa aaggcaaaga tgtccactct aaccacttct 30960
   attcaacatt atactagagg tcatagccag tataatgagg caagaaaaaa cataagaggc 31020
   attaatattt aaaggaagta aaactgtttc tatttgtagg taatgtcgaa aatgtgaaag 31080
   aaactatcag aacctgctag aactaaaaag tgaattcagc aaggtctcag ggtacgagat 31140
   cattcaaaaa ctaatcagaa atgaaatttc aaatgcaata tcgttaggaa taatttaata 31200
   agaaatgtac aagatttaca cgctgaaact gaaaatgttg ctgaaagaaa ttaaagacct 31260
   aaataaatgg aatgttgtac catgttcatc agctgaaaga ctaagtgtct ttacagtgtc 31320
   agttctccct tcattgaact gtagattcat aaccccagtc ataactgcag ggtttttttt 31380
   gtagaaattg attgtaaaat gtatatggaa gctgggcacg gtggcttatg cctgtaaacc 31440
   agcactttgg gaggctgagg cagacagatg acttgaggtc aggaatttga gaccagcctg 31500
   gctaacatga cgaaaccccg tctctacaaa aaatacaaaa attagccaga catggtggca 31560
   cacacctgta atcccagcta cttgggaggc tgaggcatga gaatcacttg aacccggagg 31620
   tggaggttgc agtgagccaa gatcatgcca ctgcacgccc gcctgggcaa cagagtgaga 31680
   ctttgcctca aataaataaa taaataaaac gtatatggaa atgcaaagga cctagaacat 31740
   ccaaagtaaa cttgaaagag aacaaaatta aggggcttat gtgatgtgac tttatagtca 31800
   tttgattttt aacagaggca ccagaacagt ccggtgcggg aaaggaaagt cttttagcac 31860
   gtgccggatg atggcaaatg acatccatac cagacaaaaa tgaaccttga ccttacccta 31920
   aatattagtt tcttagagtt gccacaaact aggtggctta aaaccacaga aatttggctg 31980
   ggtgcggtgg ctgacgcctg taatcccggc ctttgggagg ccgagtcggg cagatcacct 32040
   gaggtcagga gtgcaagact agcctggcca acgtggtgaa actttgtctc tactaaaaat 32100
   acaaaaaact tagtcaggtg tagtggtgca cacatataat cccagctagc caggaggctg 32160
   aggcaggaga attgcatgaa cctgggaggt tgcactgagc caagattgca ccactgcact 32220
   ccagcctggg tgacagagtg agactccgtc tcaaaacaaa accacagaaa tttgctgtct 32280
   cataattcca gagtctagaa gtccaaaatc aactgtcatc agggccatgg tctctgtgaa 32340
   acctgtaggg gagtccttcc ttgcctcttc ctagcttcca gtggttggca acctttggca 32400
   ttctttgatt tgcagctgca ttacttcaat atctgtcttc ctcatcacac agcattctcc 32460
   atgtatgtct ctgtctttac atggcctcct tcttgtaaga acaacagtaa tattggattg 32520
   gggcctgccc tactccagtg tgacctcacc ttaactaatt acacctgcaa caacgctgtt 32580
   tccaactaag gtctcagtgt gaggtactga gggttaggac ttcaacatat cttttttggg 32640
   ggacatagtc caacccatga cactacttca caccatacat aaaaattaat tcatgagaga 32700
   ttatagacct aaatgtttta aaagctaaaa atataaagct tctgcaagaa aacataggag 32760
   aatatctgtg cagtccaaag aataaggttt tttttaaaca caatttcact ctgttgccca 32820
   ggctggagtg cagtggtgcg atctcagctc actgcaacct ctgcctcccg agttcaagcg 32880
   attcccatgc ctcagccttc caagtagctg ggattacagg tgtgcgccac catgcctggc 32940
   taatttttgt atttttagta gaggcagggt tttgccatgt tggccaggct ggtctcaaac 33000
   tcctgacctc aagtgatctg cctgcctcgg cctcccaaag tgctgggatt acaggtatga 33060
   gccaccgtgc caggccaaaa aaagatttat taggatacat gaagcaatag ctattaaaag 33120
   aaaaaataaa ttggacttca ttaaaattta aaacttttgg tcctcaaaag ataccattaa 33180
   gaaaatgaaa agacagagct gggcgtggtg aggcttgcct gtagtcacta ctgggtactt 33240
   gagaggctga ggcaggagga tcacttgagc ccaggagttc taggccaaca taggcaatat 33300
   agtgagacag tgtctcttaa aaaaaaaaag aaaaagaaaa aacaaaagaa aataggctga 33360
   gagctgtggc tcatgcttgt aatcccagca ctttgggagt acaaggcaga tggattgctt 33420
   gagctcagga gttcaagacc agcctgggca acagtgaaat cccatctcta caaaagatac 33480
   aaaaaattag ccaaccatgg tggcgtgcac ccgtagtcca tagtcccagc tactcgggaa 33540
   gcagaggcag gaggattgct tgatcccaag gaggtcaagg ctgcagtgag ctgaggtcgc 33600
   gccaccgcac cccagcctgg gtgactgagt gagaccctgt ctcaaagaaa atgaaaagac 33660
   aagtccacca agaaaacagt tgagaccagt catagaaaaa atagaaagtc atatctgaca 33720
   aaggatttgt attcagaaga attcctataa ctcggtaata aaaagacaac ctgattttta 33780
   aagggtgaaa atattttaat ggatactttt tttttttttt ttgagacaga gtctcactgt 33840
   gtcacctagg ctggagtgca gtggtgccat catacatagc tcactgcaga ctcgaactcc 33900
   tgggctcaag tggtcttcct gcctcagcct cctgcgtagc taggactaca agcacgtgcc 33960
   actatgcctg gctaattttt aaattttttg tagacatggg ctggtgtgtg tctgtgttgt 34020
   ctaggctggt cttgaactcc tgggctcaag tgatcctccc acctctgccc cccaaagtgc 34080
   tgggccacca cgcctggtgt ggtgtcacca gagatgagcc accatgccct gcctgatact 34140
   tttcttttat catctgttta tttcttgttt atgggtgttt ctgttctgat tttatgacta 34200
   ttctgtattc taagatttct ctgagaatat tatttgtttc ctctaaggac agacttggtc 34260
   tctcttccat ggggctggtt cttgagccgc cagggatggg gggaggacct gggtagctgc 34320
   tgcgctgccc cttctctgct gtggtggagt ggcccgttgc ctgcggggtc tgtctctgaa 34380
   gggtggtttg gagaagttct gtggaggcga ggcaggggtc agctcttgcc ccatggaccc 34440
   tcagagactg ggaaaaggta ggcatcacct gggctgctgc cagccacatc aagagccttt 34500
   ggggtgggtg gggtgggggc cggggaaggg aaggttctag ctgttgattt agaaaagacc 34560
   cattgatttc ttgcctgagg gaagaagtgg caccttgcca ctccaagttt gctctgctca 34620
   ccagccccaa cagcatcagc ctcacctggg agcttattga aggtgccctg ccccaaagct 34680
   tgtgggtcgg gatctggcag tcagcagccc ccaggtgttg cacacatgac agcactgctt 34740
   gcccctcctc tcgtctggtc tggtctggtc tggtctggtc tggtctgaga gcttctttgg 34800
   ggctctgaca ggtatacagg ctctcatctg ctctacagct ctcttgtagt acattcctct 34860
   ctgttccatt agtcaacatg ttcttgccta cttccagcat ctagaaattt gctgagtgta 34920
   tcttatccgt tcagaggtcc tctcctgcta ttgcctctgt tagaaatttc ttccctttca 34980
   tatgtctgta cttcggtttc tttgaggttt gaagggtcaa gtggcaatta catgtaggca 35040
   gttggccatc ctctggtgaa ggctttaaag cccaggaata acaagatcca ttatgtctaa 35100
   aactagcaaa aaccctacct gtcacatggt gaactgaatt agaggtgatg gggacagaag 35160
   ccagattgga gtgagtggaa ggtgagggag tggagattgg gtatagacaa ctctctttaa 35220
   gaagttttgt tgtgatggtg accagaaaaa taggagtggg gtagaggggg atgagggtgt 35280
   ttttcacgtg ggtgaatatg agagcatgtt tacatccttc tgggaatggc ccaggagaga 35340
   ggggagcgtg agaaggagga aatggggacc tggaggagca gactccttgt gtggaggggg 35400
   gaagcggcat ctagagctgt tgcaggaggt gggctttgag taaatgcttc ctctttggta 35460
   aagacgggga acgggaacgg tagaagttgc acggaccctt tgttaaagct cagaagctga 35520
   gcgagaatta gagcttaagt ttatggagta gtcagaataa agtaccccta actgtattct 35580
   tgcacagtgc aggaagccag gggtctgaaa tcaagatgtt gcagggctcc attcccccca 35640
   gagcctctag catggatcct tccttgcctc ttccagcttt tggtggctgc ctggcagtcc 35700
   tagactttcc ttggcttgca gttgcattgc tccattctct gcttgtgtct tcacatggcc 35760
   ttctctgtgt ccccgtattc ttctctggat gcttgtcact gtattaggcc caccctaaat 35820
   ccaggatgat ctcatatcaa gatccttcac ttaattacag aagcaaagac cttccaaata 35880
   aggtcagagc cacagggtct gggggttaag acatacacct aactattcgg ggctaccatc 35940
   gaacccctgt agaagcctgt ccatttccag gatagcagag cccaaggaag ggccagaagt 36000
   ccccccaaaa cagttgtttg cattcaccag attctaagct ataagcagat gggcagtggc 36060
   agtcggtcct aatccatata ccattggcaa caatagttta gttcactgta gacataatga 36120
   gatgcttatc ttctgctaag tagtcctcat ggtaacagcc atattactcc tggctttgag 36180
   tgacagcgct gtgctcttgt ctggtgccca tatacttcag cagctggaaa caagacagtg 36240
   ctcatgattc accggaaagt ttctgtagtt aaaatcaagt gatcccttga gtctattcta 36300
   atatttgtct gtaccatgtt ctgtgacgag acatggaaac aaaacattaa gaagtgaaag 36360
   tatctttgat tatgctcttg aagacaactt tttgtttgtt tgttttgttt gagacggagt 36420
   ttcactcttg ttgcccaggc tggagtgcaa tggtgtgatc tcggctcact gcaacctccg 36480
   cattcccggg ttcaagcgat tctcctgcct cagcctccca agtagccagg attacaggca 36540
   tgcaccacca cgcctggcta atttttttta ttttagtaga gacggggttt caccatgcta 36600
   gtcagactgg tctcaaactc ctgacctcag gtgatccgcc tgcctctgcc tcccaaagtg 36660
   ccgggattac aggtgtgagc caccgtgcct ggccgaagac aactttttaa agatgcatat 36720
   gctctgggct gggtgtggtg gctcacacct gtaatcccag cactttggga ggccaaggcg 36780
   ggcagatcac ctgaggtcag gggttcaaga ccagcctggc caacatggtg aaacaccgtc 36840
   tctactaaaa gtataaaaat tagccgggtg tggtggcggg tgcctgtaat cacagctact 36900
   caggaggctg aggcaggaga atcgcttgaa ccccagaggc ggaggttgca gtgaattgag 36960
   actgtgccac tgcactccag cctgggcgac agagtgagac ttggtctcaa taaataaaat 37020
   aaaataaaga tgcatatatg ctctgaaggc tggctacatt ttattttttc tcaaacaact 37080
   tttacagtgg atagtgctgt aaaagttgtt tagctctggt cattttatct tttttgtgat 37140
   ctgcaaaagg agatcctttt ccttttcact gcctctaata cccaactcca atactccttt 37200
   gacctcacag gcacttagaa tccatcagtg gctccatctt ttctcagact tcacctcctt 37260
   cgtatcctta ttcctctcct ttgtagcttc agtgccagtc agacgtcgtt actccctggc 37320
   acactacctc agcccttcgg cactttcttg ctttgttata ttcagtcgac aaagccccag 37380
   cctcatgaaa ttcagtcatg tctgccccat gcctgctccg tcctgaagct gaatgtgact 37440
   ccttcattga ttcccccttt aagttcatgt ctaccccagg taaacttctt gccgccaagc 37500
   ccacctgcat actgcagttc cccggtctct cctagcccct cccaccctcc tcatcagctg 37560
   tcacacctcc ttgcagctga tatcttagtt cccagagagc acagaagcaa gctcaagaga 37620
   gtgtctacag gccagcgtct actccttcgc tctgcctccc tcatgctgtc acggagcgcc 37680
   tgtcccaggc cctcactcct gttctaaggc atctctccag tcctctactg cactctcccc 37740
   catcagagat tcctccctcg cctgccctag aaacttgttg ctacttctcc caccttgaca 37800
   aaagctgctc ccaacccccc acctgtctag catctctgtt ttcctttacc agcaagactc 37860
   cagtccctgt ctccacaccc tcttcaacct tctccagttg ttcctttgct gccacagctc 37920
   tgccaagctg ctccatgagg tctccagtga tgaccttcat gttgttaaat ctgggagctg 37980
   cttgtcactt cttttcccag agctgtcgtc gtcagttaac cccaataccc atacgagaca 38040
   atcctttccc tcccttgagt ctctgcactg atgtgcacct tctcagtgca aaccacagct 38100
   tttgtctctt tctagctgaa tacatgactg actgtgctta tcttagctgt cacccctgct 38160
   ggagcagaag ctccacagga caggattgtc tccttgattc acagatgtag ctcaagctcc 38220
   tagaacagtg cctggtgtct gtgggtcctc aggaagtcct aggtgagtgt gtgaatgtct 38280
   gtcctcctcg gtcttttctg tttcgcatgt gatgttggaa ggcctcaggt tcaggctttt 38340
   cttctccacc ctcactcccg aagtgattcc gtccagcctc atggctccca aatttatgtc 38400
   tccaggctga ctgggtcacg ggaactcctt atcctcccta gaccttctct ttccctggtc 38460
   ttctcctttc cagtaaacag caactccaac tcctgctcct caggccagaa acctggcgct 38520
   ggttacctca tttgttcaca aatactgtta ctccacctgc aagatggata caaaatctca 38580
   tgagttcccc tgcctccatg gcccccaccc tggtccaggt gtccagcttc cctgcctctg 38640
   cccttgcccc ctggttcttt gtttagtcct gcaatattgt gctcttctgt tcagaaccct 38700
   ccagtgggtc cctgcttgct cagagtcaca gcccaacagg atcacctctc ttgccactct 38760
   cccaccactc atgcactgcg gcctcactgg cctcctggct cctccctgga ccctccaagt 38820
   atgcccctgc ctcaaggcct tgacctctcc tctgccctct gtcccaggta tcacacatgt 38880
   ctgctcaagt gtcaccttcc caatgtggcc ttccccgagc acacccatgt ctgtaagtca 38940
   ggcagcctgc ctctgcccca ccagttcccc ctgcctagca taggacttgt caccatctgc 39000
   cacaagctgt gttactgctt ggttttgttt gtgctctccc actcccttcc cctccctccc 39060
   tctccccacc cctatagaat ctgagtccca tgaggacaga gatttttttt tttgcctctt 39120
   cctcaatata tccccagtgc ctagaatcat gcatatttta atttattgag tgaattaaca 39180
   aataaattct gcagtgggac attgctctaa ggttttgtct tataagaggg acagaagaac 39240
   atgcagcctg ctctgctgtt ggctccttag cttctccatg gctaacagga acaatattgt 39300
   ttatgagata atcaaagcca tagtcatgag ttagaaggta catataaagg caaatatgtg 39360
   gagattgagt tactttggcg ggggcggagg ggtgtgtagt tatctgaaaa catggaacaa 39420
   atcaatgaga gtctaaatag cacccacata tttgcccctc ttttaaaaca tacctccttt 39480
   atgagtgtag aatttaacag cacagtttcc cagtgcagct ttgcgaaaca agagaaatga 39540
   attttaaagg ctgtcaggtg cctttgcaat ctgtggctta gtatttgtgt aacaacattt 39600
   gtgttttgcc aacagatgta ctgcttaaag tacccatgag aatggcctct gtggttttct 39660
   actttctcgg atggaacctt aggaactgat tattgagaag aggctgatac tgcccacctc 39720
   agctcccagg caggcttcag ggccttggtt actactcctt tgggccctca ctgacccttg 39780
   cagacgtctt ttgcaacctc tgtagccaga agagacaact gctttctggc atgttgctct 39840
   ttccctaatg gctttatgag cttcttgaac accaaagcat gtgtgacttc ctcacgcctt 39900
   cccctgtata gtatagcacc cctaactgta tgtgtaagga gaaggagtga gcaattgatg 39960
   gcgtccccct ggatctccaa gggaatggga aggggcaggg tttctggatt cagtcatgtg 40020
   gaatctggtc gttggtgtct aaatgtgcct gttacttgtg cccaatgtcc aggttcacca 40080
   ggaggcttag cctgctcatt tccctgctag tagctatatt ttgggagagc attttttatg 40140
   ctctaattga actttgtggg tctcagcaat ttgaacaatg ttttgttttt cttctggtca 40200
   ggttacaaag gaaagaaaag gaatctggtg ttgtaggagg tggtgtgttg tgtaattact 40260
   tgtttttttt ttttaagttt ttttatattt ttgcaacaag gcaagttttc tttgaataaa 40320
   taatgctggg agcttatcac atagaaggca tatgtgttgt actcagtaca ttctgaatta 40380
   tctgggttta tttttatctc ttgaattcac ttataaacaa ttcaaaggct gcttttttag 40440
   ctaacagaaa taggaagaga atctcagaat ctgttttgtc attttttcaa tgggttgagt 40500
   tatagtgatt atgctaactc ggtggaaaag cagtgtagtg agtaaacccc acatacggca 40560
   tatcatgaac attttgttgc ttttttggct tctgaccatc ttcatacctt tttttgtgtt 40620
   aaaagtctcc ccattgtgta tatcttagta agaggcaggt ggaccctagt ttctacctaa 40680
   gaaaacctga agatggggtt gggggcctgt ggtgtacttc cttctcatgc tgtccatgac 40740
   cactgcagtg gctttgtaag gatccaggac ccagggcccc tcagtgggtg tcttccattt 40800
   ctcaagattg cctcttggtt gtgagatgac ggttgctgct ctagtcattg catccactcc 40860
   tccaggtagg aagaaagagg tcatagggga aaggcaaaat ggcacttgac atcttaactt 40920
   ctaggcccat tcaatatttc catttccatc ttcacaccca tcatcttgtc ctagggaaca 40980
   aacagcacca gcagctcagt agctataaca gcaattatta tttctcacac atgggtctgg 41040
   gtcagctgca agatgggatt cagatctgtt ctcaagcctg tcgttccagg acccaggagg 41100
   gagaagcagc tgccagggga agtctcttcg taggcggagg tcaggagtcc aagaggagtg 41160
   agcagagtca cagaagcctc ttaaagcctc ttcttccccc atcccatcaa cacgtgagca 41220
   agcccagagt cagtgggtgg gaggtgtgct ccgcccatag tcagtagaac aagagcaagc 41280
   ttgaaagaga acgatgaaca ggagtgcact cggccactgt ctccttagcc ataacgtagt 41340
   cccatggtct gctgggaagc ggggaataac tctccttagc tgagcgcagg gtctcattag 41400
   taaggcagat ggagaacatg gatgttgggg gacaactggc agtgcctgcc acaggggaat 41460
   ggatatattc cctccccttg ctctctggaa actaggggat ggcgatgaag cctaggtttg 41520
   gccaaaccca ttctgccttc agggactttg actcttgagg gacagtgagg gattagtcat 41580
   ggtggtggca gttgagtcac aaaaccagca gtggtggcta atgtccagtg atgacatgca 41640
   acagtgccca gggtgggtgt cccaaccagc aggtccctgc agtataacct tggctgtgtt 41700
   tgcagtggtg cagcctctat tcctgctgct tttctgagcc tagttctcta gttctctctc 41760
   catttccaga tctgctgccc aagaactcct ttgtgattga atttaaccag agttgatttc 41820
   tgttgcttga aactcaggac cttgatgcag tgtgcagggt ctgggcaagg taaaaacaca 41880
   ctttcatgat ttcgcctcaa gtatagtaga tgagcttagg ctttttgagc caggtagcct 41940
   tgggattaat tctcagctct tgcacctagc aggtgacctc agcaatcaag tcacttgccc 42000
   tccttgagcc tcagtgttgc cctcagttaa acattgctct cgtgtgttgc tgagcattca 42060
   gtgagattgt atataaagtg atcattgtga aggtattggg acataatttg ggaaagcgtc 42120
   ctggtctaag cttttgattg gctctccatc atttgggtaa tgggatagtg atttaggagt 42180
   atcatagcag ctcaaggggc ccttctggaa agggtatgag gaaggtcagg gacaagtctg 42240
   aaatgaatca tcctaggctt aatgcctgtg actgggcagt gggtacaatt atctcaggct 42300
   agtcagtagt catttattgt gagaatacat gggcagattt tttaatttcc ttctcagtct 42360
   agacttgaat gcaaacagtt cttattgcta acttaccact agtcaccact aatgaacaaa 42420
   gactatgaac aggaaattca taaaagaaga gatacagatg gccaatgaag ataggaaaag 42480
   agttctgcct gctggtaatc aaagagatgc aaacgagaac aaaaatgatg ccttttcacc 42540
   taccaaattt gtcaagatta aaaagaaagc aaagagccag cgtcagctga tgttcatacc 42600
   tgcacctgct cggtagcttg ctaatgttct gcctgctcca cacgccaggc cagcctccac 42660
   ggcgcagcca ggtgaggcct ttccttgtat acaggcacgt ttggattcac tgttgcataa 42720
   gtgagacatg tgcatgcttc acaattcagg aagtgcaaac tccagagtga tgcaaggtct 42780
   gagtgtccct gctgccctgg tctcccagct gcctaggcct ctcttcaggg aacggctgca 42840
   tccagggtct tgtctgtgtc ttttgaggtg ttctatgtac acgcagacac atctaaacct 42900
   atttttattt tccctgcacc tgcttgggct actgtaagct gttaaatttt tttttctagg 42960
   aatttgtcca ttttgtctga ctagcataaa gttgttcatg ttatagttta tacatttgca 43020
   acatctgtaa ttatgtctgc tttttcattc ctagtgtttg ttgtttactt gtaccttctc 43080
   ccgtttttcc ttgtcttggt aaaagctcat taggttcatt ggtcttacaa gaagcagaca 43140
   tgtaggcatt attgccctgt attactctgt gtctcttttc tagttcatta aattctgctt 43200
   ttgcttttat ctttattatt ttttctatgg tcttagagta cattctctgt gttttgttgt 43260
   tgttgttgtt gttgtttgtt ttttgttttg agagagagag tcttgctctg tcgcccaggc 43320
   tggagtgcag tagtgcaatc tcagctcact gcaacctcca cctcctgggt tcaagtgatt 43380
   ctcgtgcctc aacctcccga gtagctgaga tttacaggca tttgccccac acccggcaaa 43440
   ttttttgtat ttttagtaga gatggggttt tggcatgttg accaggcggg tctcgaactg 43500
   ctgacgccag gtgctccacc cgccttggcc tcccaaagtg ctgggattac aggcatgagc 43560
   caccacgcct ggccctaact tcttaagtta taaatttggt tcattgattt ttctaccctt 43620
   ttttcaaatg taaacattta aggctaaccc tctggaacta cttcaactgt atctagcaag 43680
   ttctgatagt attgtcactt taagttagcc caaagatatt ttaaattttc tttgtttctt 43740
   cctttgactc acatattatt ttaaaatgtg tttttcaatt tccaagcaca tggctttggg 43800
   tttgtttttt ttttttccta gttgctctag aaaaaaaaat tgactgcatg aggtggctca 43860
   tgcctgtaat cccagcactt tgggaggccg aggcgggcag atcacctgag gtcaggagtt 43920
   cgagaccagc ctggccaaca tgccaaaacc tcgtctctac taaaaataca aaattagccg 43980
   ggcatggtgg cacacacctg tagttccagc tactcgggag gctgaggcag gagaatcact 44040
   tgaaccggga ggcagtggtc acagtgagcc aagattgtgc cagtgtactc cagcctgggc 44100
   aacaagagca atactccgtc tcaaaaaaaa aaaagaaaaa aagaaaaaat caacttctaa 44160
   ctaaatggca ctgtgatcaa agaacttggt ctgtgtgaca ccagttcttg cagttggttg 44220
   agaagtgctc catgacacat tatccacaac cagtggacag aatatgtcac caccaagcat 44280
   gggtgcagga ttctacatat attcattacc tctgtcttat ccgtttgttg tgaaagtatt 44340
   ttctcttctt actgtcattt tgtttacttg atttatcaac tgatggaata cgtgttaaaa 44400
   cctactgtgg tgatggacac atcagtttct ccacagagtt tggtcatttt cattttatta 44460
   tttattttga ggccacattt tcagttttag atacagtttc acattcctgg tgtattgaac 44520
   cctttggtat tatgaagcga ccatactaat atgttatttt tttttaaatt attatttatt 44580
   ttatttattt ttctttcttt tttttttttt tgagacaggg tctccctctg tcacccagtc 44640
   tggagtgcag tggtgcaatc tcagctcact gcaacctccg cctcctgggc tcaagtggtc 44700
   ctcccacctc agcctcctga gtagctggga ttacaggctt gcaccagcgc gcctggctaa 44760
   gtttttgtat ttttagtaga gacagggttt tgccatgttg cccagactgg tctcaaactc 44820
   ctgggctcaa gcagttcacc agccttggcc tctaaaagtg ttgggattac aggtgtgagc 44880
   caccacgccc ggccatccca gttttttatt ttcctccttt ctgtgtcttt atgcatcagt 44940
   gaggtggagc ttttgaaaat gtcagatagc tagatttttt ttttttgaga cggagttttg 45000
   ctcttgttgc ccaggctgga gtgcaatggc gtgatctcgg ctcactgcaa cctccacctc 45060
   ccaggttcaa aacaattctc ctgccgcagc ctcctgagta gctgggacta caggcacgtg 45120
   ccaccacacc cggctaattt tgtattttta gtagagatgt ggtttcacca tgttggccag 45180
   gctggtctca aactcctcac ctcagctgat cacccgcctc agcctcccaa agtgttggaa 45240
   ttacagacgt gagccaccat gcccagctga tagctagatt tttttttttt tttttttttt 45300
   gagacggagt cttgctctgt cgcccaggct ggagtgcggt ggcgcgatct cggctcactg 45360
   caagctccgc cccccgggtt catgccattc tcctgcttca gcctccagag tagctgggac 45420
   tacaggtgcc cgccaccacg cgtggctgat tttttttttt tttttttttt tgtattttta 45480
   gtagagacgg ggtttcaccg tgtcagccag gatggtctcg atctcctgac ctcgtgatcc 45540
   gcccgcctca gcctcccaaa gtgctgggat tacaggcgtg agccaccgca cccggcctcg 45600
   agatttttta aaaaatccat tgtctgtttc gtcctttaac atctatttga tttctgatgt 45660
   atttagattc atttatagca tatttgtgca ttctattttt ccatattttc tgttctttta 45720
   aattttcctt ctcctttctt gccttctctt agacttttaa aaaaattgta gtaaaataca 45780
   catcacctaa aatttaccat ttttaaatgt acagttcagt ggcattaaat acaaattgga 45840
   ggccaggcat ggtggctcat gcctggaatc ccagcacttt gagaggctga ggtgggagga 45900
   ttgcttgaac ctggaggtcg aggttgcagt gaggtgtgat cacactactg ctatagccca 45960
   ggcaacagag tgagaaccta tctaaaaaaa caaacaaaca aacaaacaaa cagattggca 46020
   tcacacagtg tgtgtgctgc tgtgctgtgc tgttatcatt taacattaaa acataaacat 46080
   cagcactgtt atggactgaa tgtttgtgtc ccccaaaatt ccagtgttga agctcaaacc 46140
   tccaatgtga tgttatttgg agatggggta tttgagaggt ggttaggttt agatgaaatc 46200
   aggagggagg gtgggtttcg catgatgaga ttagtgtcct tataacaaga gacagcagag 46260
   agcttgcttt ctccaccatg tgagtatgca atgagaaggc agccatctcc aagtcagaaa 46320
   gagagccctc accaggggct gaatgtgctg acaccttgat cttggacttc tagcctccag 46380
   aactgtgaga aatttcagtt gaataggccc cccagtctgt actattttgt tatagcttga 46440
   gcagactaat acacccatgt tatgaaaagc tataaacatc cctttaaagg ccacctaacg 46500
   cgtcgtatta cctcatgcat cttctctgct gagctttcca aaagagtggc ttaggctggg 46560
   cctggtggct catgcctgta atcccagcac tttgggaggc caaggcaggt ggatcacctg 46620
   aggtcaggag ttcaagacca gcctgaccaa catggagaaa tcccgtccct actaaaaata 46680
   caaaattagc tgggcgtggt ggcgcatgcc tgtaatccca gctacttggg aggctgagcc 46740
   aggagaatca cttgaacctg ggagatggag ggtgcagtga gccgagatcg tgccattgca 46800
   ttccagcccg gccaacaaga gcgaaactcc atctcaaaaa aaaagagtgg ctggccgggc 46860
   gcagtggctc acgcctgtaa tccccgcact gtgggaggcc gaggtgggtg gatcacgagg 46920
   tcaggagttc aaaaccagcc tggccgagat ggtgaaaccc cgtctctact aaaaatagaa 46980
   aaactagcca ggcgcagtgg caggcgcctg taatcccagc tacttgggtg gctgaggcag 47040
   gagaatcgct tgaacctggg gggcggaggt tgcagtgagc caagatggtg ccactgcact 47100
   ccagcctggg tgacacagtg agaccccgtc tcaaaaaaaa aaaaaaaaaa aaaaaaagtg 47160
   gcgtatgttc ccggtctcca cctctcattc gccagcccct gtgaccaaac ccctgggatt 47220
   agatctccat cgggtccctc ccctcaccct aacctcacat gcagtggttc ctgagaagct 47280
   gctgtttatc tgatcacggg atccagcagg atctgagggc tcattcactg aatctgtact 47340
   acccaacaag gcaggcttgt ccttacccac acttttcagg tgaaaactta atcagattca 47400
   agcgtttatc ttagtggcct cttcgtggtg tgtgatttcc ttcttgcagc tctttactgg 47460
   ctaaaactct tcactgggag ttaaaccagg tgtggtcctt gaccctcatc attttgtccc 47520
   atctcctatg ctgggctctg tatccttgtc tccaagcagt ctcgtgtgac agggaagtta 47580
   cctattattg tatggtcccc tcttcttgtc acctctggtg tggcacttgt tccatttctt 47640
   gttcaatgaa tgtgatttgt gaatgtgcca catctgtggg aggaaggcag ttcgcagcaa 47700
   gagttgtagt tcccttgctt tgccctgagg gccaggactt tacactagat ttttttgttg 47760
   cattcctgca ctcataaagt acacgtaaac ttagaggtgg tgtgttaaat gctagaactt 47820
   aaaactgagt tcagctaggc tcaagtgccc cacaattttt gtcccaccag acaatttcat 47880
   gaatatctac agattgttcc ctcaatatgc ctcaatattg aggcatattg atttggggag 47940
   cacagacaag tcaaggttga tgtgcatccg ctgaacatga tcttaagttg ttgaagtagg 48000
   tattattaat gaacgaacgt ctattttagc tccaagagcc cctggtcttc cagaagagac 48060
   tcccaatgat cttccagaag cagctgggct tcttttggtc ctcccccacc caggtgcagt 48120
   cccgttctcc ctgcctgggt gagtttgatt cctctgccgt ggaataggct aggctgctac 48180
   ctcttgagcc tcttccacca ggccatccat taggtggcga cagagagcac taaaaggaac 48240
   tactatggaa ataaaacttg ttttgcttct tggggggaaa aaaaaagaaa aaggcttgtg 48300
   gggcgtgtgt gcattttagt cagattttac tgtgcaaaac atttgagaga tttctgccct 48360
   ctttctccct tccattcttc tcaacccact gggcgcccta ccacccctgt ctccttcaag 48420
   ataaggtaga tcagaagacc aaatagacaa atgccatgtc cactgttttc tgtcacagtt 48480
   gatagccata ccagtcaccc aagctggaaa cccaacaggc ccctgccctt cactgcccac 48540
   atccaaggga gccaccaagt cccacaaatt gttcctttaa ctgtttgtcc ctcttttgtc 48600
   ttctgtcatc ccttctaccg ctgtcctggt tcaatccctc atcttgctct gggactccca 48660
   tgataactta tgatctttcc tccacctctc tatcttccac acaatcccta tcatcaatct 48720
   cattcccctg ctttaaaccc aagagtggca ccctgtgttt gagtgacatc agggcccctc 48780
   atgtggctcc tgcttctctt tgtggcccct tctgttgctg ctgcttttct tatggacata 48840
   gccctcagca gtccaggact gcttgtagtt cctcccaggt accatgtttc ttgcactcct 48900
   ggcctttgtc attggctccc ctgcccaagt ccccttctct tctcctttcc ttcccctgtg 48960
   gccttgcctt gtcgtgctta tccttgaaga ccctgtcagg tatctcttcc ccaaagagcc 49020
   ctgcctaacc accgcccccc tcccccccgc gacacacaca cctgggttag gccctgcctc 49080
   tccacgcctg tgcctcctgc caagtcctca gaggtctctg atcccatgtg cctccaggca 49140
   tggcataggg ggactgtgac acagcctctc ggcccacaga cccctgattg tgggcttgct 49200
   cactgcccca gtcagtcctc ttattgagga ttccgtgcca gttcctgact ccagcacaca 49260
   cacgcatgta ggccgccctc ctagtgcctg gctccttccc tgtctcttct gccactcatt 49320
   cccatggtcg tatcctggag ttggtcatct ctggaactcc acccacggag ttctaactta 49380
   gctctgccta tgatgaggac ttactgtctt cccagcttgc agtgtcaacc actgtttgat 49440
   gcccctggga ctccattggt cttccctcct cttgtgaact gtgcccatca ccagacgaac 49500
   ttgttttaga atttaccatc gtaactgagc tcccttcagc gcatacctcc ttggctcctg 49560
   ctcagtctct gtttctcctt ttagccaaac tctctaagag ttctctccac tcatgggccc 49620
   tcttccttgc ctgctgctcc ttgctcagct tgctccatga acacttgctt ttccacattc 49680
   catgaaataa tcttaacagt catcagtggc tgtcactcct tctcagccct ttccttgtgt 49740
   ctcttctggt acctcatact ctccaggtgg ccatctcacc tcacatcttg aaccctctgg 49800
   gtttgggaga attctcacct cacgagttga tgggaggcct ggcgacctct gattgtagtc 49860
   actgcaaatg ccagatgctt atttgcctag cttccctttc ggtcagggca gcagttggca 49920
   aacggtatct caagggccaa gtccggccca ctgcctgttt tcgtaaatga agctttacca 49980
   ggacccagtt ccaccccgtc ctttgtgcat tgtccaagac tgctctcctg tacaacagca 50040
   ggtgggctgg ttgtgacaga tactgtctgg cccacaaggt ggaaaatact atctggcccc 50100
   ttacagaaag aaattacctg agttctacac gagggtgtag gtatgtaccc tggcctctgc 50160
   cagtcagact cacatgctag gaggagcagg gccaggcagg agagccgtgt ctcctggcat 50220
   tggggctgca ggaaagacga gctcctaggc cacagtgcca gtggccagtg ctgctgcctt 50280
   gcagggtcgc acaggcagtg ctggctgttt ggactggctt ggcagaagga tttgaagtat 50340
   tgttcctagc caactggact caaacctgtt cctccaaccc tctagggatt ctcttatcac 50400
   ccagtagcct tttcccccta gttttaaaaa ttgcagtaaa atacacaaaa gataaaatgt 50460
   actgccttaa ccatttttaa gtgtacaatt ctgtggtatt gagcacattt atattgtgca 50520
   accatcacca catgcatctt caatagcttt tcatcagtcc ttgtctgcat aaatcagcta 50580
   gagttggttt ctattgattg ccaccaataa tcttgacaga tatgtctgat cttccttctc 50640
   tgcactgact cctctttctc tgtctcctct gctgattctt tctcctcatc acaactccaa 50700
   atagcagggg gagggttgtt tacacttata ttcagcacgc cctcacgcac tcgcctggct 50760
   cttcagacct ctcccctgaa ctccagattc atacatccag ctgcccttta ggcctctaga 50820
   aagctaaata cctcagtatc aaaaacatga gaaacggaag ccagagtcct ggatctctcg 50880
   ccctttcgct gtcccggtgc ccatgccctt cagggatggc acctagacca gctccctgca 50940
   tccctgccta gactctgcag tagtctcacg tggcagaccc ggaggtcact ctcctctctg 51000
   gatgccctgc ggacactccg gttagcctca gctgcaagag cctcctcacc caaggtcacg 51060
   ccatttccag gacagcaccc tggtgactga gcgaggtaag ggtacaaggc ccttataggg 51120
   ccttctcttt gacttcttcc cacctccctt tatggatgtc agttcctgat gaacatctta 51180
   cacctcaaac cgtgacccaa catctgcttc tgcagagccc ccctgtggca tcttgtgccc 51240
   tcctgcccca ctccctctac tctatatctt cccatagtta gaatgggctt tttttttttt 51300
   tttttgagat aaggtctccc tttgtcaccc aggctggaat gcaatggcac agtcacgact 51360
   cactgcagcc tcaacctcct gggctcaagc agtcctccca cctcagcctc ccaagtagct 51420
   gagactacag gtatgcacca ccacacctgg ctaattttta tattttttgt agagactggg 51480
   gtctccctat gttgcccggg ctggtctcga actcccaggc tcaagcaatc ctcccgcctt 51540
   ggcctcccaa aatgcaggat tacaggcgtg taccagtgtg cccagcctca gatgggctct 51600
   ttgaaaaatg tttattagat caggttactc ccttgagaaa aaccctctca gaataaaacc 51660
   cgaagtccat tacttgaaag cagagccagc ttcatgggcc tttgtaactg cccatgggct 51720
   tgctggaacc caggcttggt ttaatactct actctcacca tctcaaaatt cttaattttt 51780
   gaacaagagg ccctgcattt tcattttgca ccaggctcca caaattgtgt aactgggcct 51840
   gcctcagcag ccacatggaa catgatccct ccttgcctat ttgagctcat tctctaccat 51900
   tctctccctt gctcacctgg ctcccactgc tcttgctctt cctggaactt gccataatgt 51960
   tgctagagtc agagccttgg ctcttgctgc tcctcattgg ctggacccct ctttacttga 52020
   cctgctccct caccactcac ttccttcaga tctgtgttca gatttcgtct tctcagagag 52080
   gcttttggcc cctgtccatc tctctgaatt tacctctgac ctctccccca ccaccactgc 52140
   gctaagctgc ctcagaactt tgtagacatt ctgtctggtc ttctgatgtt tcccccttgg 52200
   aagaatccca aggtgcctga agaatgcttt ctttatcctt gaagttgagt aggttgacta 52260
   gagtctggct tgctattgag cattctttat caaattgtcc tgggaacatg gtgtattctt 52320
   tcagtctgca gatgtagtcg ttgctgtttc aggtcagctc tcttatggat ctttgacagc 52380
   atcttctgtt ccatttgttg agttctgtac ttcagggaca caaattcctc atgttggatt 52440
   gtctttgtct ctcttccaat gctattagct ttgccgtaat tggtttagct tttgtctttt 52500
   tcatctgcat tcactttgtc taatttgatt ttcagctatg tatattctgt ttctggctgt 52560
   ttttcaatgt atttattagt ttcataatga tgtgttttgg tctgcagttt gtttctctag 52620
   gttggaaatt tgtcttttca tcttattctg ttttatcatc ccatctttga actcttttat 52680
   tgggaacatg ttcttatgaa gttgtgggga atttttttcc cttccttgtg tattctcttc 52740
   ttggtgggag actttgcctt tctcgtgcca tctccctccc tgggcctctt ttttttcttc 52800
   tggccataat atgtttgcct agttaccatg tcacttcttt tcgtcttggc tcaggcttgg 52860
   atggctctgc atagtcgttc tgtttgcttt gagacagtgg aggaattctt ggctctctct 52920
   tcccagtttc ttggcatctt ctcttgctgt tttcccctct gagctatcgc gtgcaggctt 52980
   gttatcttgt atccggagag aatttgcacg ctggagggag ctgcagccat gtagtcttca 53040
   gccctatctg gattcttctc tttgttctaa gaactgtgtt ggatgtctta ctaaggctca 53100
   ctctagctgc acgagggatg tgtgtgccat ttccatgggg atagggggca cctcagtctc 53160
   tgggtggttc cataatctgt gtatacctaa gagcagttgc ttcccacaga gctgggctgg 53220
   ctcactgggc actttgccat ttctcctgca cctcccagct ggagtttctg ggtctaaaag 53280
   gaaaaagtga aggactccca cttggttgct tctctccagc ttactgactg caaattccca 53340
   gggcgttgcc cgctccctag ggtggtttct gggggacggg gcaggagcct ggctttgctg 53400
   ctgctttgtc ctctggagtc ttttctcaga ctgctttgaa ttacacccct ttcctttgtg 53460
   tgctgaaatc ttcccttcac actctccttc cctgcctttc ttttgtgtct tatcttacta 53520
   ggactggaag agggcagttg tgcaggaggt ctgcatctaa ttccctaatc catgtgagag 53580
   tgctcctgtt gtgtgctttg tgacatgatg gtgagaaata ctccctagag cagtggctac 53640
   cagaaggaag ccatcccccc ccctgcacac acacacacac acacacacac acacacacac 53700
   acacacacac actccttctg gctgtcctca gatgcctatt ctaggtaaat gtcagattcc 53760
   aaagaaaatc cagttgagat cttttttctt tttttgagac gagagtcttg ctgtgttgcc 53820
   caggctggag tgcagtggca caatctcggc tcactgtaac ctccgcctcc tgggttcaag 53880
   cgattctcct gcctcagcct cccaagtagc tgggactaca ggcgcgcacc accacaccag 53940
   gctaaaattt ttttgtattt ttagtagaga cagggtttca ctgtgttggc caggctggtc 54000
   tcgaactcct gacctcgtgt tctgcccgcc tcagcctccc agagtgctgg gattacaggc 54060
   ttgagccatc gcgcccggcc tccagttggg atcttgactg gaatgactgg tgttcaatca 54120
   ttatagtttc cacctaattt gtatttgtac acaggacagt tactaatttg ttggtacttg 54180
   tttgatcccc agtccctaga gttgtttatg gggtggagct tcagtccctg ctgcttcccc 54240
   tgtggcagca gctggagtca gggtggggac ccagggtgct gctggcagat tcttgagaca 54300
   ggtagaatct cctctatatt ggtgtctctc tctgtcccag cagtgcccag gaaaacctgg 54360
   ccagcctgtc actgacctct ccacctaggg cactggtggt tcaggtcctt ctatttgcca 54420
   ccggcaaacc gtacttctgc cagtctggct cttaggccca gtttctctga tcttgcagat 54480
   tttcttgggc tctgctacgg aatcctcata cctccggcag gtccctcctt tgcccatgtg 54540
   tttaactgtg gtggaatcgt gtgagagctg cttctctcgc atggatccca gccacaccac 54600
   attctacagc ggttcctctg aaggcattga tagagatatt tcctcctgtt ttgcatttcg 54660
   ttggtcattt cagtagaatc agggtgaaat aaacatgggg gctcagatgt cagcattacg 54720
   aaccaagtac gtcaggcagg ctgatgtgga ctgacctaca ctagtgagac gcaagatgac 54780
   gaaaacaagg gcactcactc caagttactg atgagatgtt tggatcaaat gagccagtcc 54840
   ttaagcagag ttctctagta aaagagatct cctttctgcc ctttcttgtt ccccaaaatg 54900
   tgttgccttc atggtgaaaa tttattttgg cagattttct cttctttgat aaaagcagcc 54960
   aacactttgt taaaagtctg tgaaacttat ttacatgaag tatgtaaagg taagaaaaaa 55020
   acattatcaa caagaaatgg agaaagccag cagctgagga cagaaaagtc atgcacagtg 55080
   tcagtgtcta tggaaacagg ccacttggac cttgaggact aggtatttgg aattggaggt 55140
   gagcttggcc tggtgagtct ctaaccactt gtgtgtagga tcagtgtgag aaccctgcta 55200
   gaatatagtg gcagagatgc aaggggaaat cattggagaa gttaccaggg aatgatgagc 55260
   taatctgaaa aaaatacatg tttctaagtt gggcgtggta gctcatctgt agtcccagct 55320
   acttgagagc ctgaggcagg aggatcgctt gagcccagcc tgggcagcac agcgagaccc 55380
   tatctcccta aaaaaaactt tttcgttgtt tagttttggg attttttttt ttcctggtct 55440
   tttttccccc tttttgtgaa taacgggatc tcactatgtt gcccaggcag atctcgaact 55500
   cctgggccca agcaatcctc ctgcctctgc cttcctaaga ttacaggtat gagccactgt 55560
   gttaagcaaa aaaacttttt taaatgaaaa tcatttttta aaagacaggc tttccagggg 55620
   agggtattat tccacttata tgaagtgtca acagtaggca gatttgtgga gacaaataga 55680
   ttagtggtta ccaggggctg agaggagtgg gagtggggag caactgctta atgggtaaag 55740
   ggttgtcttt ggaactagag agtagtgatg gtcgcatgac attgtgaatg tactaaatgc 55800
   tattaatgat aaattttatg ttatgtgtat tttaccacaa ttaaaaaaaa aagatcaaat 55860
   gtcctcagaa tagccaacaa ccttccactt ggctaaatgc ctactcatta aacttcttga 55920
   actaaattcc tttctgattg tcatggttat tgtgtcctgg gcttcagagt ttcacattca 55980
   ggttggcttg gtccagtctg tcatgtatca ctataggtcc ccacattggc ctcttcctca 56040
   gacggacagc ccatctatct gccggggctc tgtgccacag ccagatagac ttgctctgag 56100
   acagctgtgt gggctctgag cactggccag gcatcacaaa acctatcttt atgatttaga 56160
   ataattggtg gtcagctgct gttttaatgt tgttgttttt tttaatttag atataattca 56220
   cataccatga aatttactca tttaaagtgt acaattcatt cttcagtata attcataggc 56280
   tcacagaaaa aattgtttaa aaataaaatg tgcaattcag tgtcttttag tacattcaca 56340
   gagttgtgca accatcgcct ctgtgtcatt ccagaacact ttcagcaccc aaaagaaacc 56400
   ccagacacag gagcagtcac ctcttattac ccgcagcccc tggcaacaac tcatccactt 56460
   cctgtctcta tggatttgcc tattctggac atttcctata aatggaatta tgcactattt 56520
   ggccttttgt gtctggcctc tttcactgag cgtaatgtcc tcaaggttca tctgcattgt 56580
   agcatgtgtc agaatttctt tcctttttga ggctgaatga tattatatcc tatagataat 56640
   gaggttttga ttatccaccc atcccttggg aatgcatatt tgggttgccc ccaccatttg 56700
   gctgttgtaa actgtgctgc catgaacact ggtgtacgga tatctgtttg gttactggtt 56760
   ttggtttttt gtttgtttgt tttggttttt tgagacaagg tcttgctctg tcgcccaggc 56820
   tggattacag tggcacgatc tctgctcact gcaacctcca cctcccaggt tcaagcaatt 56880
   ctcctgcctc agtctcctga gtagctggga ctacaggtag cactcaccac catgcccggc 56940
   taaatttttt tgtattttta gtagagacaa ggtttcgcca tattggccag gctggtctca 57000
   aactcctgac ctcaggtgat ccacccacct cagcctccca aagtgctggg attacagacg 57060
   tgagccaccg cacccggcat agttgtggct ttttgagagt gtatggctag gagtaaaatt 57120
   gccaggtcat atggtaactc catgtttaac atttgagaaa ctgccaaact gttctccaca 57180
   gcaggaattt tttaacctgt atgtggtggg cttgtgtttc ggttttcatt ttacacatct 57240
   ataaagatga gatttgctgt atggcactgg ttgcctgtat ttggggaggg ttctgctttt 57300
   ggttggcaag aactgcattt tatttaagct tagcaaaaca taactggttt ctcgcatctt 57360
   ctcaaaagtg gaggattaag aaatggactg cgaattcaga gcagggcagc tgaacctcag 57420
   gctccaccct tgtagccttc aagctgaacc tcattctctc ttgctccctg gagaccactg 57480
   agacactctg cctgtgccag tttgatttct cacattttta aagggccaaa gcttgtgtct 57540
   caaagtgcta tagcctttat tgattcatgc agagaagcct ccttgattcc gtaattctgc 57600
   agctaatact ggaagtagaa agaattggaa acaccatctg gatgacactt tagggtggaa 57660
   gcagccagta caaggggggg ctcattattt cctctggtcc cagactgttc acctggagct 57720
   gtagccacca ccctgccctt aggttaactg cctcgagtgg tagtttagct ctttgtgctg 57780
   tgccgaggga taactggaag tgaaaggtgc tgagaaatgc catctcctga aagtggcgag 57840
   catgagtgaa tttacgaaag gttgggatat tgctggggct ctggaagttt ctctggagct 57900
   cactccaggg gacagggagg gggctggatt ccaattcaag tgaaaaatac ctttcatctg 57960
   ccttgttcac ctggcttttt tgcctttttg taaaatctga aaacctcagg gattgagtag 58020
   tctttcctta actgcagttg cctgtctggc cacacctgcc agctgttgct tgtacccctg 58080
   taatttgcac ggccttccgg gcctttctca caagatcact gcaggtcaca ttcatgagga 58140
   aaatgcaggc agttcctgcc atcagacccc tcaggatgtc atggtttggc ctgaaaacaa 58200
   gattcctgca actctaattt tcctttgcta gatcaaatga aggatttgat ctaatgtttg 58260
   cattctagca gcaaaatcat tgaatatttt atttcttaag agccttactt catattttgt 58320
   aggtatttta agattttgta aaggcctttc tgcttcaacg tgtgatgtgt gcattcttag 58380
   aaaaagatct tgtgttctgt aaatcacaca ataaaaacat gagttcgtgc aggaaaaact 58440
   ggggcggggt ggatcactcc aaacttgtgt ggtgtggtaa ctggagctca ctgatgaaac 58500
   catgaacagt tctggctgaa agaaccccac agtacactga ggtctgttgg catcgccgcc 58560
   agcaccgccc cggtcccttt gtgcgcgcca ccacacctgg ctaatttttt atttttagta 58620
   gagagggggt ttcaccatgt tggtcaggat ggcctcgaac tgacctcgtg atccgcccac 58680
   ctcagcctcc caaagtgctg ggattacagg tgtgagcctc cgtgcccagc cttcagcttt 58740
   ttttcttaat gtctttgtgt aatatgaagg cattctcttt aattgttaaa aagcttgcct 58800
   accactgctt caaaatatta ctgtcagttg gcatagcttc tgattatatt gatgtcatct 58860
   cccttctttt aaacatgtta ccatattggc actgtatttc ccctagccca ttgatcactt 58920
   gagagttagt catagtcctc gtgctgtttc actcctaaac gtttaagcat gcctttccta 58980
   agagcagaca gtacagttaa gacactcagg aagtttagca atgagctaac acagaacctt 59040
   acatttctcc acatacccca taaatgcctt ttttagagcc tttgagcctg gaatgcagag 59100
   tccaggactg tgtgttgtat tcgattgtgt catcccttca gtctccttta tcagaaatgt 59160
   tcccccaccc ccttgttttc tctcatcatc ttgagtccag accgtggttg catggcacgc 59220
   cctctctgga ctcttcctgc tgtctcctca cggtaagctt cagtctccta ctcgtgatgc 59280
   tggttcatct cagcagcact gaggggcctg agctcagttt ggtcgtgtgt taaggtggtg 59340
   cctgccagat ttctccacag aaaagggccc cgtactttta tttgctcttc agcctgtgta 59400
   ttccttcctt tcttgccagc ttgtgggtgg cttctctagc agcttctgta agatactcag 59460
   tttggcagtt gtagttgttt cagcaggaga ggtgtctgca tacctgacca ccacatggct 59520
   agaagtcgat ccatcctctg gcgtaaccat cctccatgct actgtccctg gggcactgag 59580
   gccctcctca gtgacttcct ccacctcatc ttccgccatc tccaagccac cagtgtccaa 59640
   cggactactg atatgtccca aactcatcat ttgtttattc accaattcag agcccacctt 59700
   tttttcagca ctgagctagc ctctccttgc tagaagctta cggtcgaagg tctccagcca 59760
   tcagaagaag cacgtggagc gctgcgtccg tgttgtggtt attcatccag catgtgttga 59820
   gtaagggttg cacctgtgcc tggcattatg cattgagcgg ggagatgggg gttggcacgc 59880
   acagtggggt gttctaagta cactgagggc tcgggtgccc tggctcatag agcagggagg 59940
   gaggcaggag cagggaaggt gtctcagaag tgccatcttt tttttttttt ttttgggaag 60000
   tggaatcttg ttctatcgcc tggtctggag tgcaatggca ctatctcaac tcactgcagc 60060
   ctccgcctcc caggttcaag cgattctcat gcctcagtcc ccgagtagct gggaccacag 60120
   gcgcacacca ccacacccgg caaatatttt gtatttttag tagaggcgga gttttgccat 60180
   gttggccagg ctggtcttaa actcctggcc tcaagtgatc tgctcacctc ggcctcctaa 60240
   ggtgttggga ttacaggcat gagccactgc gcccggtcta gaagtgccat cctaactgaa 60300
   cctgaaagat gaaagttctc cagatgaacc tgaaagttct ccaaatgaaa aggtgggagg 60360
   gggtgacagg gttaggccca gagcctctgg tgacacaggg tgggcatcat tggtcacttt 60420
   ttcctcgagg gaggggcgtc acacgggtga tagggtggga gctataacca tgttgatagt 60480
   gccgcctctg cccatctggc ctggcatgcc ctgagccctc tgtcccacct gtggaactca 60540
   taagccctga cagcccactc actcctgatt cattatccac acccctgtgc ttccgctgtg 60600
   cctggagcaa gctttcttca gggggaaggg aggctggaac tatgttgtag ttacctattt 60660
   gtcctccctt ccaaactgtg agttcttgga ggtggaagga tgctgcagga tctggctcag 60720
   gacgaaggca gttggtgaac agacacgtgt gtttttgact cacggtgatc tcagacaagt 60780
   tcctctgtct agtcgaactt ctttttttcc atctgtaaca ctcaggagtt gaataggtgg 60840
   tttttctgag gatacttcaa ctgtaaaatg tatgaacttg tgaactagct atttagttct 60900
   cctcataatc aagattgtgt gtgtgtgggg ggttctgatt agagggagga tgaagagagg 60960
   tgtatggggt tttttttgtt ttgttttctg tttgtttgtt tgtttttgag atggagtctc 61020
   actctgtcac ccaggctgga gtgcagtggc acgatttcgg ttcactgcaa cctctgcctc 61080
   ctgggttcaa gtgattctcc tgcctcagcc tcctgagtaa ctgggattac aggcatgcac 61140
   caccatgccc agctaatttt tgtattttta atagagatgg ggttttcgcc atgttggcca 61200
   ggctggtctc gaactcctga cctcaggtga tccacctgcc tcggcctccc aaagtgctgg 61260
   gattataggc atgagccacc acacctggca ggtttctttg aaaaagtttg tgtttcggca 61320
   aacaccataa accccctggg ggacagcctt ggggagtcac ctggcaccct agcccagcct 61380
   ccctcccttg ggtcctgcag tgaaggctta gtgagggtgt gcaaatgccc aggtcaccct 61440
   gggactgggc aggccctctg ggctaagggt aaactcattt ggaatacctg ttttctatca 61500
   ttgtttttta tttgttaaat ttaaagggta caagtgcagt tttgttgcgt ggatatattg 61560
   tatagtagtg aagtctgagc tttcagtgta accatcacct gaatagtgga cattgtaccc 61620
   gttaagtaat gtctcatccc tcaccccctc ccacccttcc cagtctctcc agtgtctgcc 61680
   attcctcact ctgtccatgt gcacatgcta ttcagctcct gcttctaagt gagaacgtac 61740
   ggtatttgac tttctgtgtc tgagctgtgt cactgaagac aatggactcc agctccatcc 61800
   acgtttttta tcatttttac ctgcactcca cacccagcac aatccaggct tctttgtggg 61860
   ttttttgaaa tttgtcttta attataaaag tagcagccag caaattaaca aacacccatg 61920
   tgcctttcat tgcacagaat tgaaaatcat catactatat ttgcttcaag taatttccat 61980
   tagaaagaac tagaatatta cagtagagtt aaagaccctt tatttcccat cttcagtgct 62040
   cttaaaagtt catttagggc caggcatggt ggctcacacc tataatccca gcactttgag 62100
   aggcccaggt gggtggatca cctgaggtca ggagttcaag accagcctgg ccaacatggc 62160
   gaaaccccat ctctactaaa aatacaaaaa ttagctgggc gtggtggtgc gcatctgtaa 62220
   tctcagctac tcgggaggct gaggcaggag aatcgcttga actcaggagg gaggcagagc 62280
   ctgtatgcag taagccgaga ttgcgccacc acaccccagc atgggtgaca gagcaagact 62340
   ccgtctcaaa aaaaaaaaaa aaaaagttca ttcattgtac acttagaaat agttaaaaag 62400
   gtaaactttg ttttgtgtgt gtgtgtatat atatatatat atatatatat atatatatat 62460
   atatatatgt attttttttt gagacatata tgtgtctggt ttgttcgccc aggcaggagt 62520
   gcagtggcat gatcaacggc tcactgcagc ctcaacttcc taggctcaag tgatcctcct 62580
   gcctcagcct cccgagtagc taggattaca ggcacacacc accatgccca gctaattttt 62640
   tttttttttt tttttttttg tagagacagg gttttgctat gttgcccagg ctggtctcaa 62700
   actcctgagc tcaagcgatc cacctgcctc acctcccaaa gtgctgggat tacaagtgta 62760
   agccaccaca cctgacctgt tttgtatatt ttaccacaat aaaaagcctt taaaacccca 62820
   agcagacagt tcattttcat tcaggcccaa ctcagaatct gatcacagcg gggtttcccc 62880
   cctttctagc gagtagctga agaactgttt tctctccttg atggtataac tgtctctgtg 62940
   ggtgttgctc ccctgccgct ccagtggttt tgtttttgtt tttgtttttt tgagacggag 63000
   tcttgcgatc tcagctcact gcgacctctg cctcctgggt tcaggcgatt cttctccctc 63060
   agccttccat gtagctggga cttacaggca cctgccacca cgcccggctc atttttgtat 63120
   ttttagtaga gacggagttt caccatgttg gccaggttgg gctcgaactc cagacctcag 63180
   gtgatccacc tgcctcagcc tcccacagtg ctgggattac aggtgagagc cactgcaccc 63240
   agccggcccc tcagtctttt ccttctcaat cagtggcacc accatcttcc caggctttgg 63300
   acatggtccc tgactcaccc ttgcccctca cccccacact aatccacctg cgagctctgt 63360
   tgcttcacca cctagaccag ccccaaatcc tcaactgccc ccaccctggg ccacacctgg 63420
   accactgcta gaggcctctc atgggccctc cctgtttttc tcttgcactc cccgggcttt 63480
   ctggcacaag atgccccaga agcagaatca catatctctc ctgggagcca atctagtgtg 63540
   tttactgccc ctggagtgtt attgttggcc ttagaatatg tcccactaca ggtttgcaga 63600
   gcactgtagt caaaagtcat ttgaaataaa tcttttctct gtggtatatt gtcaatttga 63660
   tatagaatta aatttgtttc ttttcttttt tcttttcttt tttttttttt ttaagagaca 63720
   gggtcttggc caggcgtggt gtctcatgcc tgtaatcccg gcactttggg aagccaaggt 63780
   gggtggatcg cctgaggtca ggaattcaag accaacctgg ccagcatggt gaaaccccgt 63840
   ctctgctaaa aatacaaaaa ttagccgggc gtgatggcag gtacctgtaa tcccagctac 63900
   tcgggaagct gaggcaggag aaggcttgaa ctcaggagtc ggaggttgca gtgagccaag 63960
   atcacgccat ggcactctag caagactctg tctcaaaaaa aaaaaaagag agagagagag 64020
   acagggtctt gctctgtcac tcaggctgga gtgcagtgat gtagtcatgg ctcactgcag 64080
   cctcatactc ctgacctcag gtgatcgacc cgcctcggcc tcccaaagtg ctgggatcgt 64140
   aggctgaagc caccatgcct ggcccgaact catttgtttt tatttgcatt aagtgtaata 64200
   aggttttgtt acttttagtt tgaattttat tttgggtaat ataaacattt acatgattca 64260
   gaagtcagaa ttacactgag gcatgttcag ctaggcctca ctcctgtgcc tgtaccctta 64320
   ccttttcccc cctaccccat gcagggaatc aatttcatta gttcctggtg tgtccttcct 64380
   taataccccc ccttctttct ttggtagaat gcagtagata tgtttgcgct ttgctccctt 64440
   tgcttcacag tggatcctgg aaatgactcc atcgcagttc ttagagctct tgtttagtcc 64500
   ctttggatct gcacagtact ccagtgtgtg ggcgcaccat aagtttattc agcaagtgcc 64560
   ctggtgatag ggatcggggg tattggaagc ctttggctgg taaaaataat gttgtagcaa 64620
   ataacatcat gcatatgttc tttgagattt ctggaggtgt ctctttagca tagatttcta 64680
   gaaggcattc cttacgtcac gtttaggttt atagtttgac ctcatggtga ttaaggtatt 64740
   tgggaaatgc aaatgagagt ttcgaagaag ccagatcttt cccattagtt cactgttttt 64800
   ctctgacatt aggaccgtcc gggttctagg gagcagccct ggcgttaggc agtgccatga 64860
   tggattgtgt agaagtagcg attcccatct gtctgctttc ttggcccact ctgctggtgg 64920
   ctccccttcc ctccctcctt tatggggagc tggggagctg cctaggggtc cattctcaaa 64980
   ggctgatctc tggtgggcaa caggccacac ctagctttcc agggttcttc gtcatttccc 65040
   attgagagct gtaagactca gagacatgaa aaggaagctc tggctgggca cggtggctca 65100
   cgcctgtaat cccagcactt tgggaggctg aggcgggtcg atcacctgag gtcaggagtt 65160
   cgagaccagc ctgcccaaca tggtgaaacc ccgtctccac taaacataca aaaattagcc 65220
   tggcgtggta gcacacgcct gtgatcccag ctactccgga ggctgaggca ggagaataac 65280
   ttgaacccgg caggcggagg ttgcgatgag ccgatattat gccatcggca acagagcgag 65340
   actccatctc aaaaaaaaaa caaaaaacaa aacaaggcga gctctgtgct gggacagatt 65400
   agggacccct ctttacagca agaaagactg ctctgtgggt tgtaggatgc ctttgtgtat 65460
   gcagtggctc taggtgactc tggcagccac actctgggcc ctaaacttct ggaggaagat 65520
   acaggatagg gaggaactca ggggtgagtc atggtgggga caagacattc cctcactcta 65580
   agaccttgtc actagattgg aacatctctt gcctccctac acctgacctg atggttctgg 65640
   agagatacgt ccttgcagct tctgagtccc agcactaagc agcctttggt aacttcccta 65700
   catcatttga gttctggttt cctaaggatg cttgccagtg agtgccatgg tgccctcatt 65760
   gcacagtctg tgcagtgtag acaagagggg aagtctcttg gggtagacca gccgcaaggc 65820
   ggtgactagc actgatgtga accacatggg acaggggagt tgtggggctg agaacacgga 65880
   gggtgggagt agtcatgctc ttttccagaa tgaactgcta acgaagggac tcgcaggtgg 65940
   ctgctgcttc tttccaagct gcccctgttg ttgcagaggc tctggagtcc taggaggttt 66000
   cacggtggca tactcgacag agtactagag catcatggcc agatagtgat gctggggtgt 66060
   ggggcctcac ggtggccatt tctgacgaga ccccaccggg ccaaagtgat gtgtagagaa 66120
   ggagctgctt cggtcaccag aaaagaacgg ggaagcctcc cacactagaa taggtagggt 66180
   gctcttctcc accggcagga aggggatgta tggctctgcc tggaccacac ctttttcctt 66240
   gctcttccct ccgcatctgc tgtggccgag gccattcctc atcagggaac atgtgttaga 66300
   ggctcacgcc acctgggacc acttgtctta tcacccccag gaccctaggc ggtagtttcc 66360
   tgtggcctga gttagctgat atttatatag tgccattgtg ccttttctcc tgtgatgctc 66420
   acagtaagga tgcctagatg gggttacctg ttgtcaagat aaggaaactg aagcacagaa 66480
   tgctgaggtc atttgctggg ttcatgtttg gaaagcggca aaggatttca gtgcaggttg 66540
   gctggctcca aacctgtgtg tgctttccat gacactgtac tgtgtgcctc attgagcctc 66600
   attctagaaa accaaaaaca cacccaaggc ccggccttca caaaggagac ccctccccca 66660
   tttggctccc tttccagcag tcgacggcct cttgtcagcc atcgagccca gagtcccttg 66720
   aagtgcgact catgctgggg tggtatgctc aggagccgca gtgtttccgc tcagaggaaa 66780
   gggctctgat tctcctgcag tgctaggaga cttgtgggtg gccacagtgc aggtcaggca 66840
   caccggccag caccacccac agcccaaatt cctaaagaaa tatttgggtc ccagcttggc 66900
   ccgagtctct gttgtcctgg ggaaggacat caagatctga gtgtatgatg gcctggggcc 66960
   ttgcatgtgg tgggggtcca agcctgcctc tgctcacttg ttctgcagac tggcatgttc 67020
   tctgtgatac ttacatactt gtttaacact tcagggaaga aaagtcagaa gaccaggacc 67080
   tccagggcct caaggacaaa cccctcaagt ttaaaaaggt gaagaaagat aagaaagaag 67140
   agaaagaggg caagcatgag cccgtgcagc catcagccca ccactctgct gagcccgcag 67200
   aggcaggcaa agcagagaca tcagaagggt caggctccgc cccggctgtg ccggaagctt 67260
   ctgcctcccc caaacagcgg cgctccatca tccgtgaccg gggacccatg tatgatgacc 67320
   ccaccctgcc tgaaggctgg acacggaagc ttaagcaaag gaaatctggc cgctctgctg 67380
   ggaagtatga tgtgtatttg atcaagtaag taagagcaac tcctatctct acagggcagg 67440
   gagggcaggg acaaggatcc ctcatggagc aggaaaatgt atgtgcccag ggtggggtcg 67500
   gggggaacat aaacaatgaa cactgagacc aggtgtgctt gaaatgaccg tgtacagagg 67560
   tcgctgccct gagtgggaag ttctcaaggt agcaggccct ctatcctctc cacacctcaa 67620
   gtctttatct ggggatggaa tagctgcgga agcagaggaa cttgcagagc taggggttca 67680
   gaggggtgaa gaagcatgtt tcagttctgc cttttaaatg atcccaaaaa ggttagcagt 67740
   tttcaaatga catttgcaga cagcctcatt taattccatg agaagggtga gcaaaggatt 67800
   atcttgttga aactgattcc tggagagact gagcaccgta cctgagttca aacttgggaa 67860
   tgttctagat ggtgactcag gcccaggcac caaccagcag aatgggcctc agcctgacaa 67920
   cccttctgta ccaggcctga ctctttggtt gctgaacttt ggagaggcct gggggggtca 67980
   gcggcaggca gacgagtgag tggctttggt gacaggtcct caggggcagc caggcagtgt 68040
   gactctcgtt caatagtaac gtttgtcaga gcgttgtcac caccatccgc tctgccctat 68100
   ctctgacatt gctatggaga gcctctaatt gttccttgtg tctttctgtt tgtccccaca 68160
   gtccccaggg aaaagccttt cgctctaaag tggagttgat tgcgtacttc gaaaaggtag 68220
   gcgacacatc cctggaccct aatgattttg acttcacggt aactgggaga gggagcccct 68280
   cccggcgaga gcagaaacca cctaagaagc ccaaatctcc caaagctcca ggaactggca 68340
   gaggccgggg acgccccaaa gggagcggca ccacgagacc caaggcggcc acgtcagagg 68400
   gtgtgcaggt gaaaagggtc ctggagaaaa gtcctgggaa gctccttgtc aagatgcctt 68460
   ttcaaacttc gccagggggc aaggctgagg ggggtggggc caccacatcc acccaggtca 68520
   tggtgatcaa acgccccggc aggaagcgaa aagctgaggc cgaccctcag gccattccca 68580
   agaaacgggg ccgaaagccg gggagtgtgg tggcagccgc tgccgccgag gccaaaaaga 68640
   aagccgtgaa ggagtcttct atccgatctg tgcaggagac cgtactcccc atcaagaagc 68700
   gcaagacccg ggagacggtc agcatcgagg tcaaggaagt ggtgaagccc ctgctggtgt 68760
   ccaccctcgg tgagaagagc gggaaaggac tgaagacctg taagagccct gggcggaaaa 68820
   gcaaggagag cagccccaag gggcgcagca gcagcgcctc ctcacccccc aagaaggagc 68880
   accaccacca tcaccaccac tcagagtccc caaaggcccc cgtgccactg ctcccacccc 68940
   tgcccccacc tccacctgag cccgagagct ccgaggaccc caccagcccc cctgagcccc 69000
   aggacttgag cagcagcgtc tgcaaagagg agaagatgcc cagaggaggc tcactggaga 69060
   gcgacggctg ccccaaggag ccagctaaga ctcagcccgc ggttgccacc gccgccacgg 69120
   ccgcagaaaa gtacaaacac cgaggggagg gagagcgcaa agacattgtt tcatcctcca 69180
   tgccaaggcc aaacagagag gagcctgtgg acagccggac gcccgtgacc gagagagtta 69240
   gctgacttta cacggagcgg attgcaaagc aaaccaacaa gaataaaggc agctgttgtc 69300
   tcttctcctt atgggtaggg ctctgacaaa gcttcccgat taactgaaat aaaaaatatt 69360
   tttttttctt tcagtaaact tagagtttcg tggcttcagg gtgggagtag ttggagcatt 69420
   ggggatgttt ttcttaccga caagcacagt caggttgaag acctaaccag ggccagaagt 69480
   agctttgcac ttttctaaac taggctcctt caacaaggct tgctgcagat actactgacc 69540
   agacaagctg ttgaccaggc acctcccctc ccgcccaaac ctttccccca tgtggtcgtt 69600
   agagacagag cgacagagca gttgagagga cactcccgtt ttcggtgcca tcagtgcccc 69660
   gtctacagct cccccagctc cccccacctc ccccactccc aaccacgttg ggacagggag 69720
   gtgtgaggca ggagagacag ttggattctt tagagaagat ggatatgacc agtggctatg 69780
   gcctgtgcga tcccacccgt ggtggctcaa gtctggcccc acaccagccc caatccaaaa 69840
   ctggcaagga cgcttcacag gacaggaaag tggcacctgt ctgctccagc tctggcatgg 69900
   ctaggagggg ggagtccctt gaactactgg gtgtagactg gcctgaacca caggagagga 69960
   tggcccaggg tgaggtggca tggtccattc tcaagggacg tcctccaacg ggtggcgcta 70020
   gaggccatgg aggcagtagg acaaggtgca ggcaggctgg cctggggtca ggccgggcag 70080
   agcacagcgg ggtgagaggg attcctaatc actcagagca gtctgtgact tagtggacag 70140
   gggagggggc aaagggggag gagaagaaaa tgttcttcca gttactttcc aattctcctt 70200
   tagggacagc ttagaattat ttgcactatt gagtcttcat gttcccactt caaaacaaac 70260
   agatgctctg agagcaaact ggcttgaatt ggtgacattt agtccctcaa gccaccagat 70320
   gtgacagtgt tgagaactac ctggatttgt atatatacct gcgcttgttt taaagtgggc 70380
   tcagcacata gggttcccac gaagctccga aactctaagt gtttgctgca attttataag 70440
   gacttcctga ttggtttctc ttctcccctt ccatttctgc cttttgttca tttcatcctt 70500
   tcacttcttt cccttcctcc atcctcctcc ttcctagttc atcccttctc ttccaggcag 70560
   ccgcggtgcc caaccacact tgtcggctcc agtccccaga actctgcctg ccctttgtcc 70620
   tcctgctgcc agtaccagcc ccaccctgtt ttgagccctg aggaggcctt gggctctgct 70680
   gagtccgacc tggcctgtct gtgaagagca agagagcagc aaggtcttgc tctcctaggt 70740
   agccccctct tccctggtaa gaaaaagcaa aaggcatttc ccaccctgaa caacgagcct 70800
   tttcaccctt ctactctaga gaagtggact ggaggagctg ggcccgattt ggtagttgag 70860
   gaaagcacag aggcctcctg tggcctgcca gtcatcgagt ggcccaacag gggctccatg 70920
   ccagccgacc ttgacctcac tcagaagtcc agagtctagc gtagtgcagc agggcagtag 70980
   cggtaccaat gcagaactcc caagacccga gctgggacca gtacctgggt ccccagccct 71040
   tcctctgctc ccccttttcc ctcggagttc ttcttgaatg gcaatgtttt gcttttgctc 71100
   gatgcagaca gggggccaga acaccacaca tttcactgtc tgtctggtcc atagctgtgg 71160
   tgtaggggct tagaggcatg ggcttgctgt gggtttttaa ttgatcagtt ttcatgtggg 71220
   atcccatctt tttaacctct gttcaggaag tccttatcta gctgcatatc ttcatcatat 71280
   tggtatatcc ttttctgtgt ttacagagat gtctcttata tctaaatctg tccaactgag 71340
   aagtacctta tcaaagtagc aaatgagaca gcagtcttat gcttccagaa acacccacag 71400
   gcatgtccca tgtgagctgc tgccatgaac tgtcaagtgt gtgttgtctt gtgtatttca 71460
   gttattgtcc ctggcttcct tactatggtg taatcatgaa ggagtgaaac atcatagaaa 71520
   ctgtctagca cttccttgcc agtctttagt gatcaggaac catagttgac agttccaatc 71580
   agtagcttaa gaaaaaaccg tgtttgtctc ttctggaatg gttagaagtg agggagtttg 71640
   ccccgttctg tttgtagagt ctcatagttg gactttctag catatatgtg tccatttcct 71700
   tatgctgtaa aagcaagtcc tgcaaccaaa ctcccatcag cccaatccct gatccctgat 71760
   cccttccacc tgctctgctg atgacccccc cagcttcact tctgactctt ccccaggaag 71820
   ggaagggggg tcagaagaga gggtgagtcc tccagaactc ttcctccaag gacagaaggc 71880
   tcctgccccc atagtggcct cgaactcctg gcactaccaa aggacactta tccacgagag 71940
   cgcagcatcc gaccaggttg tcactgagaa gatgtttatt ttggtcagtt gggtttttat 72000
   gtattatact tagtcaaatg taatgtggct tctggaatca ttgtccagag ctgcttcccc 72060
   gtcacctggg cgtcatctgg tcctggtaag aggagtgcgt ggcccaccag gcccccctgt 72120
   cacccatgac agttcattca gggccgatgg ggcagtcgtg gttgggaaca cagcatttca 72180
   agcgtcactt tatttcattc gggccccacc tgcagctccc tcaaagaggc agttgcccag 72240
   cctctttccc ttccagttta ttccagagct gccagtgggg cctgaggctc cttagggttt 72300
   tctctctatt tccccctttc ttcctcattc cctcgtcttt cccaaaggca tcacgagtca 72360
   gtcgcctttc agcaggcagc cttggcggtt tatcgccctg gcaggcaggg gccctgcagc 72420
   tctcatgctg cccctgcctt ggggtcaggt tgacaggagg ttggagggaa agccttaagc 72480
   tgcaggattc tcaccagctg tgtccggccc agttttgggg tgtgacctca atttcaattt 72540
   tgtctgtact tgaacattat gaagatgggg gcctctttca gtgaatttgt gaacagcaga 72600
   attgaccgac agctttccag tacccatggg gctaggtcat taaggccaca tccacagtct 72660
   cccccaccct tgttccagtt gttagttact acctcctctc ctgacaatac tgtatgtcgt 72720
   cgagctcccc ccaggtctac ccctcccggc cctgcctgct ggtgggcttg tcatagccag 72780
   tgggattgcc ggtcttgaca gctcagtgag ctggagatac ttggtcacag ccaggcgcta 72840
   gcacagctcc cttctgttga tgctgtattc ccatatcaaa agacacaggg gacacccaga 72900
   aacgccacat cccccaatcc atcagtgcca aactagccaa cggccccagc ttctcagctc 72960
   gctggatggc ggaagctgct actcgtgagc gccagtgcgg gtgcagacaa tcttctgttg 73020
   ggtggcatca ttccaggccc gaagcatgaa cagtgcacct gggacaggga gcagccccaa 73080
   attgtcacct gcttctctgc ccagcttttc attgctgtga cagtgatggc gaaagagggt 73140
   aataaccaga cacaaactgc caagttgggt ggagaaagga gtttctttag ctgacagaat 73200
   ctctgaattt taaatcactt agtaagcggc tcaagcccag gagggagcag agggatacga 73260
   gcggagtccc ctgcgcggga ccatctggaa ttggtttagc ccaagtggag cctgacagcc 73320
   agaactctgt gtcccccgtc taaccacagc tccttttcca gagcattcca gtcaggctct 73380
   ctgggctgac tgggccaggg gaggttacag gtaccagttc tttaagaaga tctttgggca 73440
   tatacatttt tagcctgtgt cattgcccca aatggattcc tgtttcaagt tcacacctgc 73500
   agattctagg acctgtgtcc tagacttcag ggagtcagct gtttctagag ttcctaccat 73560
   ggagtgggtc tggaggacct gcccggtggg ggggcagagc cctgctccct ccgggtcttc 73620
   ctactcttct ctctgctctg acgggatttg ttgattctct ccattttggt gtctttctct 73680
   tttagatatt gtatcaatct ttagaaaagg catagtctac ttgttataaa tcgttaggat 73740
   actgcctccc ccagggtcta aaattacata ttagagggga aaagctgaac actgaagtca 73800
   gttctcaaca atttagaagg aaaacctaga aaacatttgg cagaaaatta catttcgatg 73860
   tttttgaatg aatacgagca agcttttaca acagtgctga tctaaaaata cttagcactt 73920
   ggcctgagat gcctggtgag cattacaggc aaggggaatc tggaggtagc cgacctgagg 73980
   acatggcttc tgaacctgtc ttttgggagt ggtatggaag gtggagcgtt caccagtgac 74040
   ctggaaggcc cagcaccacc ctccttccca ctcttctcat cttgacagag cctgccccag 74100
   cgctgacgtg tcaggaaaac acccagggaa ctaggaaggc acttctgcct gaggggcagc 74160
   ctgccttgcc cactcctgct ctgctcgcct cggatcagct gagccttctg agctggcctc 74220
   tcactgcctc cccaaggccc cctgcctgcc ctgtcaggag gcagaaggaa gcaggtgtga 74280
   gggcagtgca aggagggagc acaaccccca gctcccgctc cgggctccga cttgtgcaca 74340
   ggcagagccc agaccctgga ggaaatccta cctttgaatt caagaacatt tggggaattt 74400
   ggaaatctct ttgcccccaa acccccattc tgtcctacct ttaatcaggt cctgctcagc 74460
   agtgagagca gatgaggtga aaaggccaag aggtttggct cctgcccact gatagcccct 74520
   ctccccgcag tgtttgtgtg tcaagtggca aagctgttct tcctggtgac cctgattata 74580
   tccagtaaca catagactgt gcgcataggc ctgctttgtc tcctctatcc tgggcttttg 74640
   ttttgctttt tagttttgct tttagttttt ctgtcccttt tatttaacgc accgactaga 74700
   cacacaaagc agttgaattt ttatatatat atctgtatat tgcacaatta taaactcatt 74760
   ttgcttgtgg ctccacacac acaaaaaaag acctgttaaa attatacctg ttgcttaatt 74820
   acaatatttc tgataaccat agcataggac aagggaaaat aaaaaaagaa aaaaaagaaa 74880
   aaaaaacgac aaatctgtct gctggtcact tcttctgtcc aagcagattc gtggtctttt 74940
   cctcgcttct ttcaagggct ttcctgtgcc aggtgaagga ggctccaggc agcacccagg 75000
   ttttgcactc ttgtttctcc cgtgcttgtg aaagaggtcc caaggttctg ggtgcaggag 75060
   cgctcccttg acctgctgaa gtccggaacg tagtcggcac agcctggtcg ccttccacct 75120
   ctgggagctg gagtccactg gggtggcctg actcccccag tccccttccc gtgacctggt 75180
   cagggtgagc ccatgtggag tcagcctcgc aggcctccct gccagtaggg tccgagtgtg 75240
   tttcatcctt cccactctgt cgagcctggg ggctggagcg gagacgggag gcctggcctg 75300
   tctcggaacc tgtgagctgc accaggtaga acgccaggga ccccagaatc atgtgcgtca 75360
   gtccaagggg tcccctccag gagtagtgaa gactccagaa atgtcccttt cttctccccc 75420
   atcctacgag taattgcatt tgcttttgta attcttaatg agcaatatct gctagagagt 75480
   ttagctgtaa cagttctttt tgatcatctt tttttaataa ttagaaacac caaaaaaatc 75540
   cagaaacttg ttcttccaaa gcagagagca ttataatcac cagggccaaa agcttccctc 75600
   cctgctgtca ttgcttcttc tgaggcctga atccaaaaga aaaacagcca taggcccttt 75660
   cagtggccgg gctacccgtg agcccttcgg aggaccaggg ctggggcagc ctctgggccc 75720
   acatccgggg ccagctccgg cgtgtgttca gtgttagcag tgggtcatga tgctctttcc 75780
   cacccagcct gggatagggg cagaggaggc gaggaggccg ttgccgctga tgtttggccg 75840
   tgaacaggtg ggtgtctgcg tgcgtccacg tgcgtgtttt ctgactgaca tgaaatcgac 75900
   gcccgagtta gcctcacccg gtgacctcta gccctgcccg gatggagcgg ggcccacccg 75960
   gttcagtgtt tctggggagc tggacagtgg agtgcaaaag gcttgcagaa cttgaagcct 76020
   gctccttccc ttgctaccac ggcctccttt ccgtttgatt tgtcactgct tcaatcaata 76080
   acagccgctc cagagtcagt agtcaatgaa tatatgacca aatatcacca ggactgttac 76140
   tcaatgtgtg ccgagccctt gcccatgctg ggctcccgtg tatctggaca ctgtaacgtg    76200
   tgctgtgttt gctccccttc cccttccttc tttgcccttt acttgtcttt ctggggtttt    76260
   tctgtttggg tttggtttgg tttttatttc tccttttgtg ttccaaacat gaggttctct 76320
   ctactggtcc tcttaactgt ggtgttgagg cttatatttg tgtaattttt ggtgggtgaa 76380
   aggaattttg ctaagtaaat ctcttctgtg tttgaactga agtctgtatt gtaactatgt 76440
   ttaaagtaat tgttccagag acaaatattt ctagacactt tttctttaca aacaaaagca 76500
   ttcggaggga gggggatggt gactgagatg agaggggaga gctgaacaga tgacccctgc 76560
   ccagatcagc cagaagccac ccaaagcagt ggagcccagg agtcccactc caagccagca 76620
   agccgaatag ctgatgtgtt gccactttcc aagtcactgc aaaaccaggt tttgttccgc 76680
   ccagtggatt cttgttttgc ttcccctccc cccgagatta ttaccaccat cccgtgcttt 76740
   taaggaaagg caagattgat gtttccttga ggggagccag gaggggatgt gtgtgtgcag 76800
   agctgaagag ctggggagaa tggggctggg cccacccaag caggaggctg ggacgctctg 76860
   ctgtgggcac aggtcaggct aatgttggca gatgcagctc ttcctggaca ggccaggtgg 76920
   tgggcattct ctctccaagg tgtgccccgt gggcattact gtttaagaca cttccgtcac 76980
   atcccacccc atcctccagg gctcaacact gtgacatctc tattccccac cctccccttc 77040
   ccagggcaat aaaatgacca tggagggggc ttgcactctc ttggctgtca cccgatcgcc 77100
   agcaaaactt agatgtgaga aaaccccttc ccattccatg gcgaaaacat ctccttagaa 77160
   aagccattac cctcattagg catggttttg ggctcccaaa acacctgaca gcccctccct 77220
   cctctgagag gcggagagtg ctgactgtag tgaccattgc atgccgggtg cagcatctgg 77280
   aagagctagg cagggtgtct gccccctcct gagttgaagt catgctcccc tgtgccagcc 77340
   cagaggccga gagctatgga cagcattgcc agtaacacag gccaccctgt gcagaaggga 77400
   gctggctcca gcctggaaac ctgtctgagg ttgggagagg tgcacttggg gcacagggag 77460
   aggccgggac acacttagct ggagatgtct ctaaaagccc tgtatcgtat tcaccttcag 77520
   tttttgtgtt ttgggacaat tactttagaa aataagtagg tcgttttaaa aacaaaaatt 77580
   attgattgct tttttgtagt gttcagaaaa aaggttcttt gtgtatagcc aaatgactga 77640
   aagcactgat atatttaaaa acaaaaggca atttattaag gaaatttgta ccatttcagt 77700
   aaacctgtct gaatgtacct gtatacgttt caaaaacacc ccccccccac tgaatccctg 77760
   taacctattt attatataaa gagtttgcct tataaattta cataaaaatg tccgtttgtg 77820
   tcttttgttg taaaaatcaa gtgatttttt cataaggttc ttttactatt ggaaaagatg 77880
   ggcagcacgc agttttattt tatttttgta agttttttaa tacatgtgaa agcaaagaat 77940
   actcagcatg cctttctaag tgacgcgttt gcaccttttg ttgggaagta ctgtatcctg 78000
   tgctgttagc attctcgata aatctctctg tgaaagtgac tcaaggtctg ggctttcatt 78060
   ataagacaga agtccccctc cagctcacat gacagcatgg tgctgcgttt cctcattgga 78120
   tctggctgtc cctggacaca ggtagctgcc ttcaggcctg ccacgagcgg ccaagggaag 78180
   cctcctccat atgctggcct cgctggcccc tcagcttctt ccaagccagt gctctccagg 78240
   cacactgctc cagcgtgtga cgggaagggc ctggcatgag tcagcctgca gcacaacctc 78300
   cctgctccag acccgtatgg taggggcacc ccctaggtct ggatgtgctg tggtgctttt 78360
   ggacaccccc acccccgcag gctgtggctc ctcctgtgtc tcattctggc caggaccctc 78420
   acgtgccctc tgttgactgc taacgtggtt ctctgaccag gcaagggcag gctgaggggt 78480
   ttgcccaaag ggggccccct tgttactggc ttccttggct ctcaggagca gcctcaccag 78540
   gttggtaagg ggctggagga gacaactgct caaaggagtc cagcttcaca tgcacatgct 78600
   agaaggtacc ctcggaaggc ctggccttca aaggtagatc ccagggttga aaagtcaact 78660
   tgtatgcatt gagcatctcg tatgccagcc ctgttccgtg agctgatggg cctttgtgtg 78720
   taagtaggac caagtgcccc cgtggaggtt agcatgggtg tgcagtcatt tcagatactt 78780
   gagttggtac atctcagtaa agtctgtccc gtgagaagcc atgggtttca tggtatggtt 78840
   ggcatcttcc ttgggagtgg ccacagtggt ggtggcttca ggaaagagac tccaacaggg 78900
   gccagctgtg ggccttgggc acttctcgtt tctaggaaaa gtcctaagtc tgtagggcta 78960
   ggggtgggga accccttcgc tgtcaggatc aagagggcaa ggggaactgt cgctggagga 79020
   gacatccagc tggagaaaca aaagagtaag tctgcgttgc tgcttgtggg gtcttcccca 79080
   tctcagggcg gggaccgggg gtggcggtcc agacaagtaa tcaaggacga tgcccaggag 79140
   gggacaggta cggggtggca ggagctctgc cggcgggctc aggaagcctt caccacagct 79200
   gcctgagctc acccttgcca aatgagggct ggggcagcag caacgcatac actcacggct 79260
   gtggcgggca gcgttctcgg catatttcag gacacctaag gagactgaat ggctcaaggc 79320
   tgctgccgtg tgcagggggc tagacgtggg gcgggcaggc agggctcctg gtaacagccc 79380
   tgcaggccgc agtggagagc agggttccgg cagggccgcc caggagcttt cggaaggccc 79440
   ggccccggcc cctttccgag cagcccgggc ctccgccctg ccctctgtcc ccaacgccgg 79500
   gagccgccgt tcgtcctcca gagccccgcc cgggcgagcc cgggaggccg atcgccgctc 79560
   gcggaacccg ccgggacccg ggccctcccc ggcgcggggc gcccccgtgt gacccagcgc 79620
   gcggccgcgg cgcgcaagat ggcggcgggc ccgggcaccg ccccttccgc cccgccgggc    79680
   gtcgcacgag gccggctcga aggggaagtg agtcagtgtc cgcggacccg gccggcccag    79740
   gcccgcgccc gccgcggccc tgagaggccc cggcaggtcc cggcccggcg gcggcagcca    79800
   tggccggggg gccgggcccg ggggagcccg cagcccccgg cgcccagcac ttcttgtacg    79860
   aggtgccgcc ctgggtcatg tgccgcttct acaaagtgat ggacgccctg gagcccgccg    79920
   actggtgcca gttcggtggg tggcggcggg ctgccggggg gcgggaggcg cgcgggctcc    79980
   tggcgccgac gcctgacgcc c                                             80001
<210> 3
   <211> 20
   <212> DNA
   <213> Artificial sequence
<220>
   <223> Primer
<400> 3
   tatttgatca atccccaggg 20
<210> 4
   <211> 20
   <212> DNA
   <213> Artificial sequence
<220>
   <223> Primer
<400> 4
   ctccctctcc cagttaccgt 20
<210> 5
   <211> 22
   <212> DNA
   <213> Artificial sequence
<220>
   <223> Primer
<400> 5
   aggagagact ggaagaaaag tc 22
<210> 6
   <211> 20
   <212> DNA
   <213> Artificial sequence
<220>
   <223> Primer
<400> 6
   cttgaggggt ttgtccttga 20
<210> 7
   <211> 23
   <212> DNA
   <213> Artificial sequence
<220>
   <223> Primer
<400> 7
   ctcaccagtt cctgctttga tgt 23
<210> 8
   <211> 22
   <212> DNA
   <213> Artificial sequence
<220>
   <223> Primer
<400> 8
   aggagagact ggaggaaaag tc 22
<210> 9
   <211> 25
   <212> DNA
   <213> Artificial sequence
<220>
   <223> Primer
<400> 9
   cttaaacttc agtggcttgt ctctg 25
<210> 10
   <211> 21
   <212> DNA
   <213> Artificial sequence
<220>
   <223> Primer
<400> 10
   cgggggacat aaaagttatt g 21
<210> 11
   <211> 22
   <212> DNA
   <213> Artificial sequence
<220>
   <223> Primer
<400> 11
   tgcattgttt taccagtgtc aa 22
<210> 12
   <211> 20
   <212> DNA
   <213> Artificial sequence
<220>
   <223> Synthetic oligonucleotide
<400> 12
   aactctctcg gtcacgggcg 20
<210> 13
   <211> 20
   <212> DNA
   <213> Artificial sequence
<220>
   <223> Synthetic oligonucleotide
<400> 13
   cacactgacc tttcagggct 20
<210> 14
   <211> 20
   <212> DNA
   <213> Artificial sequence
<220>
   <223> Synthetic oligonucleotide
<400> 14
   gatcactgga acacaatggt 20
<210> 15
   <211> 20
   <212> DNA
   <213> Artificial sequence
<220>
   <223> Synthetic oligonucleotide
<400> 15
   cgtgccatgg aagtccttcc 20
<210> 16
   <211> 20
   <212> DNA
   <213> Artificial sequence
<220>
   <223> Synthetic oligonucleotide
<400> 16
   ggtttttctc ctttattatc 20